(19) Europäisches Patentamt

European Patent Office

Office européen des brevets

(11) **EP 3 623 793 B1**

(12) **EUROPEAN PATENT SPECIFICATION**

(45) Date of publication and mention of the grant of the patent:
**07.09.2022 Bulletin 2022/36**

(21) Application number: **18798973.6**

(22) Date of filing: **09.04.2018**

(51) International Patent Classification (IPC):
*G01N 33/487* (2006.01)  *G01N 27/416* (2006.01)
*G06Q 10/04* (2012.01)  *G06N 99/00* (2019.01)

(52) Cooperative Patent Classification (CPC):
**G01N 27/416; G01N 33/48721; G06N 99/00; G06Q 10/04**

(86) International application number:
**PCT/JP2018/014926**

(87) International publication number:
**WO 2018/207524 (15.11.2018 Gazette 2018/46)**

(54) **IDENTIFICATION CLASSIFICATION ANALYSIS METHOD AND CLASSIFICATION ANALYSIS DEVICE**

KLASSIFIKATIONSANALYSEVERFAHREN UND KLASSIFIKATIONSANALYSEVORRICHTUNG

PROCÉDÉ PROCÉDÉ D'ANALYSE DE CLASSIFICATION ET DISPOSITIF DE CLASSIFICATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.05.2017 JP 2017092075**

(43) Date of publication of application:
**18.03.2020 Bulletin 2020/12**

(73) Proprietor: **Aipore Inc.**
**Shibuya-ku**
**Tokyo (JP)**

(72) Inventors:
• **WASHIO Takashi**
  **Suita-shi**
  **Osaka 565-0871 (JP)**
• **TANIGUCHI Masateru**
  **Suita-shi**
  **Osaka 565-0871 (JP)**
• **OHSHIRO Takahito**
  **Suita-shi**
  **Osaka 565-0871 (JP)**
• **YOSHIDA Takeshi**
  **Suita-shi**
  **Osaka 565-0871 (JP)**
• **TAKAAI Takayuki**
  **Suita-shi**
  **Osaka 565-0871 (JP)**

(74) Representative: **Hamel, Armin**
**Iridium IP**
**Friedrich-König-Strasse 3-5**
**68167 Mannheim (DE)**

(56) References cited:
**WO-A1-2014/136316  WO-A1-2016/017533**
**WO-A1-2016/053181  WO-A1-2017/073737**
**JP-A- 2016 505 836  US-A1- 2015 275 263**

## Description

**<FIELD OF THE INVENTION>**

The present invention relates to an identification method for identifying nonconforming data included in measurement data obtained from a measurement system, a classification analysis method performing the classification analysis using the data from which the nonconforming data are removed, an identification device, a classification analysis device and a storage medium.

**<BACKGROUND ART>**

For example, as described in Non-Patent Document 1, devices for measuring minute and trace objects have been developed one after another in the field of advanced sensing device development such as nano-sensing, minute measurement, and quantum measurement.

Still, Patent Document 2 describes methods and systems for detecting microbes in a sample. The methods are generally applicable to quantifying the number of target bacteria in a sample counted from a detection region of a flow cytometer histogram. The detection methods can be employed in the presence of other microorganisms and other non-target microbe components to selectively quantify the amount of a target microbe.

**<PRIOR ART DOCUMENT>**

**<PATENT DOCUMENT>**

[Patent Document 1] International Publication WO2013/137209 bulletin
[Patent Document 2] US 2015/275263 A1

**<NON-PATENT DOCUMENT>**

[Non-Patent Document 1] FRosenstein, J. K., Wanunua, M., Merchant, C. A., Drndic, M., and Shepard, K. L : Integrated nanopore sensing platform with sub-microsecond temporal resolution, Nature Methods, pp. 487-492 (2012)j
[Non-Patent Document 2] FWeka3:Data Mining Software in JavaJ, Machine Learning Group at the University of Waikato. Internet <URL:http://www.cs.waikato.ac.nz/ml/weka/>
[Non-Patent Document 3] ꜰElkan, C. and Noto, K. : Learning Classifiers from Only Positive and Unlabeled Data, in KDD '08 Proceedings of the 14th ACM SIGKDD international conference on Knowledge discovery and data mining, pp. 213-220, Las Vegas, Nevada, USA (2008), ACM New York, NY, USAⱼ
[Non-Patent Document 4] FTsutsui, M., Taniguchi, M., Yokota, K., and Kawai, T. : Identifying Single Nucleotides by Tunneling Current, Nature Nanotechnology, Vol. 5, pp. 286-290 (2010)j

**<DISCLOSURE OF THE INVENTION>**

**<PROBLEMS TO BE SOLVED BY THE INVENTION>**

However, many of the above-mentioned advanced sensing devices output only partial information of the target because the measurement system and measurement target are minute, and the output is often affected by thermal noise, quantum noise, etc. For this reason, when the noise signal level is larger than the target signal, that is, the SN ratio is often very bad, the measurement accuracy is too low at the primary measurement stage, which is not suitable for practical use. Further, under such a measurement situation, there is no room for adopting a general noise removal method of removing a noise component by signal strength on the assumption that a small signal is noise and a large signal represents a target.

In addition, it is difficult to apply various kinds of noise filtering using the knowledge about the object and the specific properties of the problem, when the knowledge and properties are not clear. In particular, in the next generation DNA sequencer using single molecule measurement technology, the influence of noise becomes a serious problem because there are many unknown parts in the properties of target molecules and measurement systems and the noise signal is large.

An object of the present invention is to provide an identification method which can identify appropriately non-conforming data from a measurement data set, for example, can contribute to improve the reliability of measurement results by an advanced sensing device, a classification analysis method which can perform the classification analysis with high accuracy for measurement data, an identification device, a classification analysis device, a storage medium for identification and a storage medium for classification analysis.

## <MEANS TO SOLVE THE PROBLEMS>

In view of the above problems, the present invention focuses on a machine learning method for learning a classifier from a positive example set and an unknown set. For example, there is used a classifier constituted by a PU classification method suitable for binary classification of positive example / negative example (Classification of Positive and Unlabeled Examples). Therefore, this is an invention made based on the knowledge that non-conforming data can be identified with high accuracy from a measurement pattern. a classifier constituted by. Details of the PU classification method are described in Non-Patent Document 3.

A first form of the present invention is the classification analysis method comprising the steps of

introducing a sample containing an analyte into a measurement space, obtaining pulse signal data detected due to said introduction,

obtaining data to be analyzed through removing said nonconforming data detected due to elements other than said analyte from said pulse signal data, and

performing a classification analysis of said data to be analyzed by execution of a computer control program, wherein

a nonconforming data storage means is included for storing said nonconforming data identified by the identification method according to claim 1,

said computer control program includes a classification analysis program that performs said classification analysis using the machine learning,

a feature value is obtained in advance which indicates a feature of waveform form of said pulse signal,

said feature value obtained in advance is set as the learning data for said machine learning,

said feature value obtained from said pulse signal of said data to be analyzed removed said nonconforming data is set as a variable, and

said classification analysis on said analyte is performed by executing said classification analysis program.

The feature value is one or more selected from a group of

a wave height value of the waveform in a predetermined time width,

a pulse wavelength ta,

a peak position ratio represented by ratio tb/ta of time ta and tb leading from the pulse start to the pulse peak,

a kurtosis which represents the sharpness of the waveform,

a depression representing the slope leading from the pulse start to the pulse peak,

an area representing total sum of the time division area dividing the waveform with the predetermined times,

an area ratio of sum of the time division area leading from the pulse start to the pulse peak to the total waveform area,

a time inertia moment determined by mass and rotational radius when the mass is constructive to said time division area centered at the pulse start time and the rotational radius is constructive to time leading from said center to said time division area,

a normalized time inertia moment determined when said time inertia moment is normalized so as that the wave height becomes a reference value,

a mean value vector whose vector component is the mean value of the same wave height position in which the wave form is equally divided in the wave height direction and the mean value of time values is calculated for each division unit in before and after each pulse peak,

a normalized mean value vector which is normalized so as that the wavelength becomes a standard value for said mean value vector,

a wave width mean value inertia moment determined by mass distribution and rotational center when the mass distribution is constructive to mean value difference vector whose vector component is mean value difference of the same wave height position in which the wave form is equally divided in the wave height direction and the mean value of time values is calculated for each division unit in before and after each pulse peak and the rotational center is constructive to time axis of waveform foot,

a normalized wave width mean value inertia moment determined when said wave width mean value inertia moment is normalized so as that the wavelength becomes a standard value,
a wave width dispersion inertia moment determined by mass distribution and rotational center when the mass distribution is constructive to dispersion vector whose vector component is dispersion in which the wave form is equally divided in the wave height direction and the dispersion is calculated from time value for each division unit and the rotational center is constructive to time axis of waveform foot, and
a normalized wave width dispersion inertia moment determined when said wave width dispersion inertia moment is normalized so as that the wavelength becomes a standard value.

The second form of the present invention is the identification device comprising

a means for introducing a sample containing an analyte into a measurement space,
a means for obtaining pulse signal data detected due to said introduction, and
a means for identifying nonconforming data detected due to elements other than said analyte from said pulse signal data by execution of a computer control program,
wherein
said computer control program includes an identification analysis program using a machine learning to learn a classifier that classifies positive and negative examples from positive example data of a positive example set and unknown data of an unknown set in which either positive or negative example is unknown,
when type 1 data of a pulse signal are obtained under first measurement condition measured by introducing a sample not containing an analyte in said measurement space and type 2 data of a pulse signal are obtained under second measurement condition measured by introducing a sample containing an analyte in said measurement space, a storage means is included for storing said type 1 data and said type 2 data, and
said nonconforming data included in said type 2 data are identified by executing said identification analysis program, by using said type 1 data as said positive example data and said type 2 data as said unknown data.

The classification analysis device furthermore comprises a

means for introducing a sample containing an analyte into a
measurement space,

a means for obtaining pulse signal data detected due to said introduction,
a means for obtaining data to be analyzed through removing said nonconforming data detected due to elements other than said analyte from said pulse signal data, and
a means for performing a classification analysis of said data to be analyzed by execution of a computer control program,
wherein
a nonconforming data storage means is included for storing said nonconforming data identified by the identification method according to claim 4,
said computer control program includes a classification analysis program that performs said classification analysis using the machine learning,
a feature value is obtained in advance which indicates a feature of waveform form of said pulse signal,
said feature value obtained in advance is set as the learning data for said machine learning,
said feature value obtained from said pulse signal of said data to be analyzed removed said nonconforming data is set as a variable, and
said classification analysis on said analyte is performed by executing said classification analysis program.

The feature value is one or more selected from a group of

a wave height value of the waveform in a predetermined time width,
a pulse wavelength ta,
a peak position ratio represented by ratio tb/ta of time ta and tb leading from the pulse start to the pulse peak,
a kurtosis which represents the sharpness of the waveform,
a depression representing the slope leading from the pulse start to the pulse peak,
an area representing total sum of the time division area dividing the waveform with the predetermined times,
an area ratio of sum of the time division area leading from the pulse start to the pulse peak to the total waveform area,
a time inertia moment determined by mass and rotational radius when the mass is constructive to said time division area centered at the pulse start time and the rotational radius is constructive to time leading from said center to said time division area,

a normalized time inertia moment determined when said time inertia moment is normalized so as that the wave height becomes a reference value,

a mean value vector whose vector component is the mean value of the same wave height position in which the wave form is equally divided in the wave height direction and the mean value of time values is calculated for each division unit in before and after each pulse peak,

a normalized mean value vector which is normalized so as that the wavelength becomes a standard value for said mean value vector,

a wave width mean value inertia moment determined by mass distribution and rotational center when the mass distribution is constructive to mean value difference vector whose vector component is mean value difference of the same wave height position in which the wave form is equally divided in the wave height direction and the mean value of time values is calculated for each division unit in before and after each pulse peak and the rotational center is constructive to time axis of waveform foot,

a normalized wave width mean value inertia moment determined when said wave width mean value inertia moment is normalized so as that the wavelength becomes a standard value,

a wave width dispersion inertia moment determined by mass distribution and rotational center when the mass distribution is constructive to dispersion vector whose vector component is dispersion in which the wave form is equally divided in the wave height direction and the dispersion is calculated from time value for each division unit and the rotational center is constructive to time axis of waveform foot, and

a normalized wave width dispersion inertia moment determined when said wave width dispersion inertia moment is normalized so as that the wavelength becomes a standard value.

According to the first form, the classification analysis method comprises the steps of introducing a sample containing an analyte into a measurement space, obtaining pulse signal data detected due to said introduction, obtaining data to be analyzed through removing said nonconforming data detected due to elements other than said analyte from said pulse signal data, and performing a classification analysis of said data to be analyzed by execution of a computer control program.

In addition, a nonconforming data storage means is included for storing nonconforming data identified by the identification method according to the first form, said computer control program includes a classification analysis program that performs said classification analysis using the machine learning, a feature value is obtained in advance which indicates a feature of waveform form of said pulse signal, said feature value obtained in advance is set as the learning data for said machine learning, said feature value obtained from said pulse signal of said data to be analyzed removed said nonconforming data is set as a variable, and said classification analysis on said analyte is performed by executing said classification analysis program.

Therefore, in this embodiment, it is possible to perform the classification analysis on the analyte with high accuracy by the data to be analyzed from which the nonconforming data identified with high accuracy by the classifier based on the PU classification method according to the first embodiment is removed.

Each feature value described above is a feature value derived from the waveform form of the pulse signal, and by using one or more feature values in these feature value groups, the classification analysis by the machine learning can be performed with higher accuracy.

In the present embodiment, the classification analysis is not limited to the case where the classification analysis is performed using at least one or more selected from the feature value group, but the combination analysis of two or more selected from the feature value group can be performed.

According to the second form, the identification device comprises a means introducing a sample containing an analyte into a measurement space, a means obtaining pulse signal data detected due to said introduction, and a means identifying nonconforming data detected due to elements other than said analyte from said pulse signal data by execution of a computer control program.

In addition, said computer control program includes an identification analysis program using a machine learning to learn a classifier that classifies positive and negative examples from positive example data of a positive example set and unknown data of an unknown set in which either positive or negative example is unknown, when type 1 data of a pulse signal are obtained under first measurement condition measured by introducing a sample not containing an analyte in said measurement space and type 2 data of a pulse signal are obtained under second measurement condition measured by introducing a sample containing an analyte in said measurement space, an storage means is included for storing said type 1 data and said type 2 data, and said nonconforming data included in said type 2 data can be identified by executing said identification analysis program, by using said type 1 data as said positive example data and said type 2 data as said unknown data.

Therefore, in the present embodiment, a classifier based on the PU classification method can be configured to identify the nonconforming data included in the pulse signal obtained as a result of measurement with high accuracy. For example, it is possible to provide an identification device contributing to that the reliability of the measurement result by the advanced sensing device can be improved in performance.

In particular, since the classifier in the present embodiment can be configured with a nonconforming data set collected in the past and an actually measured data set in which positivity or negativity is unknown without using the specific properties to knowledge and problem on the object, it has the excellent removal performance of nonconforming data that cannot be achieved by the conventional method of identifying by simple signal strength, and has a wide applicability to analysis of various measurement data.

The classification analysis device comprises a means introducing a sample containing an analyte into a measurement space, a means obtaining pulse signal data detected due to said introduction, a means obtaining data to be analyzed through removing said nonconforming data detected due to elements other than said analyte from said pulse signal data, and a means performing a classification analysis of said data to be analyzed by execution of a computer control program.

In addition, a nonconforming data storage means is included for storing nonconforming data identified by the identification method according to the fourth form, said computer control program includes a classification analysis program that performs said classification analysis using the machine learning, a feature value is obtained in advance which indicates a feature of waveform form of said pulse signal, said feature value obtained in advance is set as the learning data for said machine learning, said feature value obtained from said pulse signal of said data to be analyzed removed said nonconforming data is set as a variable, and said classification analysis on said analyte can be performed by executing said classification analysis program.

Therefore, in the present embodiment, the classification analysis apparatus can be provided which can perform a classification analysis on the analyte with high accuracy from the data to be analyzed from which the nonconforming data identified with high accuracy by the classifier based on the PU classification method according to the fourth embodiment is removed.

Each feature value described above is a feature value derived from the waveform form of the pulse signal, and by using one or more feature values in these feature value groups, the classification analysis by the machine learning can be performed with higher accuracy.

In the present embodiment, the classification analysis is not limited to the case where the classification analysis is performed using at least one or more selected from the feature value group, but the combination analysis of two or more selected from the feature value group can be performed.

## <EFFECTS OF THE INVENTION>

According to the present invention, by using a computer terminal, it is possible to be utilized to an information compression technology of DNA storage media and a drug discovery using an artificial base pairing, or to measure a fine dust mixed in a measurement sample or an analysis substance contained in. a body fluid, and it is possible to perform the data analysis with high accuracy in the field of technology in identification and removal of nonconforming data caused by fine substances such as red blood cells, white blood cells, and platelets.

## <BRIEF DESCRIPTION OF THE DRAWINGS>

FIG. 1 is a schematic diagram schematically showing a measurement system for measuring the measurement data to be analyzed in the embodiment according to the present invention, and a diagram showing an example of a waveform of a pulse signal measured by the measurement system.

FIG. 2 is a diagram showing a waveform example of a pulse signal measured for DNA constituent molecules by the measurement system.

FIG. 3 is an outlined block diagram showing the schematic configuration of the classification analysis device as an embodiment of the present invention.

FIG. 4 is a diagram showing an outline of processing contents that can be executed by the identification classification analysis program of the PC 1 used in the embodiment.

FIG. 5 is a flowchart showing the identification processing by the PC1.

FIG. 6 is a table showing a list of 22 kinds of classifier software used for verification of identification accuracy according to the present invention.

FIG. 7 is a diagram showing a wave height vector used in the embodiment.

FIG. 8 is a diagram showing a wavelength direction time vector used in the embodiment.

FIG. 9 is a diagram for explaining an outline of processing procedure used in a learning algorithm of PU method.

FIG. 10 is a diagram showing main analysis contents in the PU method.

FIG. 11 is a schematic explanatory diagram summarizing the processing contents of a classifier based on the PU method.

FIG. 12 is a flowchart showing a classification process of a binary classifier due to the PU method in the identification process.

FIG. 13 is a diagram showing a histogram of pulse peak wave heights obtained from an identification experiment for verifying the identification accuracy of the identification method according to the present invention.

FIG. 14 is a diagram showing an F-Measure histogram obtained from the identification experiment.

FIG. 15 is an outlined side sectional diagram showing the schematic configuration of the micro ▪ nanopore device.

FIG. 16 is a diagram showing a processing program configuration necessary for explaining the classification analysis process that can be executed by PC 1 of the classification analysis device.

FIG. 17 is a diagram showing examples of pulse waveforms obtained by particle passage actually measured for Escherichia coli (E. coli) and Bacillus subtilis (B. subtilis) as examples.

FIG. 18 is a pulse waveform diagram for explaining various types of feature value according to the present invention.

FIG. 19 is a diagram for explaining the Karman filter.

FIG. 20 is a diagram for explaining each factor of the Kalman filter with actual measured current data.

FIG. 21 is a diagram showing the details of the repetition of the prediction (8 A) and update (8 B) of the Kalman filter.

FIG. 22 shows a flowchart showing the BL estimation process based on the BL estimation process program.

FIG. 23 is a waveform diagram of the bead model used for factor adjustment of the Kalman filter.

FIG. 24 is an enlarged diagram of a periphery of through-hole 12 schematically showing a state in which Escherichia coli 22 and Bacillus subtilis 23 are mixed in the electrolytic solution 24.

FIG. 25 is a table showing the number of pulses picked up from the waveform of the bead model according to the combination of m, k, and $\alpha$ of the adjustment factors.

FIG. 26 is a flowchart showing the outline of contents of the execution process of the feature value extraction program.

FIG. 27 is a flowchart showing the particle type distribution estimating process.

FIG. 28 is a diagram showing each feature value (15 A) relating to one waveform data and an image diagram (15 B) of a probability density function in the particle types of Escherichia coli and Bacillus subtilis.

FIG. 29 is an image diagram of a superposition of probability density distributions obtained from each particle type of Escherichia coli and Bacillus subtilis.

FIG. 30 is an image diagram showing the relationship between the total number of particles of k particle types, the appearance probability of particle type, and the expected value of appearance frequency of the entire data.

FIG. 31 is a diagram for explaining the derivation process of the constrained logarithmic likelihood maximization formula that performs optimization by the Lagrange undetermined multiplier method.

FIG. 32 is a flowchart showing the data file creation process.

FIG. 33 is a flowchart showing the estimation process of the probability density function.

FIG. 34 is a flowchart showing the particle number estimation process.

FIG. 35 is a flowchart showing the particle number estimation process by Hasselblad iteration method.

FIG. 36 is a flowchart showing the processing procedure by the EM algorithm.

FIG. 37 is a diagram showing an example of result analyzed by the number analyzing function according to the present embodiment.

FIG. 38 is a table showing each estimation result data of a verification example using a pulse wavelength and a wave height as the feature value and a verification example using a pulse wavelength and a peak position ratio as the feature value.

FIG. 39 is a table showing each estimation result data of the verification example using the spread of the peak vicinity waveform and the pulse wavelength as the feature value and the verification example using the spread of the peak vicinity waveform and the wave height as the feature value.

FIG. 40 is a diagram showing the number estimation result of the kurtosis and the pulse wave height as the feature value.

FIG. 41 is a table obtained by the BL estimation process based on the BL estimation process program.

FIG. 42 is a histogram showing each number estimation result when the mixing ratios of E. coli and B. subtilis are 1:10, 2:10, 3:10, and 35: 100, respectively.

FIG. 43 is a histogram showing the number estimation results when the mixing ratios of E. coli and B. subtilis are set to 4: 10, 45: 100, 1: 2, respectively.

FIG. 44 is a diagram combining dispersed states of respective particles when the pulse width and the pulse wave height are used as the feature values.

FIG. 45 is a diagram combining dispersed states of respective particles which show the relationship between the spread of the peak vicinity waveform around the peak and the pulse kurtosis as the feature value, the spread of peak vicinity waveform and the peak position ratio as the feature value, and the spread of peak vicinity waveform and the pulse wave height as the feature value.

FIG. 46 is a diagram which shows an example of waveform of detection signals obtained by using the micro'nanopore device 8 when three types of particles 33a, 33b, and 33c pass through the through-hole 12 and shows a deriving example of the probability density function obtained based on the feature values.

FIG. 47 is a pulse waveform diagram for explaining the feature values of the depression and the area.

FIG. 48 is a diagram for explaining the manner obtaining the wave height vector.

FIG. 49 is a diagram for explaining the relationship between the d-dimensional wave height vector and the data sampling.

FIG. 50 is a pulse waveform diagram for explaining the feature value of the second type with respect to the time (wavelength) and the wave width.

FIG. 51 is a diagram for explaining the relation between the wave width vector of dw dimension and the data sampling.

FIG. 52 is a diagram for explaining the acquiring process of acquiring the inertia moment with respect to the wave width from the wave width vector.

FIG. 53 is a diagram for explaining an example of the waveform vector for creating the feature value in the case divided into a plurality of directions.

FIG. 54 is a flowchart showing the processing contents of feature value extraction.

FIG. 55 is an estimation evaluation table relating to the combinations of the feature value when the sampling is performed at 1 MHz and 500 kHz.

FIG. 56 is an estimation evaluation table relating to the combinations of the feature value when the sampling is performed at 250 kHz and 125 kHz.

FIG. 57 is an estimation evaluation table relating to the combinations of the feature value when the sampling is performed at 63 kHz and 32 kHz.

FIG. 58 is an estimation evaluation table relating to the combinations of the feature value when the sampling is performed at 16 kHz and 8 kHz.

FIG. 59 is an estimation evaluation table relating to the combinations of the feature value when the sampling is performed at 4 kHz.

FIG. 60 is an estimation evaluation table relating to the combinations of the feature value with respect to all sampled data.

FIG. 61 is an estimation evaluation table relating to the combinations of the feature value when the sampling with high density is performed at 1 MHz to 125 kHz.

FIG. 62 is an estimation evaluation table relating to the combinations of the feature value when the sampling with low density is performed at 63 kHz to 4 kHz.

FIG. 63 shows a graph between the sampling frequency and the weighted mean relative error (weighted average) with respect to the combination of the top five types of feature values that can obtain the high number estimation accuracy when all the sampling data are used (50A) and when the sampling is performed with high density (50B).

FIG. 64 shows a graph (51A) between the sampling frequency and the weighted mean relative error (weighted average) with respect to the combination of the top five types of feature values that can obtain the high number estimation accuracy when the sampling is performed with low density, and a graph (51B) between the sampling frequency and the weighted average relative error (weighted average) with respect to the combination of the four types of feature values when all the sampling data are used.

FIG. 65 shows a graph (52A) between the sampling frequency (kHz) and the necessary calculation time (seconds) showing the total calculation time of the calculation time required for the feature value creation and the calculation time required for iterative calculation by Hasselblad method for each combination of four types of feature values, and a graph (52B) between the sampling frequency (kHz) and the necessary calculation time (seconds) showing the calculation time required for the feature value creation for each combination of the feature value.

FIG. 66 is a graph between the sampling frequency and the necessary calculation time (second) showing the calculation time required for iterative calculation by Hasselblad method for each combination of four types of feature values.

FIG. 67 is a schematic view for explaining the overview of the classification analysis method according to the present invention.

FIG. 68 is a diagram showing main control processing in the present embodiment.

FIG. 69 is a flowchart showing the classification analysis processing in the present embodiment.

FIG. 70 is a table showing the evaluation results by verification of the classification analysis process and details of an analysis sample in the verification.

FIG. 71 is an explanatory diagram of an F-Measure.

## <BEST MODE FOR CARRYING OUT THE INVENTION>

The classification analysis device according to one embodiment of the present invention will be described below with reference to the drawings. In the present embodiment, there will be explained the base species analysis form for classification analysis of DNA constituent molecule as an example of the analyte.

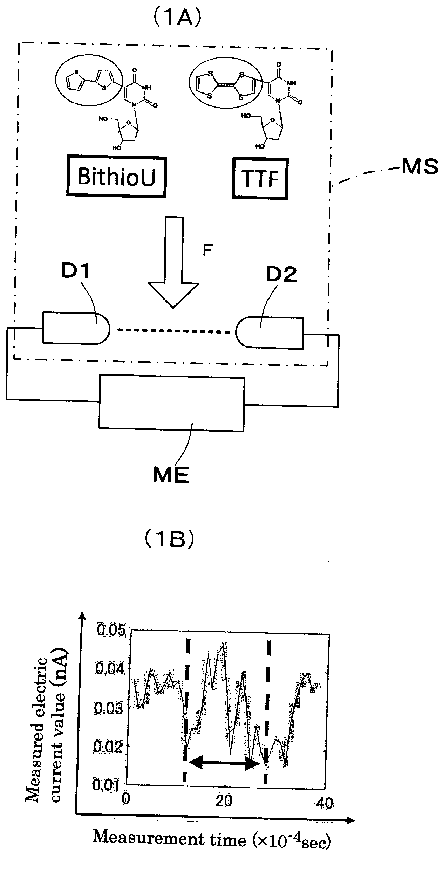

(1A) of FIG. 1 is the schematic diagram schematically showing the measurement system for measuring the measurement data to be the analyzed object in the present embodiment.

The measurement system includes a measurement space MS configured by a storage container that stores a solution containing base molecules, and a pair of fine-shaped electrodes D1 and D2 that are arranged to face each other in the measurement space MS. The electrodes D1 and D2 are nanogap electrodes formed of gold (Au) element which are arranged at a minute distance from each other. The fine distance is formed to be about 1 nm. The measurement sample in the measurement space MS is a solution sample containing a solvent (pure water) and DNA constituent molecules mixed in the solvent.

As described in Non-Patent Document 4, the nanogap electrode is a device expected as a next-generation DNA sequencer. This electrode is an electrode gap having a very fine gap created by using a technique called mechanical fracture joining. When a constant voltage is applied to the electrode gap, a current due to the quantum mechanical tunnel effect (tunnel current: see the broken line in FIG. 1) flows when the substance passes through the gap. This tunnel current is measured by the current measuring device ME as a pulse current at the moment when the substance passes.

By measuring the tunnel current pulse due to this nanogap electrode, it is possible to identify the type of DNA base molecule in single molecule units, and to identify the amino acid sequence of a peptide and a modified amino acid molecule that becomes a disease marker, which was difficult with existing technology. In the measurement system of FIG. 1, using a nanogap electrode (D1, D2) having an electrode gap of about 1 nm, the data of a pulse signal detected by measuring a tunnel current pulse flowing due to one molecule passing near the electrode is set as an analysis target.

As the molecules to be measured, two kinds of dithiophene uracil derivatives (hereinafter abbreviated as BithioU) and TTF uracil derivatives (hereinafter abbreviated as TTF), which are artificial nucleobases, were used. These molecules are obtained by chemically modifying an epigenetic site (an acquired modification site where DNA methylation or the like occurs) for easy identification. As indicated by the arrow F, as the driving force source for allowing DNA molecules to pass through the vicinity of the gap, in addition to the Brownian motion of the molecules themselves, those by electrophoresis, electroosmotic flow, or dielectrophoresis can be used.

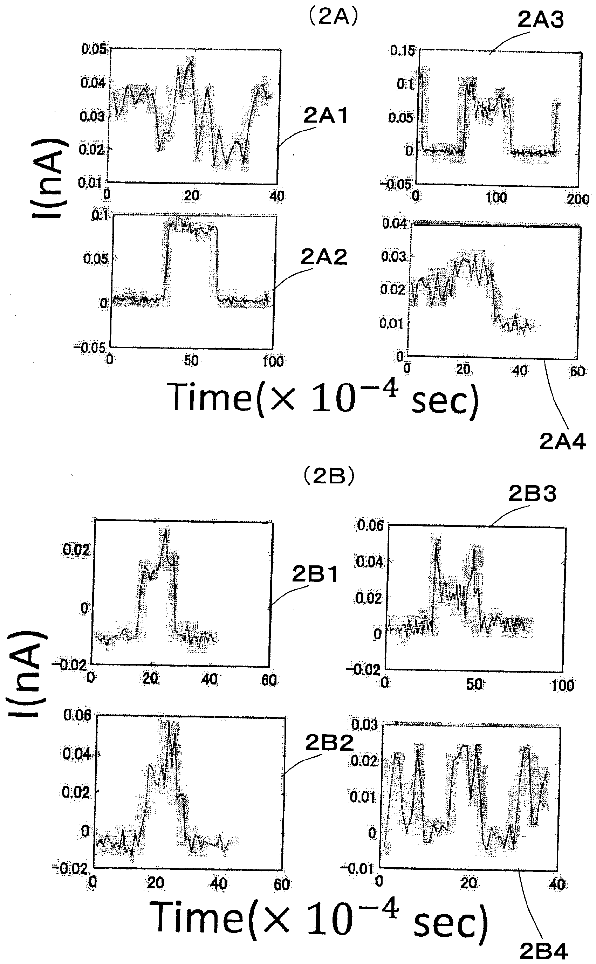

FIG. 1 (1B) and FIG. 2 show examples of the waveform of the pulse signal measured by the measurement system of FIG. 1. In these figures, the horizontal axis represents measurement time ($\times 10^{-4}$ sec), and the vertical axis represents measured electric current value (nA).

As shown in (1B), the pulse determination part of the pulse signal is a 1/3 part of the center of the measurement waveform, and this pulse waveform data is used as the data to be analyzed.

2A1 and 2A2 in (2A) show examples of waveform when the base molecule BithioU is detected. 2B1 and 2B2 in (2B) show examples of waveform when the base molecule TTF is detected. 2A3, 2A4 of (2A) and 2B3, 2B4 of (2B) show examples of noise waveform mixed when a base molecule is detected.

In the measurement system of FIG. 1, one base molecule of DNA is measured and detected as a current pulse. The measured pulses include not only those derived from base molecules but also current pulses due to fluctuations of metal atoms on the electrode surface and impurities (see 2A3, 2A4 of (2A) and 2B3, 2B4 of (2B) in FIG. 2). Because of these noise pulses, there is a possibility that a pulse originally derived from a base may be missed, or conversely, it may be erroneously determined that a base molecule pulse was measured even though it was a noise pulse, and as a result, it becomes difficult to identify molecules. The present invention can appropriately identify and remove noise pulse nonconformed data from the measured pulse waveform data set, and can enable classification analysis of base types with high accuracy.

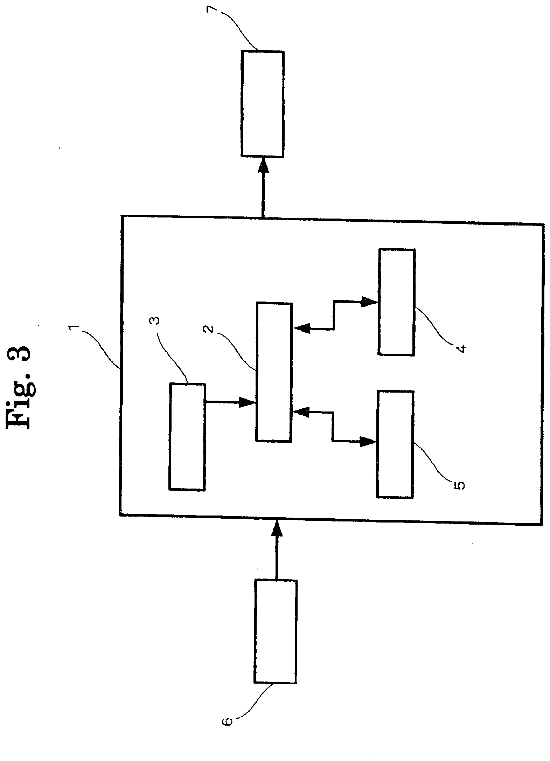

FIG. 3 shows the outlined constitution of the classification analysis device according to the present embodiment. This classification analysis device is constituted by the personal computer 1 (hereinafter referred to as a PC), and the PC 1 has CPU 2, ROM 3, RAM 4 and the data file storage portion 5. The ROM 3 stores the computer control program. The computer control program includes various processing programs such as the identification / classification analysis program for performing the identification processing of nonconforming data and the classification analysis using the machine learning, and the program for creating the feature values required in the identification / classification analysis. Various processing programs such as the classification analysis program can be installed and stored from the storage medium (CD, DVD, etc.) storing each program. The input means 6 such as the keyboard and the display means 7 such as the liquid crystal display are connected to the PC 1 so as to allow input and output. The data file storage portion 5 can store the analysis data.

The PC 1 has the identification processing function of nonconforming data and the classification analysis processing function. However, in the present invention, the PC 1 can be configured by two dedicated terminals having separately the identification processing function and the classification analysis processing function.

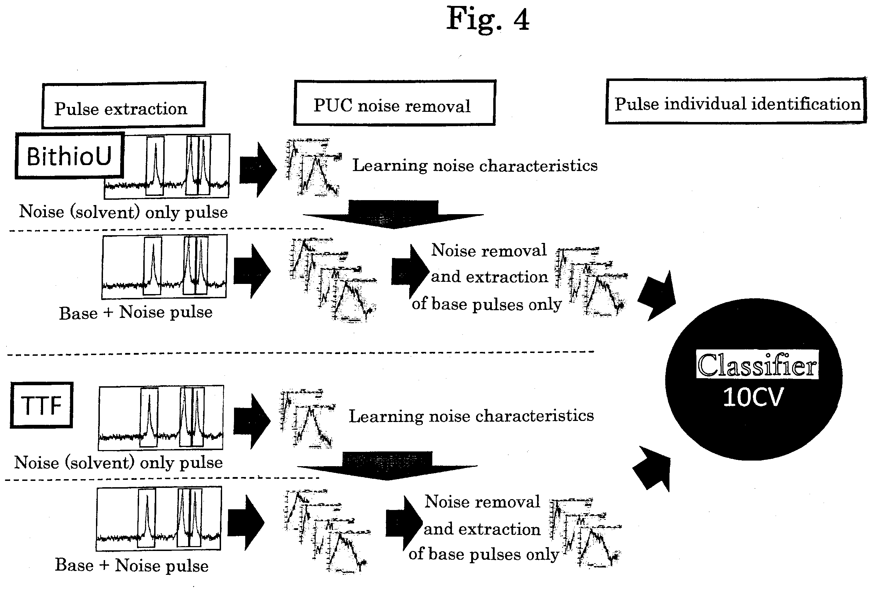

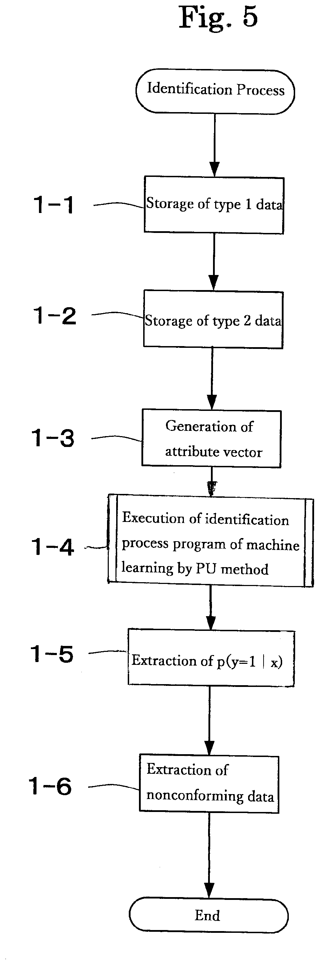

FIG. 4 shows an outline of processing contents that can be executed by the identification / classification analysis program (computer control program) of the PC 1. FIG. 5 is a flowchart of the identification processing of the PC 1.

The identification processing of the PC 1 is performed by the processing procedure based on the following identification method. The identification / classification analysis program includes the identification processing program based on the PU method using a machine learning to learn a classifier that classifies positive and negative examples from positive example data of a positive example set and unknown data of an unknown set in which either positive or negative example is unknown,

(Process 1-1) Type 1 data of a pulse signal obtained under first measurement condition measuring by introducing a sample (solvent only) not containing an analyte (DNA constituent molecule) in the measurement space MS is taken into and stored in the RAM 4 of the storage means.

(Process 1-2) Type 2 data of a pulse signal obtained under second measurement condition measuring by introducing a sample (solvent and DNA constituent molecule) containing an analyte (DNA constituent molecule) in the measurement space MS is taken into and stored in the RAM 4 of the storage means.

(Process 1-3) In order to match with the input format of the identification processing program, the attribute vectors of the type 1 data and the type 2 data are created.

(Process 1-4) The identification processing program is executed with the type 1 data as the positive example data and the type 2 data as the unknown data.

(Process 1-5) The probability $p$ ($s = 1 \mid x$) is extracted and obtained by executing the identification processing program. The probability data is stored and saved in a predetermined area of the RAM 4. Note that the attribute vector used in the analysis by the following PU method is based on multidimensional data and is represented by a vector, but in the following description, the vector notation is particularly omitted.

(Process 1-6) Based on the probability, nonconforming data detected due to elements other than the analyte (fluctuations and impurities of metal atoms on the electrode surface, not from the base molecule mentioned above) included in the type 2 data is detected and identified. The detected nonconforming data is stored and saved in a predetermined area of the RAM 4.

The classifier software of machine learning platform freeware Weka disclosed in Non-Patent Document 2 can be used for the identification processing program.

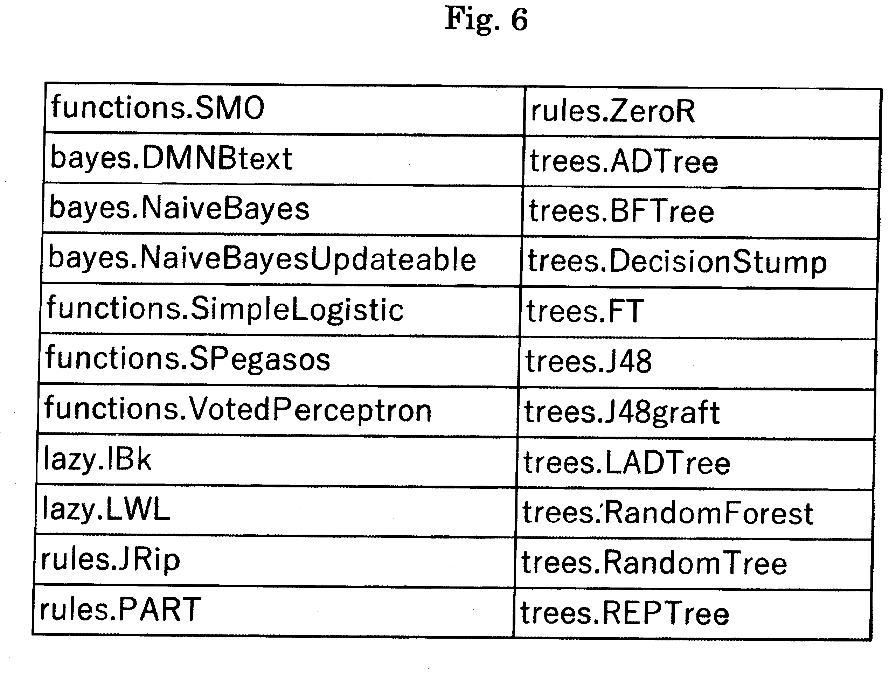

FIG. 6 is the list of 22 kinds of classifier software used for verification of identification accuracy according to the present invention. Any of the 22 types can be used as the identification processing program and can be stored in the ROM 3. Both the calculation of $p$ ($s = 1 \mid x$) in the PU method and the identification processing after noise data removal by the PU method were verified using a Weka program.

The measured pulse waveform data varies in both wavelength and wave height, so it is necessary to use the attribute vectors with uniform dimensions as input in order to identify the base type by the machine learning classifier. When the machine learning processing program is executed, as the preprocessing matching with the input format, the preprocessing that performs a kind of coarse-graining and creates the attribute vector reflecting the pulse waveform is performed in (processing 1-1) and (processing 1-2).

The measured pulse waveform data varies in both wavelength and wave height, so it is necessary to use the attribute vectors with uniform dimensions as input in order to identify the base type by the machine learning classifier. When the identification processing program is executed, as the preprocessing matching with the input format, the preprocessing that performs a kind of coarse-graining and creates the attribute vector reflecting the pulse waveform is performed in (processing 1-3).

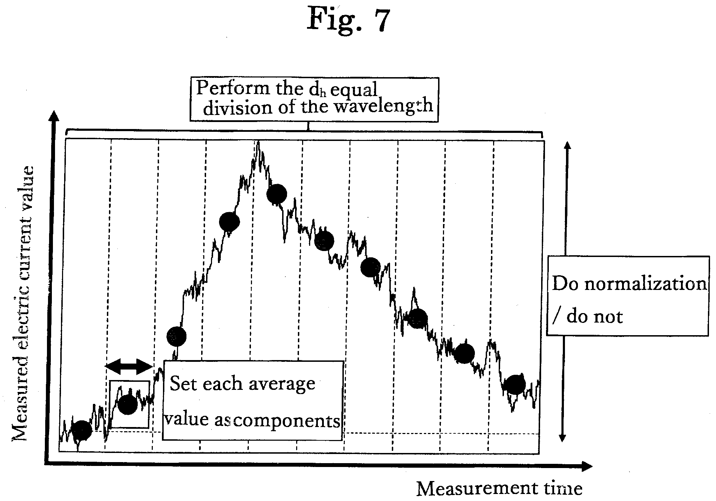

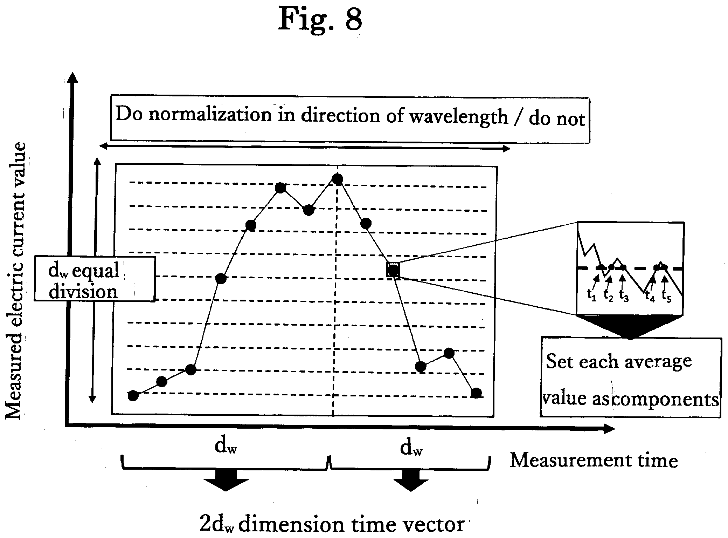

FIG. 7 shows the wave height vector. FIG. 8 shows the wavelength direction time vector.

As shown in FIG. 7, the measurement pulse waveform is divided by dh in the wavelength direction, the mean value of the measured electric current values is calculated for each divided section, and the dh-dimensional attribute vector using these values as components is used as the wave height vector. Two types of attribute vectors are created as one normalized in the wave height direction and one not normalized.

As shown in FIG. 8, when the measured current values are divided into two groups before and after the peak of the pulse and then divided by dw in the wave height direction, the measured electric current value of the pulse is divided into 2dw groups. The mean value of the number of steps from the pulse start time is calculated for each division section, and the 2dw-dimensional wavelength direction time vector having these values as components is created. In addition, it is also created a normalized wavelength direction time vector that has been normalized so that the time from the pulse start point to the end point is "1". In addition to the above described wave height vector and wavelength direction time vector, an attribute vector simply linking them is also created. These vector data are stored in a predetermined area of the RAM 4.

In the present embodiment, in order to configure a binary classifier, two feature values of wave height and wavelength are used. In the verification experiment for verifying the identification accuracy of nonconforming data according to the present embodiment, the verification was performed using the following eight attribute vectors V1 to V8 created from one pulse waveform data.

(V1) Pulse height vector (hvNrmd) with the pulse peak value normalized to "1"

(V2) Unnormalized wave height vector (hvRaw)
(V3) Wavelength direction time vector (wvNrmd) in which the pulse wavelength time is normalized to "1"
(V4) Unnormalized wavelength direction time vector (wvRaw)
(V5) (dh + 2dw) dimensional vector connecting V1 and V2
(V6) (dh + 2dw) dimensional vector connecting V1 and V4
(V7) (dh + 2dw) dimensional vector connecting V2 and V3
(V8) (dh + 2dw) dimensional vector connecting V2 and V4

In the verification experiment, the above eight attribute vectors are created, and their identification accuracies are compared. The number of division at the time of creating the attribute vector is set to be dh = 10 and dw = 5 uniformly after the preliminary analysis.

In the case of a normal binary classifier, the classifier is generated by learning from data in which the positive examples and the negative examples are given. On the other hand, in the present embodiment, since the nonconforming data are mixed in the measurement data, the classifier based on the PU method is used. As described in detail in Non-Patent Document 3, the PU method used in the present embodiment is a kind of learning algorithm semi-supervised for learning from positive examples and unlabeled data and performing binary classification of positive examples / negative examples. The outline of the processing procedure of the learning algorithm of the PU method stored in the ROM 3 is as follows.

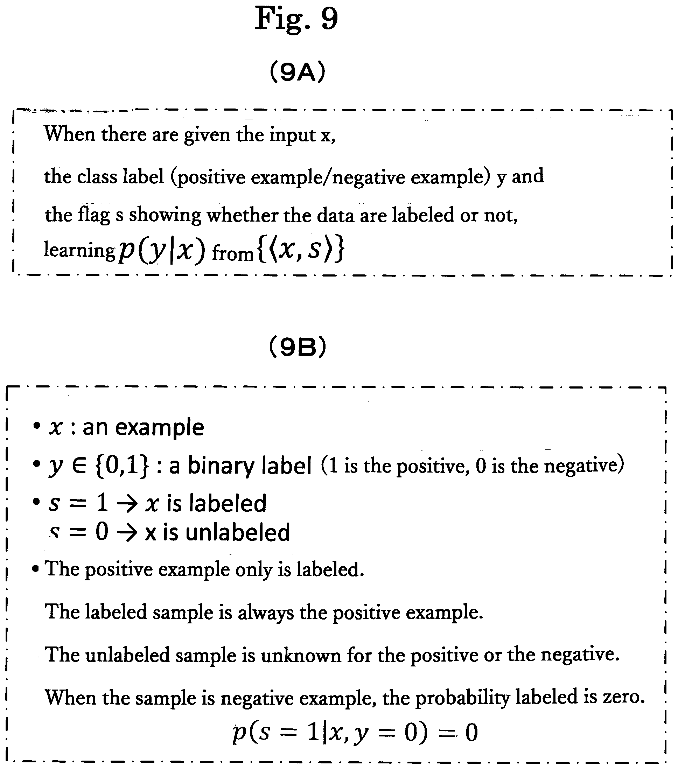

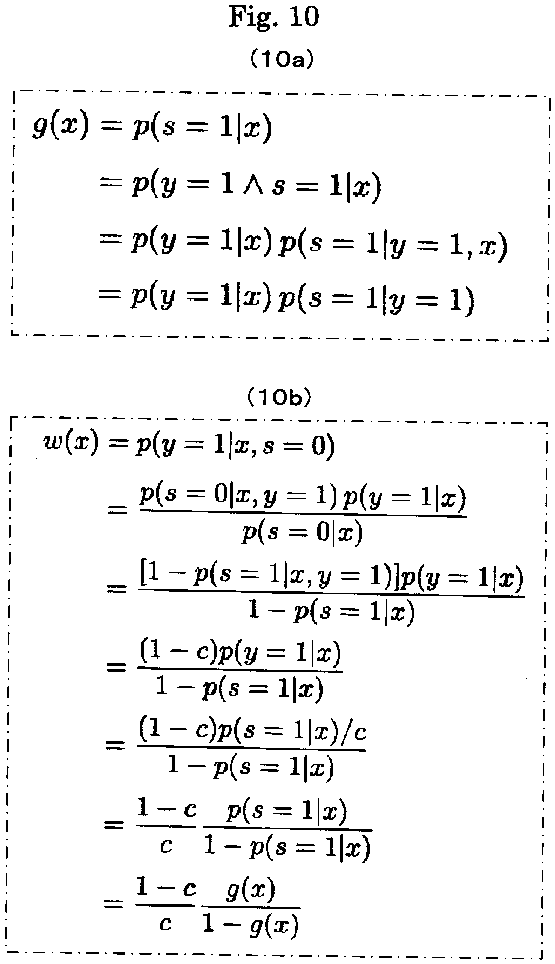

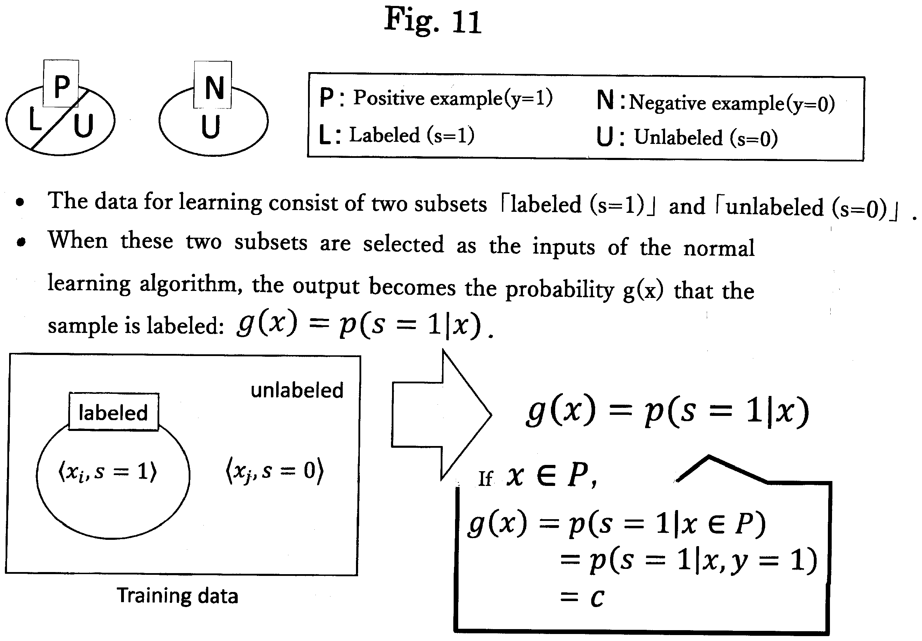

FIG. 9 is the diagram for explaining the outline of processing procedure used in a learning algorithm of PU method. FIG. (9A) shows the variables and the label flags used for learning, and FIG. (9B) shows details of the preconditions of (9A). FIG. 10 is the diagram showing main analysis contents in the PU method. FIG. 11 is the schematic explanatory diagram summarizing the processing contents of the classifier based on the PU method as explained below. In FIG. 11, the positive example set and the negative example set are P and N, respectively, where P includes a labeled subset L and an unlabeled subset U, and N includes only U.

Case x (input data) is an attribute vector related to a pulse waveform, y is its class label, and s is a flag indicating whether or not a class label is attached to the case. In the set of input cases, only a part of positive examples (y = 1) is labeled (s = 1), and other positive examples and all negative examples (y = 0) are not labeled (s = 0). That is, if the sample is a negative example, the probability of being labeled is zero and p (s = 1 | x, y = 0) = 0. Using such a case set as an input to the learning algorithm of the binary classifier, the probability g (x) = p (s = 1 | x) that the sample is labeled can be obtained. Furthermore, since what is originally desired to get is not g (x) but p (y = 1 | x), p (y = 1 | x) is extracted by applying the following correction.

In all cases set, G (x) = p (s = 1 | x) showing the probability that a sample is labeled, is derived to the relation of g (x) = p (y = 1 | x) p (s = 1 | y = 1) by the derivation process shown in FIG. 10 (10a). If c = p (s = 1 | y = 1), the probability that the sample is a positive example is given as p (y = 1 | x) = g (x) / c.

Here, the probability of labeling in the positive example set is uniformly random, that is, it is assumed that p (s = 1 | y = 1, x) = p (s = 1 | y = 1) = c is constant regardless of x. Assumes value. This is because the measurement data handled is not arbitrary data that is intentionally biased.

Here, the constant c can be estimated as follows. If it is labeled to be uniformly random in the positive example set, g (x) matches the proportion of labeled example set contained in the positive case when x is positive, and g (x) = p (s = 1 | y = 1) = c holds.

Therefore, by using g (x) obtained by the normal binary classifier that does not depend on the PU method, c can be estimated as the mean in the labeled example set L that is a positive example (c given in the following equation 1).

[Equation 1]

$$c = \frac{1}{n} \sum_{x \in P} g(x)$$

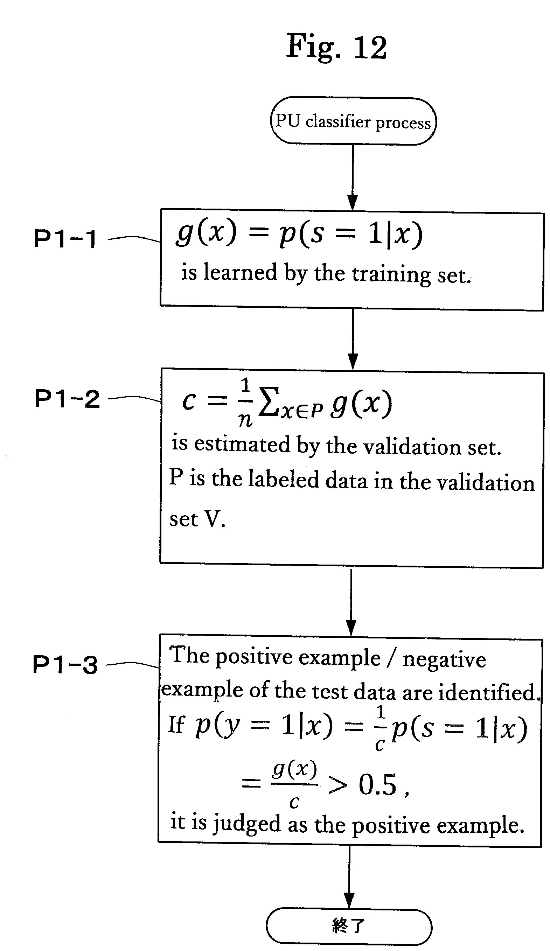

FIG. 12 shows the identification process of the binary classifier by the PU method in (Process 1-5).

In process P1-1, the process of learning g (x) from the learning data set is performed. Next, in process P1-2, the process of estimating c from the verification data set based on Equation 1 is performed. In process P1-3, the process of identifying positive / negative examples for test data is performed by g (y = 1 | x) determined from the relationship g (y = 1 | x) = g (x) / c. Is called. The judgement criterion in this case can be set as g (y = 1 | x)> 0.5.

The present invention can extract the probability that the unlabeled example is the positive example in the configuration of the classifier based on the PU method shown in FIG. 11. The extraction procedure for extracting the probability that the unlabeled example is the positive example will be described below.

All labeled examples are positive, but unlabeled examples can be either positive or negative. If the probability that the unlabeled example is the positive example is w (x), the probability that it is the negative example is 1 -w (x). Therefore, all unlabeled examples are duplicated, one is treated as the positive example, and the other is treated as the negative example. A weight w (x) is given to the unlabeled example x treated as the positive example, and the weight "1-w (x)" is given to the unlabeled example x treated as the negative example. Since all labeled examples cases are positive examples, they are treated as the positive examples with the weight "1". The classifier is created by using these weighted examples set as learning data.

Here, assuming that c and g (x) = p (s = 1 | x) are obtained by the method shown in FIGS. 9 to 11, the probability w (x) that the unlabeled example is the positive example becomes w (x) = (1-c) g (x) / (c (1-g (x))) by the derivation process shown in (10b) of FIG. 10, and by extraction of c and g (x), it is possible to obtain the probability w (x) that the unlabeled example is the positive example.

The verification experiment of the identification accuracy according to the present embodiment will be described below.

From a set of measurement pulses measured using the nanogap electrode with the DNA constituent molecule as an analyte, 1) First, the classifier based on the PU method is constructed as the pre-processing, and the noise-derived pulses (nonconforming data) are identified (see FIG. 12). The extracted nonconforming data were removed from the type 2 data, and the data set of only pulses derived from the base was obtained. 2) The base type identification accuracy was evaluated for the pulse set derived from the base.

For noise removal, the tunnel current pulses measured with the nanogap electrode are obtained in advance only for a solvent that does not contain the base (BithioU, TTF). This pulse set is the pulse derived from noise not relating to the base and is referred to as "noise pulse set". Next, the current pulse measured for the solvent mixed with the base BithioU is obtained. The TTF is acquired in the same manner. This pulse set includes both the "base pulse" derived from the base and the noise pulse. Therefore, this is called "base + noise pulse set".

Since the pulse in the noise pulse set is always the noise pulse, it is regarded as the positive example set (data set of the first data) and on the other hand, the pulse in the base + noise pulse set is unknown which pulse it is, so it is considered as the unlabeled example set. By the PU classifier process shown in FIG. 12, it is possible to identify the noise pulse (positive example) and the base pulse (negative example), and by removing the noise data that is the positive example, it is possible to obtain the set (base pulse set) consisted essentially of only the base pulse.

The PU classifier processing is used only once to classify the positive and negative examples of the noise pulse and base pulse, and no problem due to overlearning will occur, so the PU classifier is created using the entire pulse set as the learning data, and therefore, the classification analysis was performed to separate all pulse sets into the noise pulses and the base pulses. In this way, the BithioU base pulse set was obtained from the BithioU base + noise pulse set by the PU classifier, and the TTF base pulse set was obtained from the TTF base + noise pulse set by the PU classifier.

In order to evaluate the identification accuracy for the base pulse and the base + noise pulse, the identification experiment of the base type using the normal binary classifier was performed for the base pulse set of BithioU and TTF from which noise data was removed and separated. In the identification experiment, when either of the number of base pulses of the two kinds of bases was less than 10, it was excluded from the experiment object because there were too few learning examples.

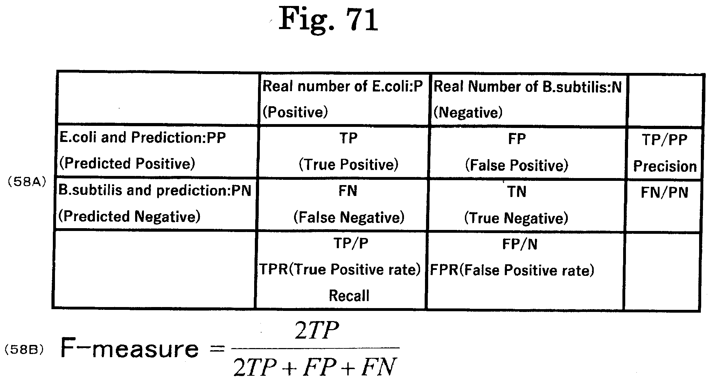

F-measure (F-scale shown in FIG. 71 described later) was used as an index of identification accuracy, and the accuracy was evaluated by 10-fold cross validation (hereinafter abbreviated as 10 CV). At 10 CV, the number of base pulses of BithioU and TTF was set to the same number. That is, when the number of base pulses of BithioU and TTF obtained by the PU classifier is NB and NT, respectively, the number of base pulses used for 10 CV is set to N = min (NB, NT) for both BithioU and TTF. For a base pulse set having a pulse number greater than N, N base pulses were randomly extracted.

In addition, in order to see the effect of noise removal by the PU classifier, the identification experiment of BithioU and TTF was also performed on the base + noise pulse set before performing the noise data removal. The accuracy evaluation was performed by 10 CV on the pulse set obtained by random extraction of N components from the base + noise pulse set for each of BithioU and TTF as in the case of the base pulse.

The experimental conditions for the verification experiment are described below.

With the machine learning software shown in FIG. 6, the identification accuracy was examined by using of 22 kinds of classifiers under various analysis conditions.

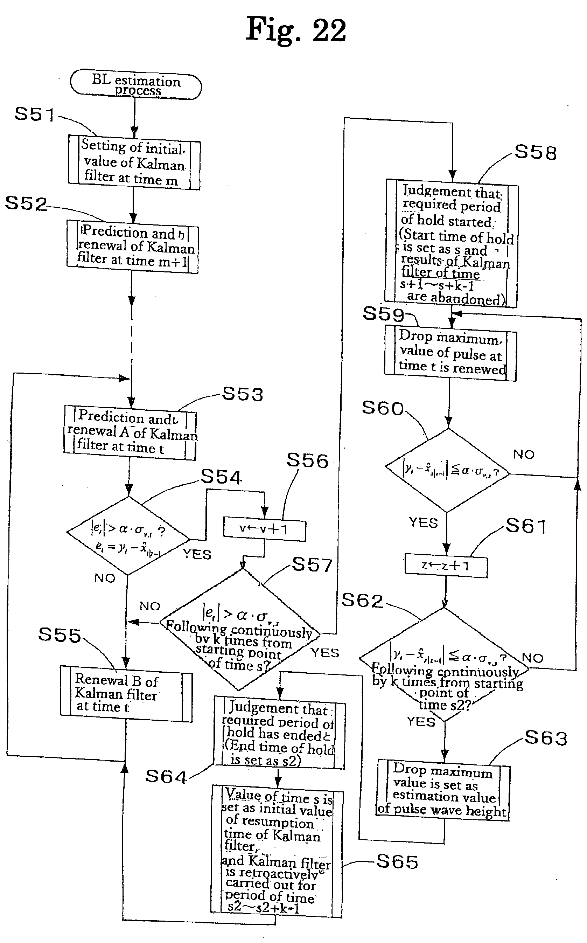

As a pulse extraction parameter, the two parameters of $\alpha$ and k (the adjustment factor shown in FIG. 22 described later) are used. When extracting a pulse, the former is the wave height threshold value $\alpha$ indicating how much the measured current value deviates from the baseline for determining to be the pulse start, and the latter is the wavelength threshold value k indicating how many steps exceed the pulse height threshold for determining to be the pulse. Various tests were performed on these parameters, and the experiment was performed on "4 types for the wave height threshold value α × 4 types for the wavelength threshold value k, that is 16 types in total". As the attribute vector, eight types of attribute vectors V1 to V8 were tested.

The ensemble learning "Rotation Forest" is adopted as the classifier for the classification analysis, and as base classifier used internally, there are used the 22 types of classifiers among those implemented in Weka, which can perform the binary classification of the input example continuous value vector. As the PU method, two methods of g (x) and w (x) shown in FIG. 10 were used.

The identification experiment of nonconforming data conducted under the above experimental conditions were performed for 3272 cases where the number of base pulses was 10 or more for both bases, while selecting from all combinations given by the pulse extraction parameter 16 types × the attribute vector 8 types × 22 classifiers implemented in Weka × PU method 2types. In this identification experiment, for the sake of simplicity, the conditions used for these three parties of 1) BithioU noise removal, 2) TTF noise removal, and 3) 2-base identification after noise removal are all common, where the above conditions consist of the pulse extraction parameter, the attribute vector, the classifier, and the PU classifier method. Under the same conditions, the identification experiment was also performed on the base + noise pulse set without using the noise removal by the PU classifier.

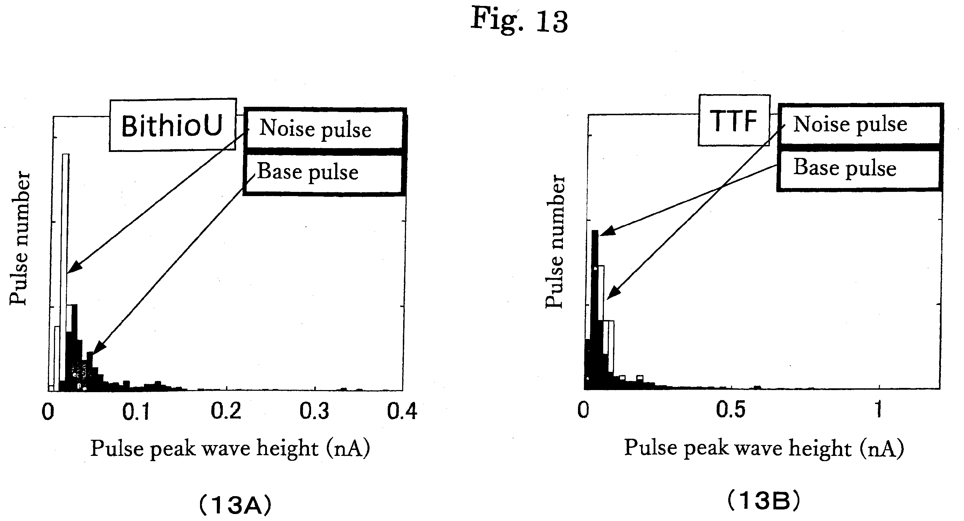

FIG. 13 shows the histogram of pulse peak wave heights for the pulses determined to be the base and the pulses determined to be noise which are the examples of F-measure> 0.9 obtained from 3272 cases (measured pulse set used under the analysis condition where F-measure was 0.93). The horizontal axis represents the pulse peak wave height (nA), and the vertical axis represents the number of pulses. (13A) shows the histogram of noise pulses and base pulses for BithioU, and (13B) shows the histogram of noise pulses and base pulses for TTF. In (13A), the noise pulses and the base pulses are distributed in wave height ranges of 0 to 0.3 and 0.02 to 0.4, respectively. In (13B), the noise pulses and the base pulses are distributed in wave height ranges of 0 to 0.2 and 0 to 1.2, respectively.

As can be seen from FIG. 13, the histogram of the pulse peak wave height includes many overlapping portions between the noise pulse and the base pulse, and it is found that it is difficult to identify the noise pulse and the base pulse by only the pulse peak wave height.

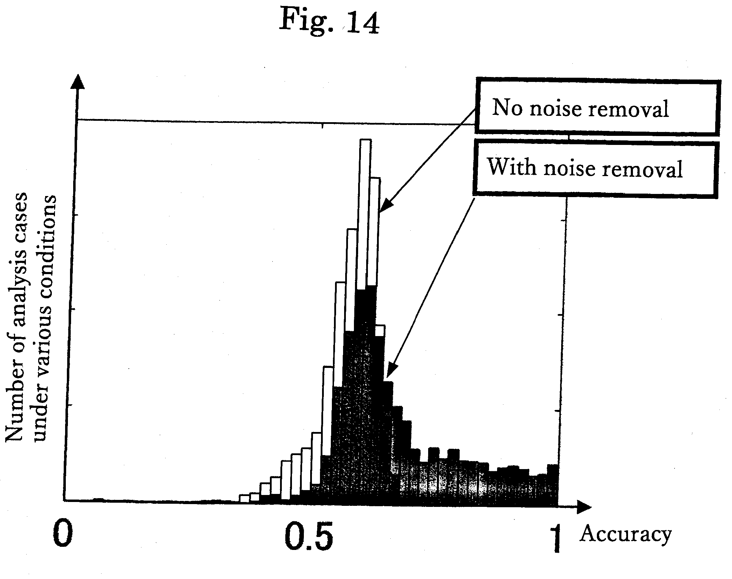

FIG. 14 shows the F-Measure histogram obtained for 3272 cases with and without noise removal, respectively. The horizontal axis represents the base identification accuracy, and the vertical axis represents the number of analysis cases under various conditions with and without noise removal. In the cases of no noise removal and noise removal, the distribution is in the accuracy ranges of 0.3 to 0.6 and 0.5 to 1.0, respectively. The total number of analysis cases is 3272 of various combinations of pulse extraction parameter, attribute vector and classifier.

As can be seen from FIG. 14, in the case of no noise removal and the case of noise removal, although the overlapping portion is large, the identification accuracy is improved to an accuracy close to 100% to 100%. Therefore, the identification processing performance of nonconforming data by PC1 can obtain the high base classification accuracy by removing the noise pulses appropriately and by using the attribute vector of the feature quantity that accurately grasps the pulse waveform characteristics, even when it is difficult to determine the base / noise only by the pulse peak height.

The classification analysis device by the PC 1 has the classification analysis function with high accuracy for the data to be analyzed from which the nonconforming data identified by above identification processing is removed. This classification analysis function includes the following analysis procedures.

(C1)The nonconforming data detected by the above identification processing which is caused by elements other than the analyte are stored in a predetermined area of the RAM 4 of the nonconforming data storage means. Not only the nonconforming data is detected and stored by the PC 1, but also a nonconforming data file stored in advance in an external terminal may be introduced and stored in the PC 1.

(C2) The data group obtained by removing the stored nonconforming data from the pulse signal data detected by introducing the sample containing the analyte into the measurement space is stored in a predetermined area of the RAM 4 as the data to be analyzed. The data group from which the nonconforming data have been removed in advance may be introduced into the PC 1 and stored as the data to be analyzed.

(C3) The computer control program installed in the PC 1 includes the classification analysis program for performing the classification analysis using machine learning, and is stored in the ROM 3.

(C4) The feature value is obtained in advance which indicates the feature of waveform form of the pulse signal, the feature value obtained in advance is set as the learning data for the machine learning, the feature value obtained from the pulse signal of the data to be analyzed removed the nonconforming data is set as the variable, and the classification analysis on the analyte can be performed by executing the classification analysis program.

By the classification analysis device through the PC 1, the classification analysis on the analyte can be performed with high accuracy based on the data to be analyzed, because the feature value is obtained in advance which indicates the feature of waveform of pulse signal, the feature value obtained in advance is set as the learning data for machine learning, the nonconforming data identified with high accuracy by the classifier based on the PU classification method are removed from the data to be analyzed, the feature quantity obtained from the data to be analyzed removed the nonconforming data is set as the variable, and the classification analysis on the analyte is performed by executing the classification analysis program.

In the present embodiment, since it is possible to perform the identification of the nonconforming data with high accuracy and the classification analysis, for example, a way to enable the identification of artificial bases is opened, and it is possible to develop applications in the information compression technology for DNA storage media and the pharmaceutical creation using artificial base pairs.

The present invention is not limited to the electric current output waveform in the embodiment described above and can be applied to the identification of nonconforming data and the classification analysis for a wide range of output waveforms such as a voltage waveform and an impedance waveform.

The present invention is not limited to the detection waveform obtained by the measurement system using the nanogap electrode, and can be applied to the detection waveform due to the measurement system of a microstructure equivalent to the sample object through which the sample object passes, such as a through hole, a well (concave), a pillar (convex), and a flow path. The applicable range of measurement data in the present invention is all of time series measurement data, and is not limited to electrical measurement, and can include detection data of physical phenomena such as optical measurement and sound.

The removal target caused by the elements other than the analyte in the present invention is not limited to the quantum level elements in the embodiment described above, and for example, can be applied to signals based on elements other than the analyte existing in a solution, a measuring instrument and a measuring device. That is, the present invention can be applied to the identification / removal technology of the nonconforming data caused by for example, fine dust mixed in a measurement sample and minute substances such as red blood cells, white blood cells and platelets in the case where measurement object is blood.

The feature value used in the identification processing and the classification analysis according to the present invention is not limited to the above-described wave height and wavelength, and various feature values derived from the waveform form can be used. The present inventors have come up to grasp the effective feature values from analysis of waveform data that appears in particle detection technology using a micro / nanopore device. The particle detection technique using a micro / nanopore device is disclosed in Patent Document 1 and the like. Hereinafter, the feature values effective for the present invention and the classification analysis processing when using them will be described in detail.

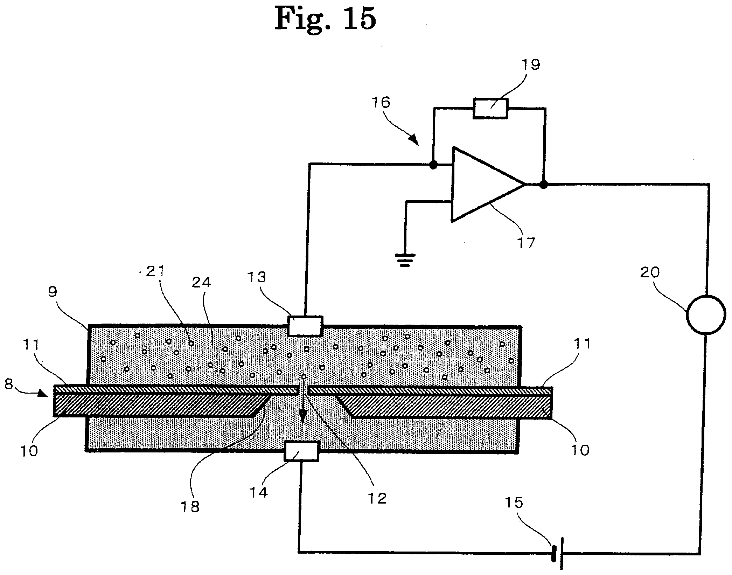

FIG. 15 shows the outlined constitution of the particle detection device using the micro nanopore device 8.

The particle detection device is constituted by the micro nanopore device 8 and an ionic current detection portion. The micro nanopore device 8 has a chamber 9, a partition wall 11 partitioning the chamber 9 into upper and lower accommodation spaces, and a pair of electrodes 13, 14 arranged on the front and back sides of the partition wall 11. The partition wall 11 is formed on a substrate 10. A small through-hole 12 is formed in the vicinity of the center of the partition wall 11. Below the through-hole 12, a recess portion 18 is formed by removing a part of the substrate 10 downward in a concave shape.

The micro-nanopore device 8 is fabricated using a manufacturing technique (for example, an electron beam drawing method or photolithography) of a semiconductor device or the like. That is, the substrate 10 is made of Si material, and a partition wall 11 made of $Si_3N_4$ film is formed as a thin film on the surface. The recess portion 18 is formed by removing a part of the substrate 10 by etching.

The partition wall 11 is formed by laminating SiN film with 50nm thickness on Si substrate having a size of 10 mm square and a thickness of 0.6 mm. A resist is applied to the $Si_3N_4$ film, and a circular opening pattern having a diameter of 3 $\mu$m is formed on it by an electron beam writing method, and the through-hole 12 is bored. On the back side of the through-hole 12, wet etching with KOH is performed to form a 50 $\mu$m square opening to provide the recess portion 18. The formation of the recess portion 18 is not limited to wet etching, but it can be performed by isotropic etching etc. using the dry etching with $CF_4$ gas or the like, for example.

In addition to the SiN film, the insulating film such as $SiO_2$ film, $Al_2O_3$ film, glass, sapphire, ceramic, resin, rubber, elastomer, or the like can be used for the film of the partition wall 11. The substrate material of the substrate 10 is not limited to Si, and glass, sapphire, ceramic, resin, rubber, elastomer, $SiO_2$, SiN, $Al_2O_3$, or the like can be used.

The through-hole 12 is not limited to the case of forming the thin film on the above substrate, and for example, by attaching a thin film sheet having the through-hole 12 onto the substrate, the partition wall having the through-hole may be formed.

The ionic current detection portion is constituted by an electrode pair of the electrodes 13 and 14, a power supply 15, an amplifier 16, and a voltmeter 20. The electrodes 13, 14 are arranged to face each other through the through-hole 12. The amplifier 16 is constituted by an operational amplifier 17 and a feedback resistor 19. The (-) input terminal of the operational amplifier 17 and the electrode 13 are connected. The (+) input terminal of the operational amplifier 17 is grounded. The voltmeter 20 is connected between the output side of the operational amplifier 17 and the power supply 15. The applied voltage of 0.05 to 1 V can be used between the electrodes 13 and 14 by the power supply 15, but in this embodiment, 0.05 V is applied. The amplifier 16 amplifies the current flowing between the electrodes and outputs it to the voltmeter 20 side. The electrode material of the electrodes 13 and 14 are, for example, Ag / AgCl electrode, Pt electrode, Au electrode or the like, preferably Ag / AgCl electrode can be used.

The chamber 9 is a flowable substance accommodation container which hermetically surrounds the micro nanopore device 8, and can be made of electrically and chemically inert materials such as glass, sapphire, ceramic, resin, rubber, elastomer, $SiO_2$, SiN, $Al_2O_3$, or the like.

An electrolytic solution 24 containing the subject 21 is filled in the chamber 9 from an injection port (not shown). The subject 21 is, for example, an analyte such as a bacterium, a microparticulate substance, a molecular substance or the like. The subject 21 is mixed in the electrolytic solution 24 which is a flowable substance, and the measurement is performed by the micro nanopore device 8. At the end of the measurement by the ion current detection portion, the filling solution can be discharged from the discharge port (not shown). As the electrolytic solution, for example, in addition to phosphate buffered saline (PBS), Tris-EDTA (TE) buffer and dilution solutions thereof, all electrolytic solution agents similar thereto can be used. The measurement is not limited to the case in which it is always performed when the subject-containing electrolytic solution is introduced into the chamber 9 and filled. The subject-containing electrolytic solution (flowable substance) is pumped out from the solution reservoir by a simple pumping device and injected from the injection port into the chamber 9 and discharged from the discharge port after the measurement. Furthermore, new solution is stored in the solution reservoir or another solution reservoir, and newly pumped out to perform next measurement, so that the continuous measurement system can be constituted.

When a voltage from the power source 15 is applied between the upper and lower electrodes 13, 14 of the through-hole 12 in a state where the electrolytic solution 24 is filled in the chamber 9, a constant ion current proportional to the through-hole 12 flows between the electrodes. When a subject such as bacteria etc. existing in the electrolytic solution 24 passes through the through-hole 12, a part of the ion current is inhibited by the subject, so that the pulsed ion current reduction can be measured by the voltmeter 20. Therefore, according to the particle detecting device using the micro'nanopore device 8, by detecting the change in the waveform of the measured current, it is possible to detect each individual presence of the particles contained in the flowable substance by passing through the through-hole 12 for each subject (for example, particle) with high accuracy. The measurement mode is not limited to the case where the measurement is performed while forcibly flowing the flowable substance but can include a case of measurement while flowing the flowable substance non-forcibly.

The measurement output of the ion current by the voltmeter 20 can be externally outputted. The external output is converted into digital signal data (measured current data) by a conversion circuit device (not shown), temporarily stored in a storage device (not shown), and then stored in the data file storage portion 5. Measurement current data acquired in advance by the particle detection device using the micro-nanopore device 8 can be externally input to the data file storage portion 5.

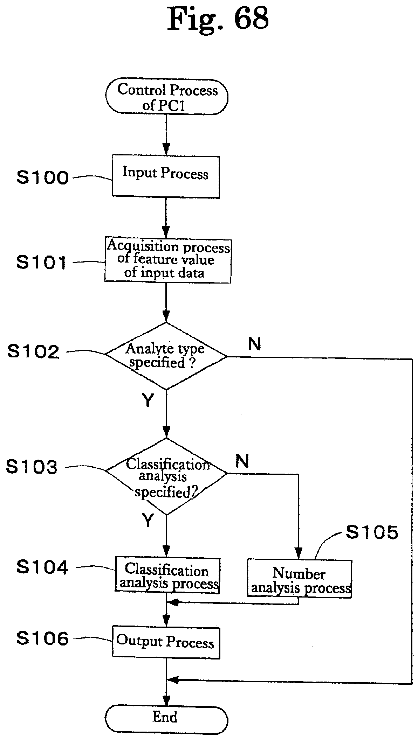

FIG. 68 shows the outlined diagram for explaining the outline of the classification analysis processing on the analyte (for example, Escherichia coli Ec and Bacillus subtilis Bs) based on the PC 1.

The classification analysis processing of FIG. 68 is constituted by the following analysis procedures (a) to (d).

(a) As a result of measurement by the nanopore device 8a on the flowable material containing the predetermined analyte (for example, E. coli Ec or Bacillus subtilis Bs), the pulse signals De and Db corresponding to the passage of analytes through the through-hole 8b are obtained as the detection signals for each type, and the feature values indicating the features of their waveform forms are determined in advance. The pulse signals De and Db are the signals obtained by passing through the through holes 8 b of E. coli Ec and B. subtilis Bs, respectively.

(b) The computer analysis unit 1a incorporates the classification analysis program for performing the classification analysis by the machine learning. The feature values obtained in advance in (a) are the feature values obtained from the known data of E. coli Ec and Bacillus subtilis Bs, and are used in the computer analysis unit 1a as the learning data for the machine learning.

(c) For example, when the mixture mixed in the flowable material with the unknown state on the content ratio or the content number of E. coli Ec and B. subtilis Bs is used as the classified analyte Mb, in the same manner as the case of obtaining known data of (a), the measurement is performed by the nanopore device 8c. By this measurement, a pulse signal Dm is obtained as the analyte data by passage of the classified analyte Mb through the through hole 8d.

(d) Using the feature value based on known data as the learning data and the feature value obtained from the pulse signal Dm of the data to be analyzed as the variable, by executing the classification analysis program , it is possible to perform the classification analysis relating to the predetermined analyte in the data to be analyzed.

According to the classification analysis described above, the classification analysis by the machine learning is performed based on the feature value, and the data to be analyzed of unknown type can be classified into one 1b derived from the passage of E. coli Ec or B. subtilis Bs and one not derived from them. In addition, the feature value according to the present invention may be created in the computer analysis unit 1a, or may be given to the computer analysis unit 1a after being created using another feature value creation program.

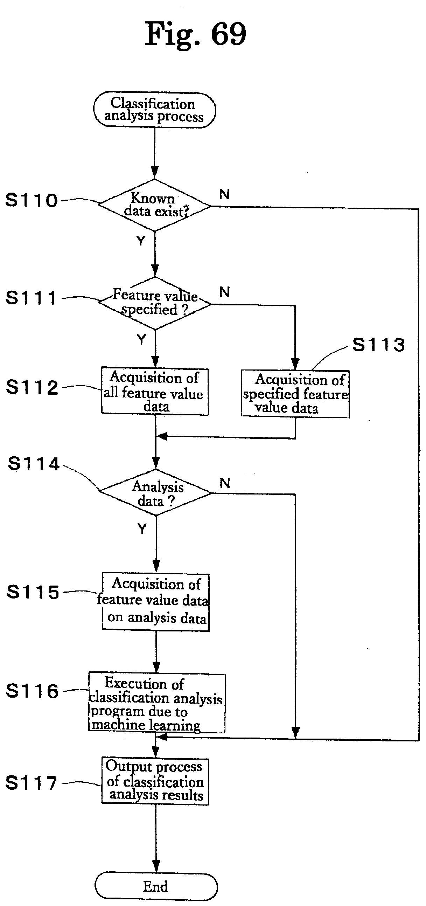

FIG. 69 shows the main control process due to PC 1.

Main control processes include input process (step S100), feature value acquisition process (step S101) for acquiring feature values from input data, classification analysis process (step S104), number analysis process (step S105) and output process (step S106). In the input process (step S100), various inputs necessary for PC operation, start input of built-in program, execution instruction input of various analyses, input of measurement current data and / or feature value data, setting input of output mode, input of the designated feature value when the feature value is designated at the analysis time, and so on. This input process also includes the removal process of the nonconforming data. The classification analysis process (step S104) or the number analysis process (step S105) can be performed (steps S102 and S103) by performing the operation of specifying the analysis type by the input means 6. The classification analysis process can be classified and analyzed using the vector value data of the feature value acquired from the input data in the feature value acquisition process (step S101). The number analysis process can perform the number analysis using scalar data of the feature value acquired from the input data in the feature value acquisition process. The present embodiment is an embodiment having the number analysis process function in addition to the classification analysis process function, but the present invention can be performed by an embodiment having only the classification analysis process function.

The computer control program according to the present embodiment includes a number analysis program for analyzing the number or the number distribution of particle types. In the number analysis process (step S105), the number analysis program can be executed. In the output process (step S106), the analysis result data can be output in the classification analysis process (step S104) and the number analysis process (step S105). For example, various analysis result data are displayed and output on the display means 7. When a printer (not shown) is connected to the PC 1 as the output means, the print output of various analysis result data is possible.

<About the number analysis process>

The classification analysis device according to the present embodiment is configured such that a flowable material (electrolytic solution 24) including one or more types of particles as an analysis target (an example of analyte) is supplied to the upper side surface of the partition wall 11 by execution of the number analysis program. The change of current flow of between the electrodes 13 and 14 is caused by the particles passing through the through-hole 12, and it has an analysis function such that the number or the number distribution of the particle types is analyzed based on the data of detection signal of the change. That is to say, the PC 1 can perform the number analysis process for the measured current data stored in the data file storage portion 5 by executing the number analysis program stored in the ROM 3 under the control of the CPU 2. The number analysis process is configured from the following processes in which the probability density estimation is performed for the data group based on the feature value indicating the feature of the waveform of the pulse signal corresponding to particle passage contained in the detection signals and the automatic analysis of the particle number of each type is performed based on the number analysis method deriving the particle number for each particle type.

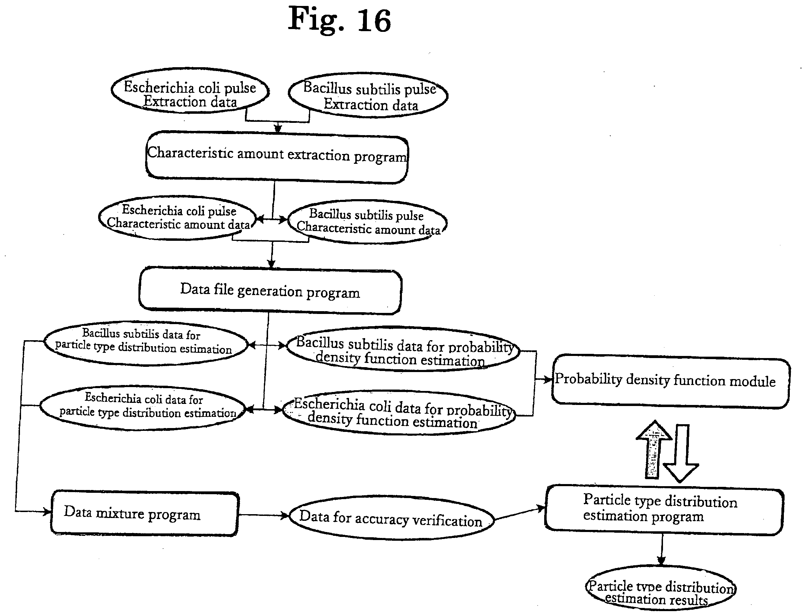

FIG. 16 shows the processing program configuration necessary for explaining the analysis process that can be executed by PC 1. Each process program is stored in the ROM 3. As an example of data of the analysis target, the measured current data (pulse extraction data of each particle) extracted using the electrolytic solution 24 including two types of particles (Escherichia coli and Bacillus subtilis) as analytes is used as original data.

The number analysis program includes the probability density function module program for obtaining the probability density function from the data group based on the feature value indicating the feature of the waveform of the pulse signal corresponding to the particle passing through the through-hole 12 obtained as the detection signal, and the particle type distribution estimation program for deriving the number of each particle type from the result of the probability density estimation. Furthermore, the number analysis program includes the feature value extraction program for extracting the feature value indicating the feature of the waveform of the pulse signal with reference to the baseline extracted from the data group, and the data file creation program for creating the data file due to the pulse feature value data for each particle obtained based on the extracted feature value.

The classification analysis process and the number analysis process are performed for the data created by the data file creation program. The feature value extraction program includes the baseline estimation process program for extracting the baseline from the original measurement current data. In the feature value acquisition process (step S101), the feature value extraction program and the data file creation program are executed to create the feature values from the data input in the input process (step S100), and to store it in the data file for feature value storage of the RAM 4. The input data for classification analysis are known data required to create the feature quantities used as the learning data, and data for analysis (analysis data). The feature value data created from the known data is stored in the feature value storage data file DA due to the known data, and the feature value data created from the analysis data is stored in the feature value storage data file DB due to the analysis data.

When the classification analysis is performed, the analysis process can be performed by acquiring the vector value data of the feature values from the data files DA and DB. The input data for number analysis is only the data for analysis (analysis data). The feature value data created from the input data for number analysis is stored in the data file for number analysis DC, and when performing the number analysis, the scalar data of the feature value is acquired from the data file DC and the analysis process can be performed.

Since the form of the true probability density function is unknown as the premise of particle type distribution estimation, the execution of the probability density function module program performs the nonparametric (not specifying the functional form) probability density estimation called Kernel method. The original data of the estimation target is the pulse appearance distribution data including, for example, a pulse height h, a time width $\Delta t$, an appearance number, etc. obtained from the pulse signals. Each data of the original measurement data distribution is expressed by a Gaussian distribution introducing measurement error uncertainty, and a probability density function is obtained by superimposing each Gaussian distribution. The probability density estimation process is performed by executing the probability density function module program and the original data can be represented by an unknown complex probability density function based on the original data (for example, pulse height, pulse width, appearance probability of the feature value).

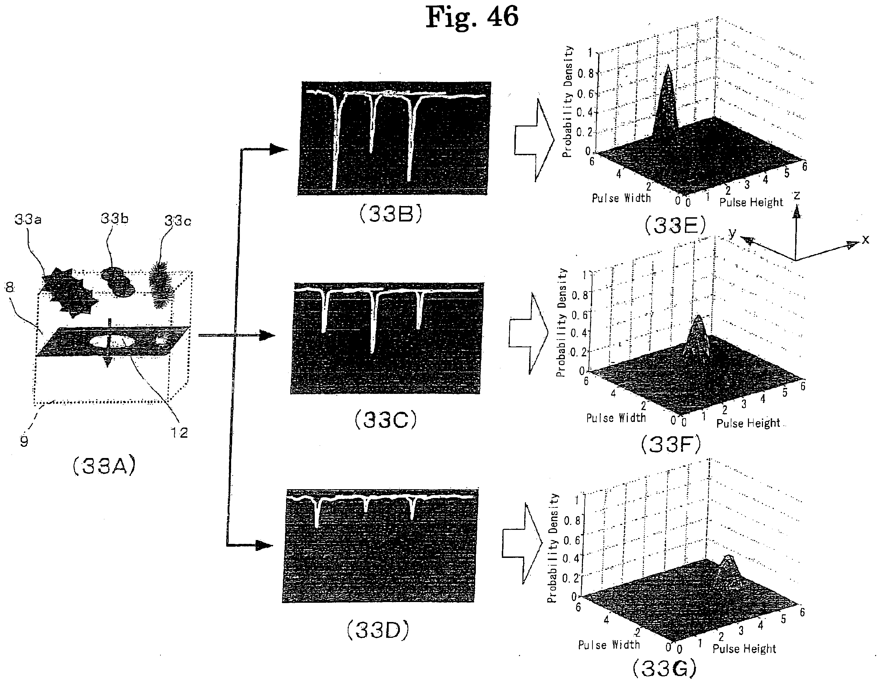

FIG. 46 shows the examples of waveform of detection signals obtained by using the micro-nanopore device 8 when three types of particles 33a, 33b, and 33c pass through the through-hole 12 and shows the deriving examples of the probability density function obtained based on the feature values. FIG. (33A) schematically shows the particle detection device using the micro'nanopore device 8. FIGS. (33B) - (33 D) show the waveform data of each detection signal. FIGS. (33E) to (33G) show the three-dimensional distribution diagram of the probability density function obtained from each waveform data. The x-axis, y-axis, and z-axis in (33E) - (33G) indicate the pulse height and the pulse width of the feature value, and the probability density obtained by the probability density estimation, respectively.

As described above, the probability density estimation process is performed based on the Kernel method which is one of estimation methods of the nonparametric density function. The Kernel method is an estimation method in which a function (Kernel function) at one data point is applied, this is applied to all data points, and the arranged functions are superimposed, which is suitable for obtaining a smooth estimation value.

By executing the probability density function module program and by considering the multivariable multidimensional probability density from data such as pulse wave height, pulse width, etc. of the measured current waveform, the weighted optimum estimation is performed extending to two or more dimensions and the estimation process of the particle type number distribution is performed. EM algorithm software executed based on Hasselblad iteration method is used for the weighted optimum estimation. The EM algorithm is preinstalled in the PC 1. The particle type number distribution result obtained by the estimation process of the particle type number distribution can be output and displayed as the histogram of appearance frequency (number of particles) for each particle type on the display means 7.

The feature value according to the present invention is, as the parameters derived from the pulse signal, either of first type showing local feature of waveforms of said pulse signals and second type showing global feature of waveforms of said pulse signals. By carrying out the number analysis using one or two or more of these feature values, it is possible to analyze the number or number distribution corresponding to the type of analyte such as particle type etc. with high accuracy.

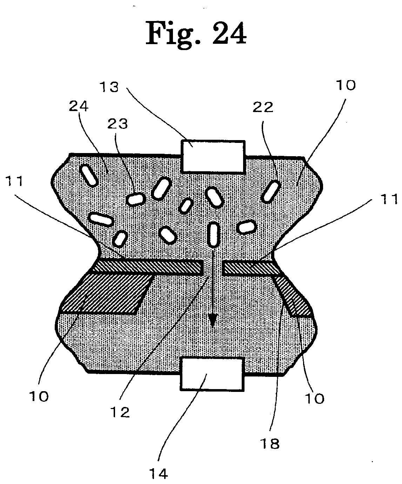

FIG. 24 is the enlarged diagram of a periphery of through-hole 12 schematically showing a state in which two kinds of particles such as Escherichia coli 22 and Bacillus subtilis 23 are mixed in the electrolytic solution 24.

<About Feature Values>

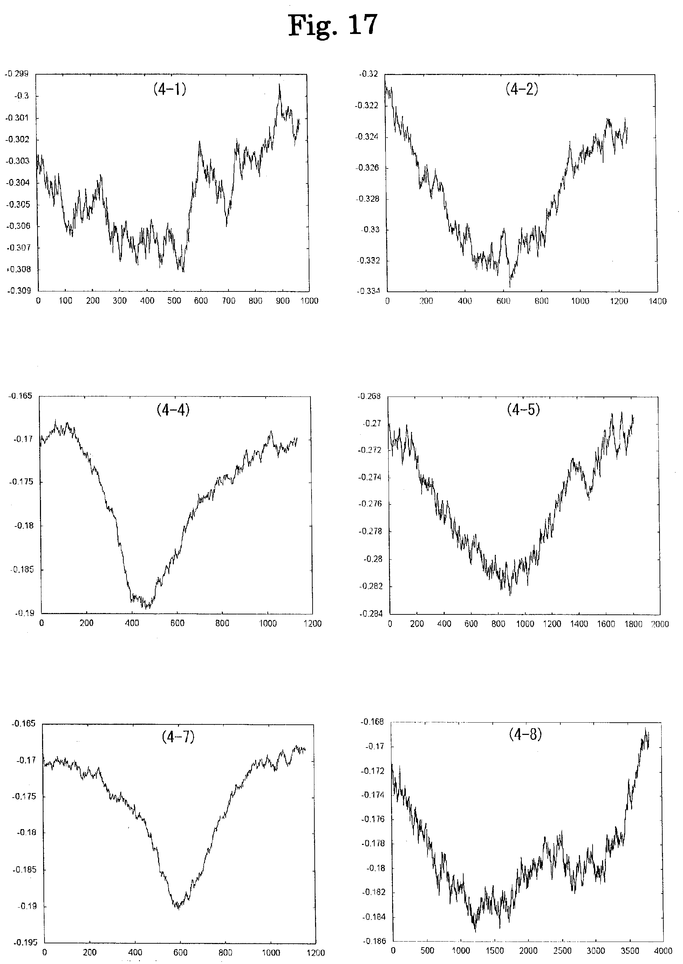

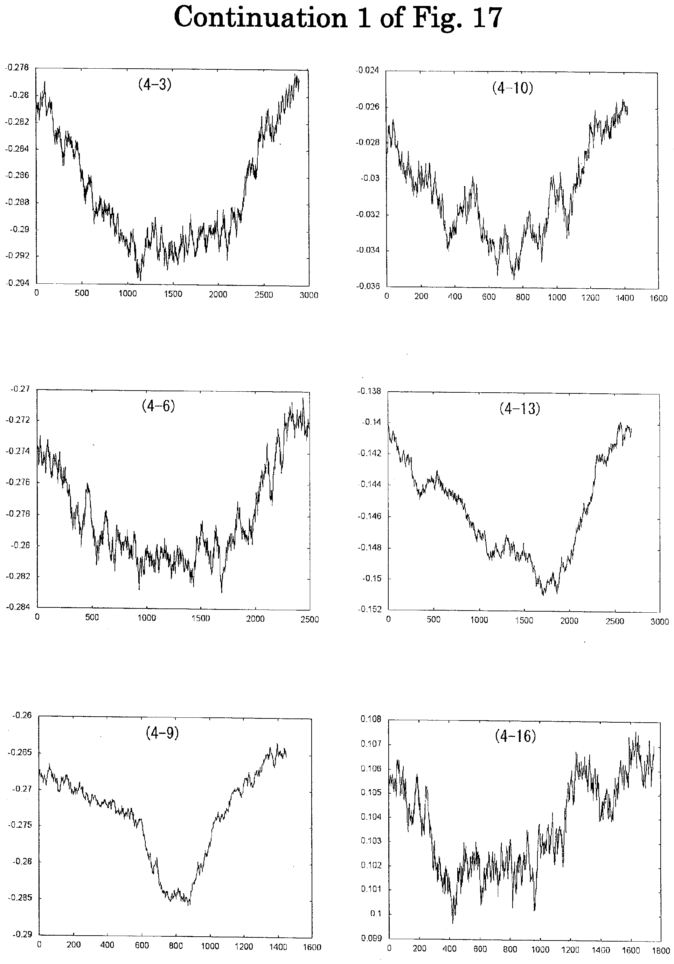

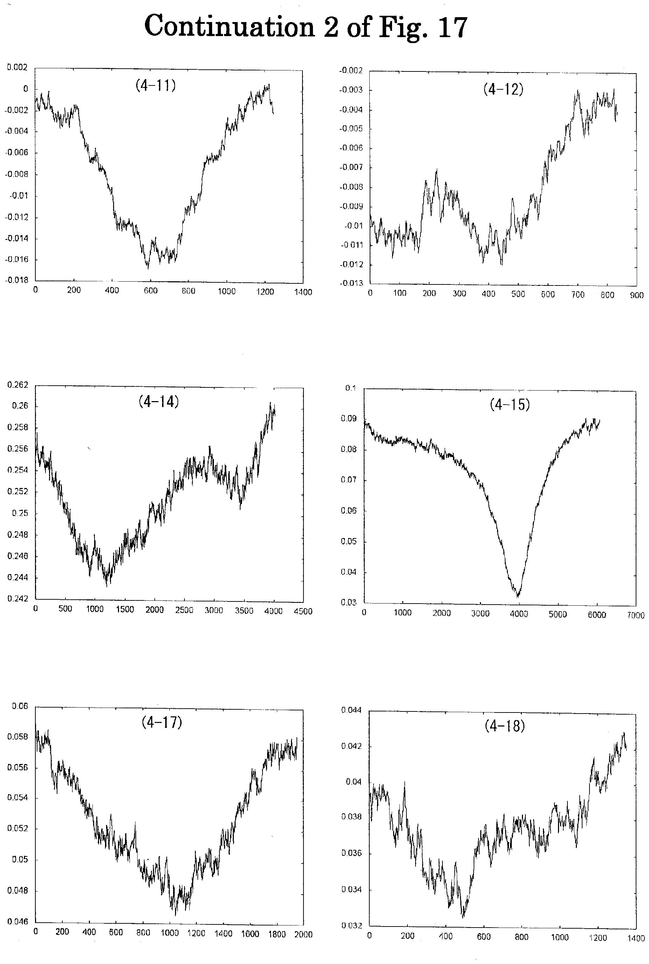

FIG. 17 shows the example of the pulse waveform due to particle passage measured for Escherichia coli (E. coli) and Bacillus subtilis (B. subtilis) in examples. The (4-1) to (4-9) of FIG.17 show the examples (9 kinds) of measured pulse waveforms of E. coli, and (4-10) to (4-18) show the examples of measured pulse waveforms of B. subtilis (9 kinds). When comparing both types in appearance, there is not much difference in the wave height and the wavelength between both types, but there are remarkable differences in attributes of pulse waveform of particle passage such as the peak position and the waveform kurtosis. For example, in the case of Escherichia coli, the peak tends to go ahead with the lapse of time and the waveform is sharp in a whole (waveform kurtosis is large). In the case of Bacillus subtilis, the peak tends to fall down backwards with the lapse of time and the waveform kurtosis is small.

Based upon the difference of the attribute of the pulse waveform form of particle passage described above, the feature values used as a base for creating the probability distribution can be extracted for each particle type (E. coli and B. subtilis) from the pulse waveform data.

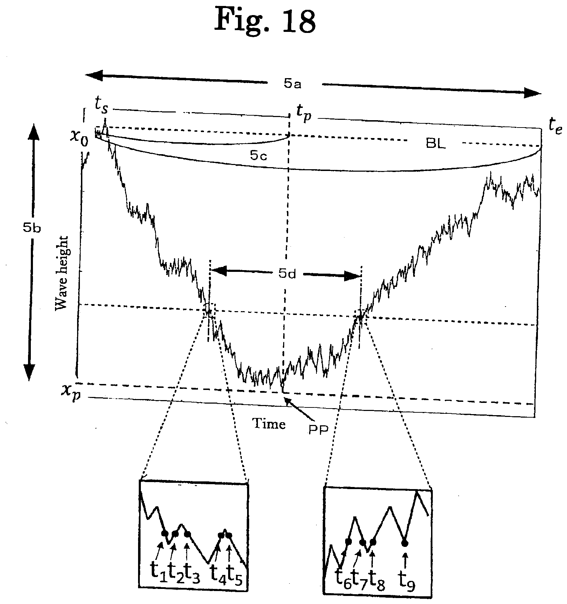

FIG. 18 is the pulse waveform diagram for explaining various types of feature values according to the present invention. In FIG. 18, the horizontal axis shows the time and the vertical axis shows the pulse wave height.

The feature value of the first type is one selected from a group of

the wave height value of the waveform in a predetermined time width,
the pulse wavelength ta,
the peak position ratio represented by ratio tb/ta of time ta and tb leading from the pulse start to the pulse peak,
the kurtosis which represents the sharpness of the waveform, the depression representing the slope leading from the pulse start to the pulse peak,
the area representing total sum of the time division area dividing the waveform with the predetermined times, and
the area ratio of sum of the time division area leading from the pulse start to the pulse peak to the total waveform area.

The 5a to 5d in FIG. 18 indicate the pulse wavelength, the wave height value, the peak position ratio, and the kurtosis, respectively. The BL in FIG. 18 indicates the base line (hereinafter referred to as the base line) extracted from the pulse waveform data (refer to BL extraction process to be described later). These four kinds of pulse feature values are defined by the following (1) to (4) on the basis of FIG. 18.

(1) Wavelength (pulse width) $\Delta t$: $\Delta t = t_e - t_s$ ($t_s$ is the start time of the pulse waveform, $t_e$ is the end time of the pulse waveform, $\Delta t = t_a$).
(2) Wave height $|h|$ : $h = xp - x_o$ (the height of pulse waveform up to xp of the pulse peak PP with reference to $x_o$ of BL).
(3) Peak position ratio r : $r = (tp - t_s) / (t_e - t_s)$ (the ratio of the time tb = $(tp - t_s)$ from the pulse start to the pulse peak pp to the pulse wavelength (= $\Delta t$)).
(4) Peak kurtosis x: It is normalized so as that the wave height $|h| = 1$, $t_s = 0$ and $t_e = 1$ hold, and there are collected the time set $[ T ] = [ [ti] | i = 1, \cdots, m ]$ which is the time crossing the horizontal line of 30% in wave height from the pulse peak PP, and then the $\kappa$ is obtained so that the dispersion of the data of the time set $[T]$ is calculated as the pulse waveform spread as shown in the following equation 2.

[Equation 2]

$$\kappa = \frac{1}{m} \sum_{i=1}^{m} (t_i - ave[t])^2$$

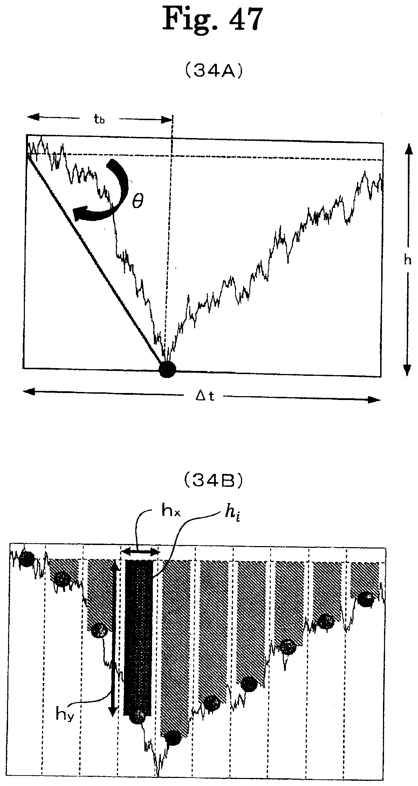

FIG. 47 is the pulse waveform diagram for explaining the feature values of the depression, the area and the area ratio. In the same figure, the horizontal axis represents the time and the vertical axis represents the pulse wave height. These three kinds of pulse feature values are defined by the following (5), (6) and (7) on the basis of the figure.
(5) As shown in (34 A), the depression $\theta$ is the slope leading from the pulse start to the pulse peak and is defined by the following equation 3.

[Equation 31

$$\theta = \arctan\left(\frac{t_p - t_s}{h}\right)$$

(6) The area m is defined as the area [m1 by the inner product of the unit vector [u1 and the wave height vector [p1 as shown in the following equation 4. In the following description, the vector notation of variable A is indicated by [A1. For example, as shown in the 10-division example of (34 B), the area m is the area representing the total sum of the time division area hi (hi = hx $\times$ hy, i = 1 to 10 when width hi and height hy).

[Equation 41]

$$m = (u, p) = \sum_i 1 \cdot h_i$$

Here, it is necessary to calculate and obtain the d-dimensional wave height vector [p1 (= (h1, h2, ..., hd)) in advance as preparation for the feature value calculation.

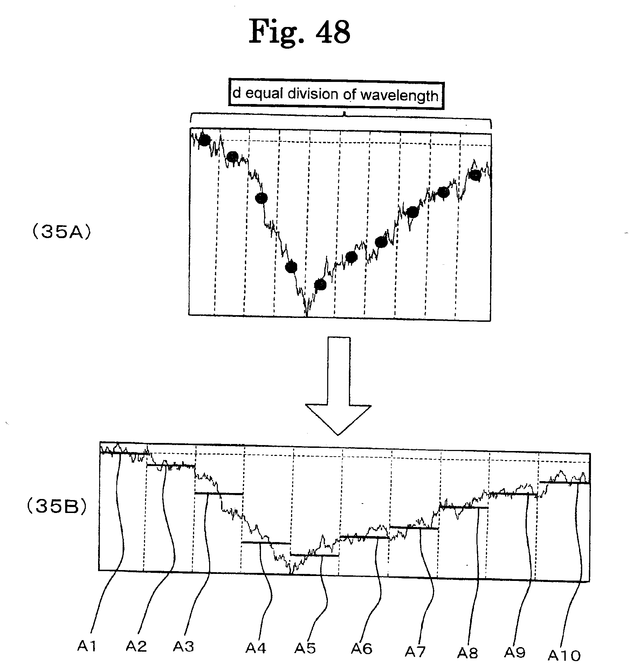

FIG. 48 is the diagram for explaining the manner obtaining the wave height vector.

As shown in (35 A), by equally dividing the wavelength into d number for one waveform data, the d number of data groups are differentiated. Next, as shown in (35B), the values of wave height are averaged for each group (each divided interval), for example, when dividing equally into 10, the average values A1 to A10 are obtained. This averaging can include a case where the wave height value is not normalized and a case where the wave height value is normalized. The area [m1 described by equation 4 indicates a case where the normalization is not performed. The d-dimensional vector having the average values thus determined as components is defined as a "wave height vector".

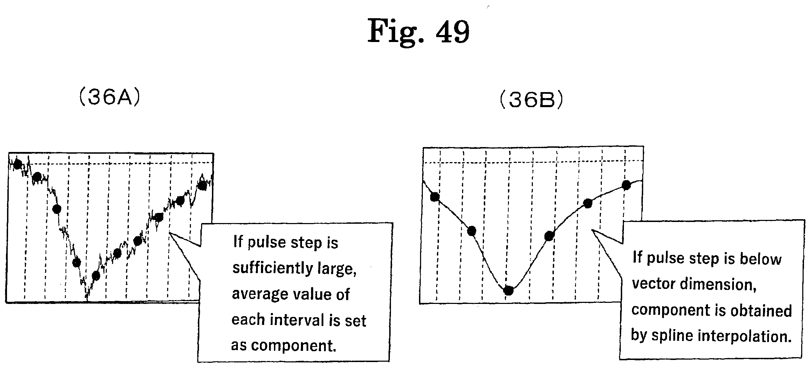

FIG. 49 is a diagram for explaining the relationship between the d-dimensional wave height vector and the data sampling.

As shown in (36 A), when the sampling rate related to acquisition of pulse data is large, since the number of steps (number of data) T in the pulse part exceeds the dimension number d of the vector, by the above acquisition steps, it is possible to obtain the wave height vector of which component is the average of each section. On the other hand, as the sampling rate is lowered, the number of steps T in the pulse part falls below the dimension number d (> T) of the vector. In the case of T <d, since the average value of each section cannot be acquired by the acquisition procedure described above, it is possible to acquire the d-dimensional wave height vector by cubic spline interpolation.

The feature value extraction program includes the wave height vector acquisition program for acquiring wave height vector data. In the case where the pulse step number T exceeds the dimension number d of the vector (T> d) (T = d) by executing the wave height vector acquisition program, the average value of each division equally divided in the time direction is obtained, In the case where the pulse step number T is smaller than the dimension number d of the vector (T <d), a cubic spline interpolation is executed to obtain the d-dimensional wave height vector. That is, by performing the interpolation process using the cubic spline interpolation method, even when the number of pulse steps is small, the number of dimensions of the vector can be made constant.

(7) The area ratio $r_m$ is defined as the area ratio of the sum of the time interval area hi shown in (34B) in the section leading from the pulse start to the pulse peak to the total waveform area. The following Equation 5 shows the area ratio $r_m$.

[Equation 4]

$$r_m = \sum_{t<t_p} h_t \Big/ \sum h_t$$

The feature value of the first type is uniquely derived from the waveform of the pulsed signal such as the pulse wave height, the pulse wavelength, the pulse area and the like, so that it is the feature value showing the local feature. The second type of the feature value is the feature value indicating the global feature with respect to the first type of the local feature.

The second type of the feature value is one selected from a group of

the time inertia moment determined by mass and rotational radius when the mass is constructive to said time division area centered at the pulse start time and the rotational radius is constructive to time leading from said center to said time division area,

the normalized time inertia moment when said time inertia moment is normalized so as that the wave height becomes a standard value,

the mean value vector whose vector component is the mean value of the same wave height position in which the wave form is equally divided in the wave height direction and the mean value of time values is calculated for each division unit in before and after each pulse peak,

the normalized mean value vector which is normalized so as that the wavelength becomes a standard value for said mean value vector,

the wave width mean value inertia moment determined by mass distribution and rotational center when the mass distribution is constructive to mean value difference vector whose vector component is mean value difference of the same wave height position in which the wave form is equally divided in the wave height direction and the mean value of time values is calculated for each division unit in before and after each pulse peak and the rotational center is constructive to time axis of waveform foot,

the normalized wave width mean value inertia moment when said wave width mean value inertia moment is normalized so as that the wavelength becomes a standard value,

the wave width dispersion inertia moment determined by mass distribution and rotational center when the mass distribution is constructive to dispersion vector whose vector component is dispersion in which the wave form is equally divided in the wave height direction and the dispersion is calculated from time value for each division unit and the rotational center is constructive to time axis of waveform foot, and,

the normalized wave width dispersion inertia moment when said wave width dispersion inertia moment is normalized so as that the wavelength becomes a standard value.

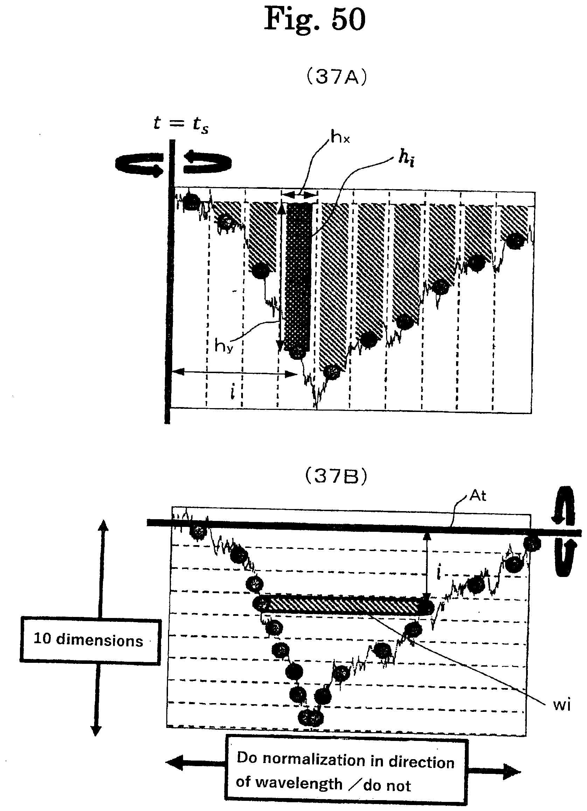

FIG. 50 is the pulse waveform diagram for explaining the feature value of the second type with respect to the time (wavelength) and the wave width. In the same figure, the horizontal axis represents the time and the vertical axis represents the pulse wave height. These pulse feature values are defined by the following (8) to (15) as shown in the same figure.

(8) The time inertia moment, as in (34 B), is the feature value determined by mass and rotational radius when the mass is constructive to the time division area hi formed by equally dividing one waveform in i-dimension with a predetermined time interval and the rotational radius is constructive to the time leading from the center to the time division area hi. That is, the feature value of the time inertia moment is defined by [I] which is the inner product of the vector [v] and the wave height vector [p] as shown in the following equation 6. Here, when the dimension of the vector is n, [v] = $(1^2, 2^2, 3^2, \cdots, n^2)$ and [p] = (h1, h2,''', hd). For example, the time inertia moment, as shown in the example of 10 divisions of (37 A), is the feature value determined when the time division area hi (hi = hx $\times$ hy, i= 1 to 10 with width $h_x$ and height hy) is regarded as the mass and the time leading from the center to the time division area $h_i$ is regarded as the turning radius, where one waveform is divided into 10 with a predetermined time-interval as in (34 B), so that in the same manner of the area m of (6), it can be obtained from the wave height vector.

[Equation 6]

$$I = (v, p) = \sum_i i^2 \cdot h_i$$

(9) The normalized time inertia moment is calculated by using the waveform normalized in the wave height direction in which the wave height becomes the reference value "1" for the waveform for which the time division area is created as shown in (8), and is the feature value defined by equation 6 using the wave height vector $h_i$ in the same manner shown in (8).

(0) The average value vector divides one waveform equally with i-dimension in the wave height direction as shown in the example of 10 division of (37 B), and the average value of the time values is calculated for each division unit (division region $w_i$) in before and after each pulse peak, and the average value of the same wave height position of the division region wi is set as the component of the vector.

(1) The normalized average value vector is the feature value in the case where the wavelength is normalized to be the reference value with respect to the average value vector of (10).

(2) The wave width mean value inertia moment is, as shown in 10 division example of (37B), obtained by equally dividing one waveform into i-dimensions in the wave height direction in the same way as in (8) and by calculating the mean value of time values for each division unit (divided region wi) in before and after the pulse peak, so that the difference vector of the mean value having the difference of the mean value of the same wave height positions in the divided region wi as the components of the vector is regarded as the mass distribution $h_i$ (the dimension number of the vector is n, i = 1 to n) and it is the feature value defined as the moment of inertia when the time axis At of the waveform foot is to be the rotation center. The definition formula is the same as equation 6, and the feature value of (12) can be obtained by the inner product of the vector [v1 and the mass distribution hi.

(3) The normalized wave width mean value inertia moment is the feature value which uses the waveform normalized in the wavelength direction in which the wavelength becomes the reference value "1" for the waveform creating the division region $w_i$ shown in (12) and is the feature value defined by equation 6 using the mass distribution $h_i$ created through the same manner as in (12).

(4) The wave width dispersion inertia moment, similar to the wave width mean value inertia moment, is obtained by equally dividing one waveform into i-dimensions in the wave height direction and by calculating the dispersion from the time values for each division unit (divided region $w_i$) in before and after the pulse peak, so that the dispersion vector having the dispersion as the components of the vector is regarded as the mass distribution $h_i$ (the dimension number of the vector is n, i = 1 to n) and it is the feature value defined as the moment of inertia when the time axis At of the waveform foot is to be the rotation center, and this feature value is defined by equation 6, in the same manner as the wave width mean value inertia moment.

(15) The normalized wave width dispersion inertia moment is the feature value which uses the waveform normalized in the wavelength direction in which the wavelength becomes the reference value "1" for the waveform creating the divided region wi shown in (14) and is the feature value defined by equation 6 using the mass distribution $h_i$ created through the same manner as in (14).

The wave width mean value inertia moment and the wave width dispersion inertia moment are the feature values defined by equation 6 as described above, and the vector [p1 in the definition is the difference vector of mean values of the time values in the case of the wave width mean value inertia moment, and the dispersion vector of the time values in the case of the wave width dispersion inertia moment. In the following description, the vector [p1 in the moment of inertia with respect to the wave widths of (12) to (15) is expressed as [pw1.

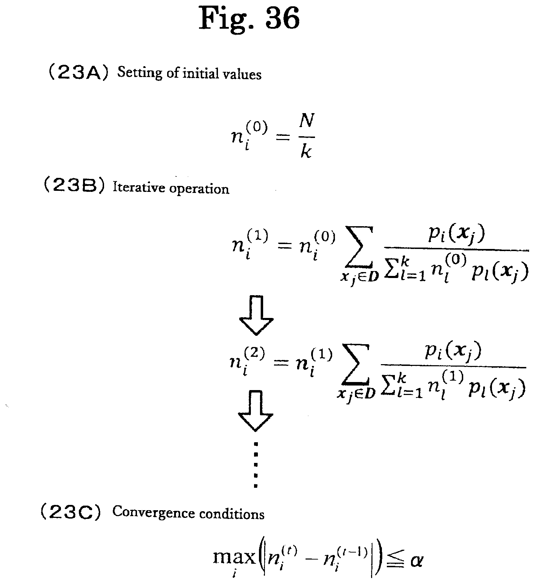

For the creation of moment of inertia with respect to the wave widths of (12) to (15), there is performed using the wave width vector [pw1 (= [p1, p2,···, pdw1) in which the vertical and horizontal axes of the wave height vector shown in FIG. 36 are exchanged.

The wave width vector is the difference vector or the dispersion vector of the mean value shown in the definition of the feature value of (12) to (15). By grasping the wave width vector as the density distribution, the wave width mean value inertia moment of (12) and (13) and the wave width dispersion inertia moment of (14) and (15) can be obtained. The wave width vector is the dw dimensional vector, for which the pulse waveform data is equally divided with dw dimensions in the wave height direction and whose components are the difference or dispersion of the mean values of the peak values obtained for each division. In the case of (37B), the dimension of the wave width vector is 10 dimensions. The time axis At shown in (37B) is different from the base line BL, and is the rotation axis line around the pulse foot obtained from the wave width vector.

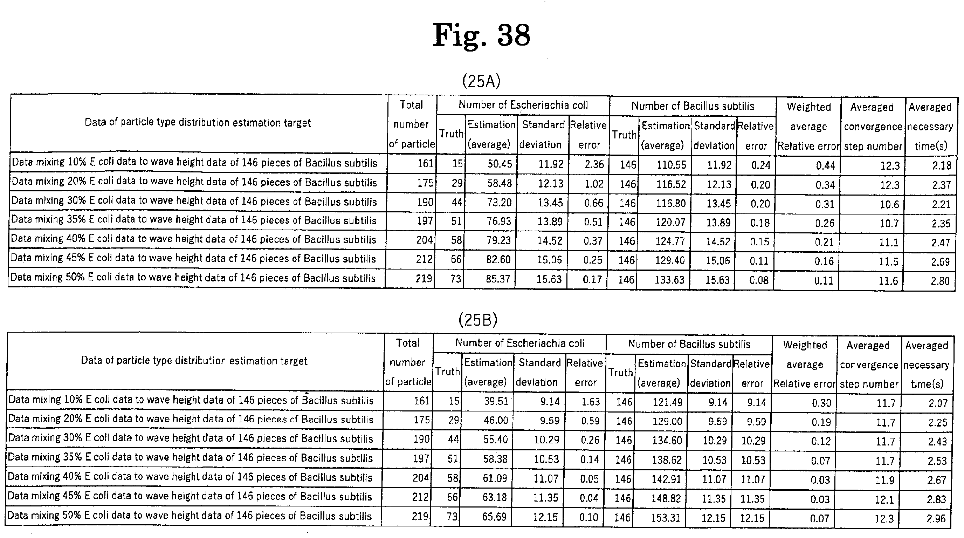

FIG. 38 is the diagram for explaining the relation between the wave width vector of dw dimension and the data sampling.

The feature value extraction program includes the wave width vector acquisition program for acquiring the wave width vector by the creation calculation processing of the wave width vector of the following dw dimension.

Since the pulse waveform data distribute at various intervals in the wave height direction, there may be cases that it is contained one or more non-existent regions Bd in which data points do not exist in the section divided in the wave height direction. In (38A), an example of the non-existent region Bd is indicated by an arrow. In the non-existent region Bd, the data points do not exist because the data interval becomes coarse, so that it is impossible to obtain the component of the inertia moment with respect to the wave width defined by the above equation 6. Therefore, as in the case of the above-described pulse waveform spread, when the wave height to the pulse peak is equally divided by dw, the time set [Tk] = [ [$t_i$] | i = 1,···,m] of each wave height is collected and the components of the wave width vector are created. At this time, in the non-existent region Bd in which no data point exists, the component data is acquired by the linear interpolation. The linear interpolation is performed on two consecutive data that extends over the values of (10k + 5)% (k = 0, 1, 2, 3,···) of the pulse peak. The (38B) shows an example of the linear interpolation point tk with respect to the height k of the non-existent region Bd occurring between the data points ti and ti+1. In the creation of the wave width vector, as shown in (38 C), when a discrepancy of the wave height data occurs in the foot region UR of the pulse waveform data, the wave height data Du on the side far from the pulse peak is truncated to align to the side close to the pulse peak. The execution process of the wave width vector acquisition program includes the linear interpolation process for the non-existent area Bd and the truncation process of the pulse height data Du for the discrepancy of the pulse height data.

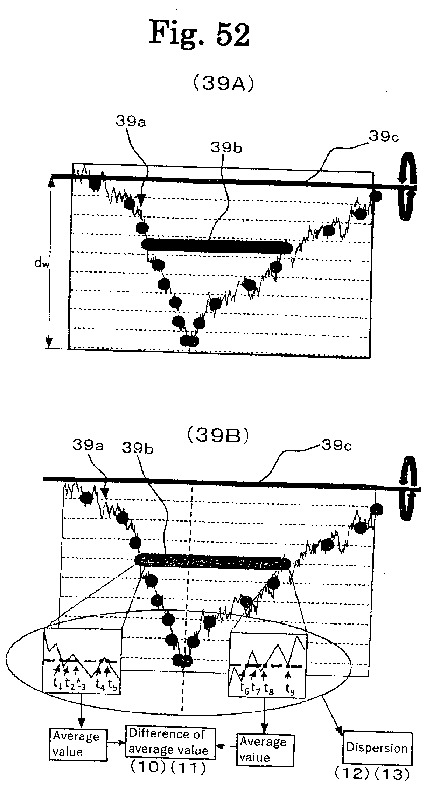

FIG. 52 is the diagram for explaining the acquisition process of acquiring the inertia moment with respect to the wave width from the wave width vector.

The (39A) is the example in which the waveform is equally divided into ten intervals in the wave height direction, and there are shown the division region 39 b of the wave width vector and the rotation axis line 39c which are obtained by performing the above linear interpolation process and the truncation process for one waveform 39.

As shown in (39 B), for each division unit, the mean value of the time values is calculated before and after the pulse peak, and there can be obtained the wave width vector which is the difference vector of the mean value having the difference of the mean value at the same wave height position in the divided region as the component of the vector. By regarding this difference vector of the mean value as the mass distribution, the wave width mean value inertia moment of (12) with the rotation axis line 39c (time axis) as the rotation center can be created. Further, by calculating the dispersion from the time value of each division unit, it is possible to obtain the dispersion vector having the dispersion as the component of the vector. By regarding this dispersion vector as the mass distribution, it is possible to create the wave width dispersion inertia moment of (14) using the rotation axis line 39 c (time axis) as the rotation center. In addition, the average value vectors of (10) and (11) are the vectors in which the average value of time values is calculated and whose component is the average value at the same wave height position of the division region, and it is represented by the time vector with 2 dw dimensions in the case of the equal division of dw.

The vector dimension number of the wave height vector and the wave width vector used for creating the feature value need not be limited to the division number but can be arbitrarily set. The wave height vector and the wave width vector are obtained by dividing in one direction of the wavelength or the wave height, but the vectors which are divided into a plurality of directions can be used for creating the feature value.

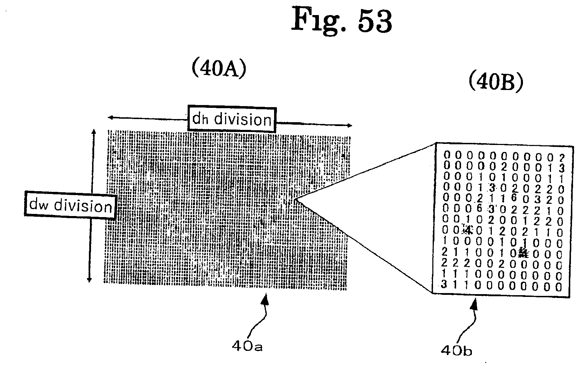

FIG. 53 is the diagram for explaining an example of the waveform vector for creating the feature value in the case divided into a plurality of directions.

The (40A) shows the data map 40a obtained by dividing one waveform data into the mesh shape. The data map 40a shows the distribution state of the number of data points in the matrix form by dividing the waveform data with the dn division in the time axis direction of the horizontal axis and the dw division in the wave height direction of the vertical axis. The (40B) shows the distribution state in which a part of the matrix-like section (lattice) is enlarged. In the distribution state of (40 B), 0 to 6 data points are distributed in $11 \times 13$ grids. By this matrix division, the waveform vector in which the number of data points in each lattice / the total number of data points is a component of the dn $\times$ dw dimensional vector is converted into a vector in which data groups of the matrix array are rearranged in a scanning manner, so that it is possible to create the feature value instead of the wave height vector and the wave width vector.

<About Estimation of Base Line>

Generally, bacteria or the like are minute objects having finely the different forms. For example, in the case of average Escherichia coli, the body length is 2 to 4 $\mu$m and the outer diameter is 0.4 to 0.7 $\mu$m. In the case of the average Bacillus subtilis, the body length is 2 to 3 $\mu$m and the outer diameter is 0.7 to 0.8 $\mu$m. In addition, flagella of 20 to 30 nm are attached to Escherichia coli and the like.

When using bacteria or the like as subject particles, if slight differences are missed from the pulse waveform data, the number judgment accuracy will be lowered. For this reason, in order to accurately calculate the feature value and use it as the estimation basis of the probability distribution, it is necessary to accurately grasp the particle passage pulse wave height. For this purpose, it is necessary to estimate the base line of the measurement signal. However, because the base line of the original data of the measurement signal contains fluctuations due to noise data and weak measured current, the pulse wave height and the like need to be detected after the base line excluding the fluctuation component etc. is determined. It is preferable that the base line estimation (hereinafter referred to as BL estimation) is practiced online (instantly) by the computer in practice.

As a method for estimating BL on a computer, by using the Kalman filter suitable for estimating the amounts that change from moment to moment from observations with discrete errors, the disturbances (system noise and observation noise) are removed, the base line BL can be estimated.

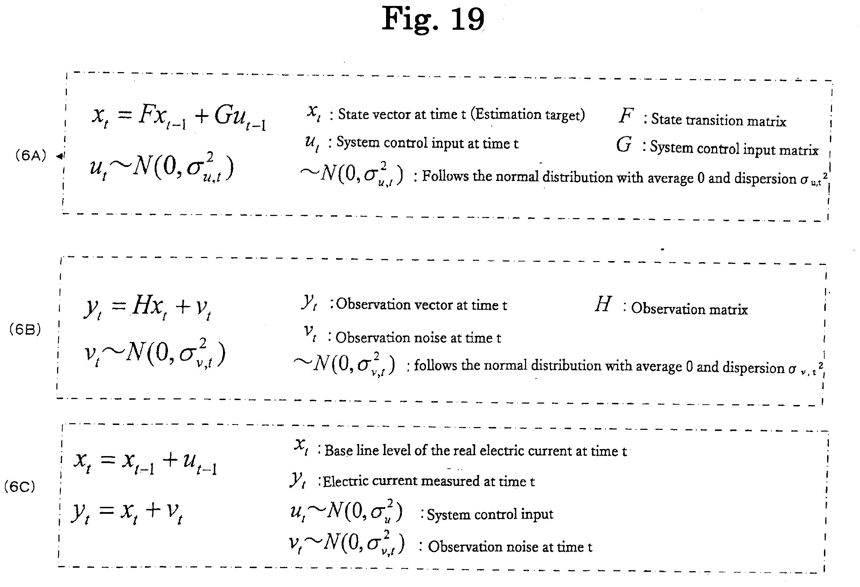

The Kalman filter is a method for estimating the value at the time [t1 of updatable state vector [x1, in which the discrete control process is defined by the linear difference equation shown in (6A) of FIG. 19. In the Kalman filter, the values of the state vector [x1 and the system control input [ut1 cannot be directly observed.

It is assumed that the state vector [x] is indirectly estimated by the observation model shown in (6B) of FIG.19. For the system control input [ut], only the statistical fluctuation range [$\sigma$u,t] is assumed as a parameter.

The measured current data [X] is not a vector but a scalar, further various matrices are also scalars, and it can be regarded as [F] = [G] = [H] = [1]. Therefore, when letting [xt], [yt] and [vt] be the base line level of the actual current value at the time t, the current measured at the time t, and the observation noise at the time t, respectively, [xt] and [yt] are expressed as shown in (6 C) of FIG. 19. The [xt], [ut], [vt] are unobservable factors and [yt] is observable factor. Let f (Hz) be the measurement frequency of the ion current detector, the time data is 1 / f (second) increments. Baseline estimation can be performed assuming that the influence of the system control input [ut] is practically very small.

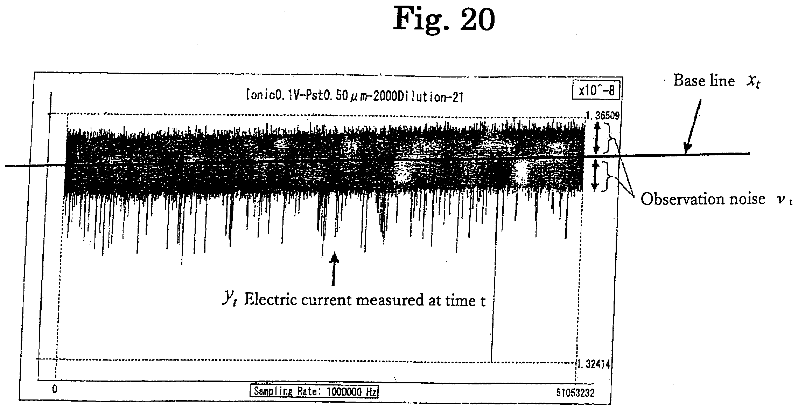

FIG. 20 is the diagram showing each of the above factors by the actual measurement current data. At the time of actual measurement by the ionic current detecting part, the particles are clogged in the through-hole 12, causing distortion of the base line. However, at the time of measurement, it interrupts at the point of occurrence of distortion and the measurement is performed after the cause of distortion is eliminated., so that only data including the base line without distortion is collected in the original data set.

The estimation due to the Kalman filter is performed by repetition of prediction and updating. The estimation of the base line is also executed by repetition of prediction and updating due to the Kalman filter.

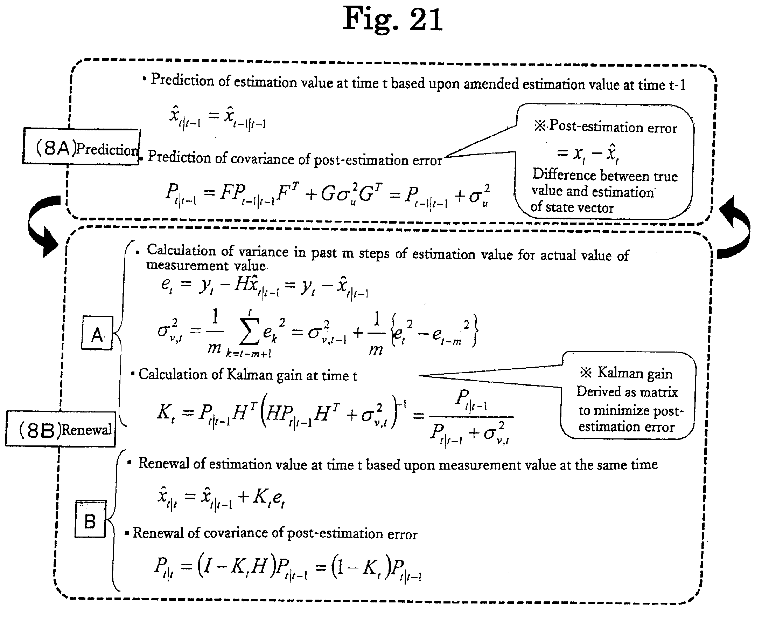

FIG. 21 is the diagram showing the details of the repetition of the prediction (8 A) and update (8 B) of the Kalman filter. In FIG. 21, the "hat" symbol added to the vector notation indicates the estimated value. The subscript "t | t - 1" indicates that it is an estimate of the value at the time t based on the value at the time (t - 1).

FIG. 22 shows the BL estimation process based on the BL estimation process program. For the BL estimation process, the estimation of BL and the extraction of the pulse peak value based on the BL estimation are performed.

When executing the BL estimation process, the values of the start time m, the constants k, $\alpha$ of the adjustment factors necessary for the prediction and update process in the Kalman filter are adjusted (tuned) and decided to appropriate values according to the data attribute of the estimation target in advance. The value of $\alpha$ is the value for adjusting the dispersion of the estimated value of the base line. The value of k is the value related to the number of executions of update A in the Kalman filter shown in FIG. 21 (see the steps S57 and S62). The start time m is the time data for the number of steps calculated with one measurement sampling as one step.

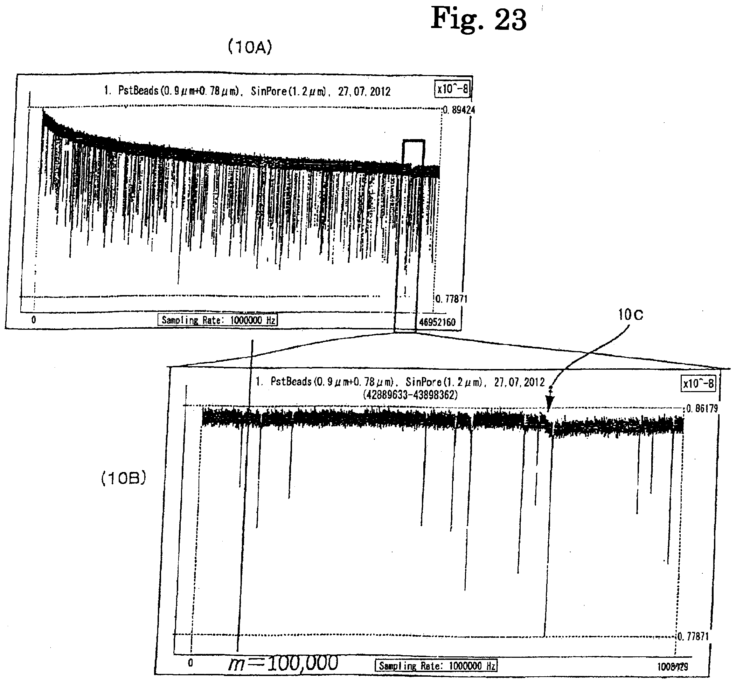

FIG. 23 shows the waveform diagram of the bead model used for the adjustment. FIG. 15 shows the solution state in the case (bead model) where the fine bead balls having the same size as bacteria or the like are mixed as particles. FIG. 23 (10A) is the waveform data acquired at the sampling frequency 900000 Hz by the ion current detection portion. The waveform of the bead model shown in (10A) shows the waveform that attenuates gradually. The violent depression has occurred in the right end portion of (10A), which is enlarged and shown in (10 B).

When the step portion (10 C) of the base line shown in (10B) is detected from the waveform of the bead model, the immediately preceding period becomes the initial value calculation period. For example, when m = 100000, the 11 to 12 pulses having significance can be visually confirmed in the period excluding the initial value calculation period.

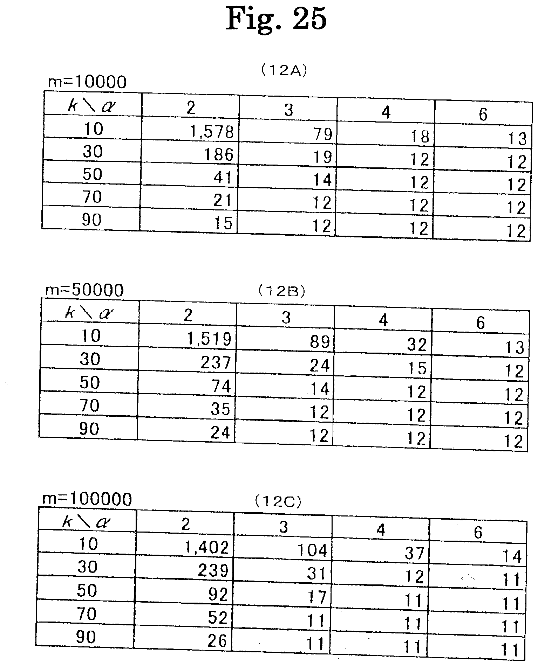

FIG. 25 is the table showing the number of pulses picked up from the waveform of the bead model according to the combination of m, k, and $\alpha$ of the adjustment factors.

The (12 A) of FIG. 25 shows the number of pulses according to the combination of k values (10, 30, 50, 70, 90) and $\alpha$ values (2, 3, 4, 6) when m = 10000. FIG. (12B) shows the number of pulses according to the combination of k values (10, 30, 50, 70, 90) and $\alpha$ values (2, 3, 4, 6) when m = 50000. (12C) shows the number of pulses due to the combination of k values (10, 30, 50, 70, 90) and $\alpha$ values (2, 3, 4, 6) when m = 100000.

Comparing the three kinds of simulation results in FIG. 25, in the case of (12A) and (12B), the number of pulses to be measured is 12, and in the case of (12C) it is 11. Therefore, in the embodiment, the smallest (12 C) of the maximum value of the pulse number is adopted, and the tuning setting of m = 100000, k = 50, and $\alpha$ = 6 is performed. These tuning setting data are stored and set in advance in the setting area of the RAM 23.

The BL estimation process of FIG. 22 is performed by the BL estimation due to the Kalman filter shown in FIG. 21 under the above tuning setting. First, in step S51, the initial value of the Kalman filter at time m is set in the work area of the RAM 23. At this time, the pulse waveform data stored in the data file storage portion 5 is read into the work area of the RAM 23. Next, the prediction and update (A and B of FIG. 21) of the Kalman filter at time (m+1) are executed (step S52). In the prediction and update, each operation of the Kalman filter shown in FIG. 21 is executed and stored in the RAM 23. After that, the prediction and update (A and B) are repeatedly performed at the predetermined unit time, and when the prediction and update A of the Kalman filter at time t are performed, it is judged whether or not the condition of the following equation 7 is satisfied (Steps S53 and S54). The unit time is the value determined by the sampling frequency of the original data, and is set in advance in the RAM 23.

[Equation 7]

$$|e_t| > \alpha \cdot \sigma_{v,t} \qquad e_t = y_t - \hat{x}_{t|t-1}$$

When the condition of the equation 7 is not satisfied, the update B of the Kalman filter at the time t is executed, and the processes of the steps S53 to S55 are repeated for each data whose unit time has elapsed. When the above condition of Equation 7 is satisfied, the number value is cumulatively stored in the count area of the RAM 23 every time (steps S54 and S56). Next, on the basis of the count value, it is judged whether or not the condition of the equation 7 has been satisfied by k times consecutively starting from the time s (step S57). If it is not consecutive by k times, the process proceeds to step S55 and the update B is performed.

When the k times consecutive, the process proceeds to step S58, and it is judged that the hold necessary period for determining the BL has started. At this time, the hold start time of the hold necessary period is stored as s in the RAM 23, and the operation result of the Kalman filter between the time (s + 1) and the time (s + k-1) is not stored but discarded.

By the start of the hold necessary period, the drop maximum value of the pulse at the time t is stored in the RAM 23 in a updatable manner (step S59). Next, similarly to step S54, it is judged whether or not the condition of the following equation 8 is satisfied during the hold necessary period (step S60).

[Equation 8]

$$\left| y_t - \hat{x}_{s|s-1} \right| \leqq \alpha \cdot \sigma_{v,s}$$

When the condition of the above formula 8 is not satisfied, the pulse drop maximum value is updated (steps S59, S60). When the condition of Equation 8 is satisfied, the number value is cumulatively stored in the count area of the RAM 23 at each time (steps S60, S61). Next, on the basis of the count value, it is judged whether or not the condition of the equation 8 has been satisfied by k times consecutively starting from the time s2 (step S62). If it is not k times consecutive, the process returns to step S59.

If k times consecutive, the process proceeds to step S63, and the maximum drop value of the pulse which is updated and stored at this time is stored in the RAM 23 as the estimation value of the pulse wave height value. The estimation value of the pulse wave height value is stored together with the data of the pulse start time and the pulse end time. When the estimation of the pulse wave height value is completed, it is determined that the hold necessary period is ended. By this termination, the hold end time of the hold necessary period is stored in the RAM 23 as s2 (step S64). Next, in step S65, the value of the time s is retroactively calculated for the period from the time s2 to the time (s + k-1) as the initial value at the restart of the operation process of the Kalman filter and the operation of the Kalman filter is executed. After step S65, it is judged whether or not the BL estimation process of all pulse waveform data has been performed (step S66), and the process is terminated at the completion of estimation of all pulse waveform data, and when there is remaining data, the process goes to step S53.

<About Feature Value Extraction>

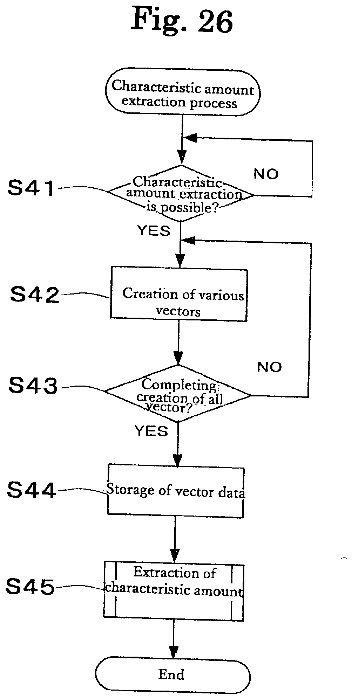

FIG. 26 shows the outline of the executing process content of the feature value extraction program.

The feature value extraction process becomes executable on condition that the extraction data of the pulse wave height value (wave height |h|) is present by execution of the BL estimation process of FIG. 22 (step S41). When there is the extraction data of the pulse wave height value, the wave height vector acquisition program and the wave width vector acquisition program described above are executed and the data generation calculation of various vectors is executed (step S42). When all the data acquisition of the wave height vector and the wave width vector is completed, the vector data is stored (steps S43 and S44). Next, the extraction process of various feature values is executed (step S45). In acquiring the data of the wave height vector and the wave width vector, the interpolation process using the cubic spline interpolation method, the linear interpolation process and the truncation process are performed at any time.

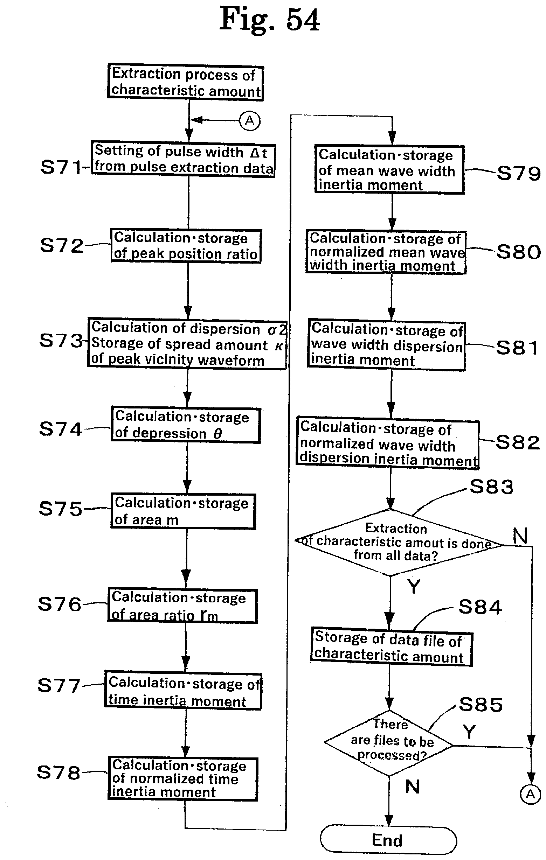

FIG. 54 shows the contents of execution process of the feature value extraction process (step S45). The Steps S71 to S83 show the calculation of the first type and the second type of feature values defined in above (1) to (13), and the process of remembering and storing the calculated feature values, respectively.

The first type of feature values are calculated in steps S71 to S76. The wavelength (pulse width) $\Delta t$ is sequentially calculated and stored with respect to the extracted data group of the pulse wave height value (step S71). The calculated feature value is stored in the memory area for storing the feature value of the RAM 4. The pulse width is obtained by calculating $\Delta t$ (= te - ts; ts is the start time of the pulse waveform and te is the end time of the pulse waveform). The peak position ratio r is sequentially calculated and stored with respect to the extraction data group of the pulse wave height value (step S72). The peak position ratio r is calculated by r = (tp - ts) / (te - ts) (the ratio of the pulse width $\Delta t$ and the time (tp - ts) leading from the pulse start to the pulse peak pp.

The peak kurtosis $\kappa$ is sequentially calculated and stored with respect to the extraction data group of pulse wave height values (step S73). It is normalized so as that the wave height |h| = 1, ts = 0 and te = 1 hold, and there are collected the time set [ T ] = [ [ti] | i = 1,···,m ] which is the time crossing the horizontal line of 30% in wave height from the pulse peak PP, and then the $\kappa$ is obtained so that the dispersion of the data of the time set [T] is calculated as the pulse waveform spread.

The depression $\theta$ is obtained based on the time from pulse start to pulse peak and wave height data and the calculation of Equation 3 above (step S74). The area m is obtained from the wave height vector data, and is calculated and stored by obtaining the time division area hi according to the number of division (number of divisions set in advance: 10) and calculating the total sum thereof. The area ratio $r_m$ is calculated and stored by calculating the total waveform area and the partial sum of time division area hi in each division leading from the pulse start to the pulse peak and by calculating the area ratio of the partial sum to the total waveform area (Step S76).

The second type feature values are calculated in steps S77 to S82. The time inertia moment is obtained from the data of the wave height vector, and are calculated and stored based on the time division area hi obtained according to the number of divisions and the calculation of Equation 6 above (step S77). The normalized time inertia moment of (9) is stored as the normalization data (step S78) by the process normalized in the wave height direction (the inner product of the wave height vector and the normalized vector) so that the wave height becomes "1" of the reference value with respect to the time inertia moment obtained in step S77. The wave width mean value inertia moment is calculated from the data of the wave width vector (the difference vector of the mean value) obtained in steps S42 to S44 by using the time value calculated for each division unit (the number of divisions set in advance: 10) before and after the pulse peak and the calculation of Equation 7 and is stored (step S79). The wave width mean value inertia moment (11) is stored as the normalization data (step S80) normalized in the wavelength direction (the inner product of the difference vector of the mean value and the normalized vector) so that the wavelength becomes the reference value "1" for the wave width mean value inertia moment obtained in step S79. The wave width dispersion inertia moment is calculated from the data of the wave width vector (dispersion vector) based on the dispersion of the time value calculated for each division unit and the calculation of the above equation 7 and stored (step S 81). The normalized wave width dispersion inertia moment of (13) is stored as the normalization data (step S82) normalized in the wavelength direction (the inner product of the dispersion vector and the normalized vector) so that the wavelength becomes the reference value "1" with respect to the wave width dispersion inertia moment obtained in step S 81.

Upon completing the extraction of the feature value from all the data, the file of each data is stored and it is judged whether or not there is another data group (steps S83, S84). If there is the data group of another file, the above process (steps S71 to S82) can be repeatedly executed. When there is no more data to be processed, the extraction process of the feature value ends (step S 85). In the above extraction process, all of the first type and the second type of feature values are obtained, but it is also possible to designate a desired feature value by designation input of the input means 6 and it is possible to extract only the feature value due to this designation.

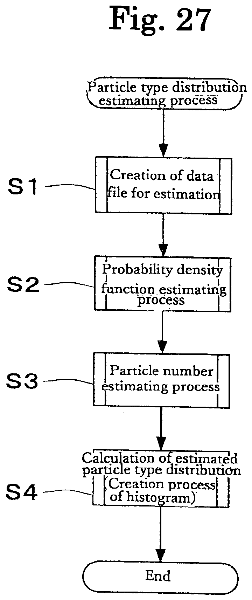

FIG. 27 shows the particle type estimation process executed based on the particle type distribution estimation program.

<About Estimation of Probability Density Function>

Since the pulse waveform to be measured is not necessarily constant even for the same type of particles, the probability density function of the pulse waveform of particle type is preliminarily estimated from the test data as preparation for the particle type distribution estimation. The appearance probability of each pulse can be expressed by the probability density function derived through the estimation of the probability density function.

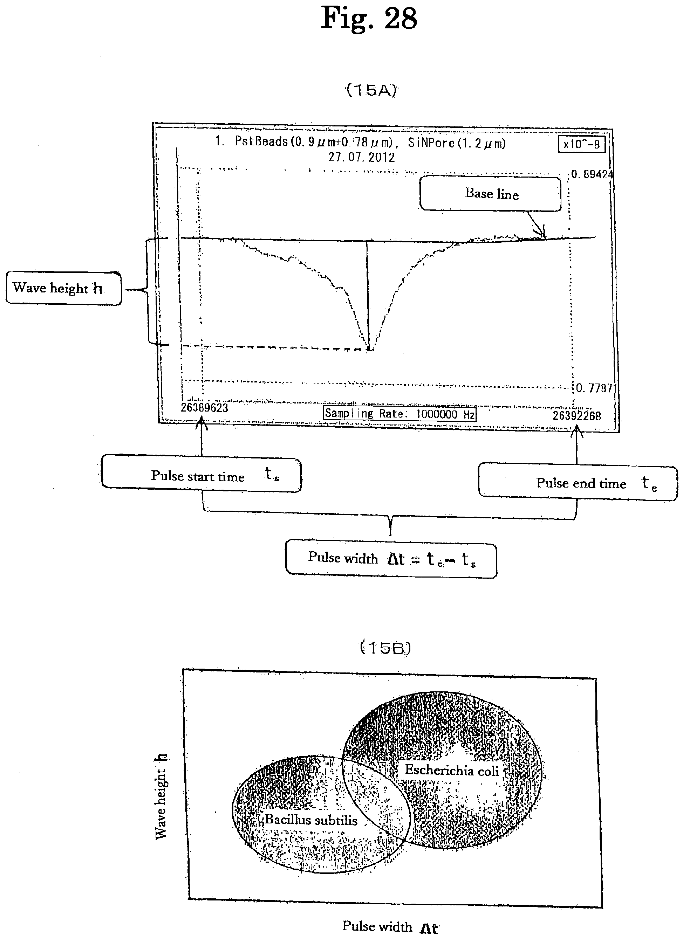

The (15 B) of FIG. 28 is the image diagram of the probability density function for the pulse waveform obtained by using the pulse width and the pulse wave height as the feature value of the pulse waveform in the particle types of Escherichia coli and Bacillus subtilis, and the appearance probability of the pulse is expressed by shading in the figure. The (15 A) of FIG. 28 shows a part of the first type of feature value relating to one waveform data.

Since the true density function of the pulse width $\Delta t$ and the pulse wave height h is unknown, it is necessary to estimate the nonparametric probability density function. In the present embodiment, the Kernel density estimation using a Gaussian function as the Kernel function is used.

Kernel density estimation is a method of assuming the probability density distribution given by Kernel function to the measurement data and regarding the distribution obtained by superimposing these distributions as the probability density function. When using a Gaussian function as the Kernel function, it is possible to assume a normal distribution for each data and to regard the distribution obtained by superimposing them as the probability density function.

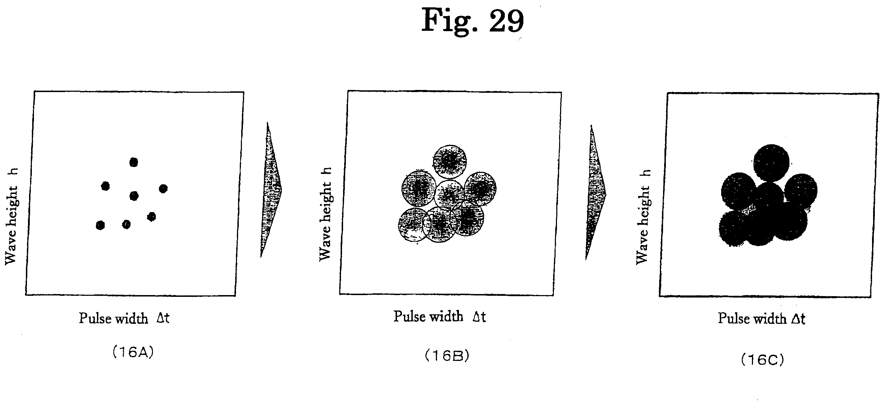

FIG. 29 is the image diagram of a superposition of probability density distributions obtained from each particle type of Escherichia coli and Bacillus subtilis. FIG. 16C shows the state in which the probability density distribution (16 B) obtained for each particle is superimposed from the feature value data (16 A) of the pulse width $\Delta t$ and the pulse wave height h.

The probability density function [p(x)1 for the input data [x1 is expressed by the following equation 9 using the number of teacher data [N1, the teacher data [$\mu$i], and the variance covariance matrix [$\Sigma$].

[Equation 91

Probability distribution function for input data $x = \begin{bmatrix} \Delta t \\ h \end{bmatrix}$

$$p(x) = \frac{1}{N}\sum_{i=1}^{N}\frac{1}{2\pi\sqrt{|\Sigma|}}\exp\left(-\frac{(x-\mu_i)^T\Sigma^{-1}(x-\mu_i)}{2}\right)$$

Furthermore, the probability density function [p(x)1 can be expressed by the product of the Gaussian functions of each dimension as shown in the following Equation 10.

[Equation 101

For simplicity of calculation, the covariance term of

the variance-covariance matrix $\Sigma$ is set to 0

$$\Sigma = \begin{bmatrix} \sigma_{\Delta t}^2 & 0 \\ 0 & \sigma_h^2 \end{bmatrix} \quad |\Sigma| = \sigma_{\Delta t}^2\sigma_h^2 \,,\, \Sigma^{-1} = \begin{bmatrix} 1/\sigma_{\Delta t}^2 & 0 \\ 0 & 1/\sigma_h^2 \end{bmatrix}$$

$$p(x) = \frac{1}{N}\sum_{i=1}^{N}\left\{\frac{1}{\sqrt{2\pi}\sigma_{\Delta t}}\exp\left(-\frac{(\Delta t - \mu_{\Delta t}^i)^2}{2\sigma_{\Delta t}^2}\right)\right\}\left\{\frac{1}{\sqrt{2\pi}\sigma_h}\exp\left(-\frac{(h-\mu_h^i)^2}{2\sigma_h^2}\right)\right\}$$

As can be seen from Equation 10, it is equivalent to assuming that each pulse attribute is an independent random probability variable that follows the normal distribution, which can be expanded to three or more dimensions as well. Therefore, in the present embodiment, it is possible to analyze the number of two or more types of particle types.
The probability density function module program has a function of computing and obtaining the probability density function for two types of feature values. That is, in the case of using the estimation target data due to two feature values [(β, γ)1, the probability density function [p(β, γ)1 in the Kernel density estimation using the Gaussian function as the Kernel function is expressed by the following equation 11.

[Equation 111

The covariance term of the variance-covariance matrix $\Sigma$ is set to 0

and when $\quad \Sigma = \begin{bmatrix} \sigma_\beta^2 & 0 \\ 0 & \sigma_\gamma^2 \end{bmatrix} \quad$ and teacher data $\quad \mu_\beta^i, \mu_\gamma^i$

$$p(\beta, \gamma) = \frac{1}{N}\sum_{i=1}^{N}\left\{\frac{1}{\sqrt{2\pi\sigma_\beta^2}}\exp\left(-\frac{(\beta-\mu_\beta^i)^2}{2\sigma_\beta^2}\right)\right\}\left\{\frac{1}{\sqrt{2\pi\sigma_\gamma^2}}\exp\left(-\frac{(\gamma-\mu_\gamma^i)^2}{2\sigma_\gamma^2}\right)\right\}$$

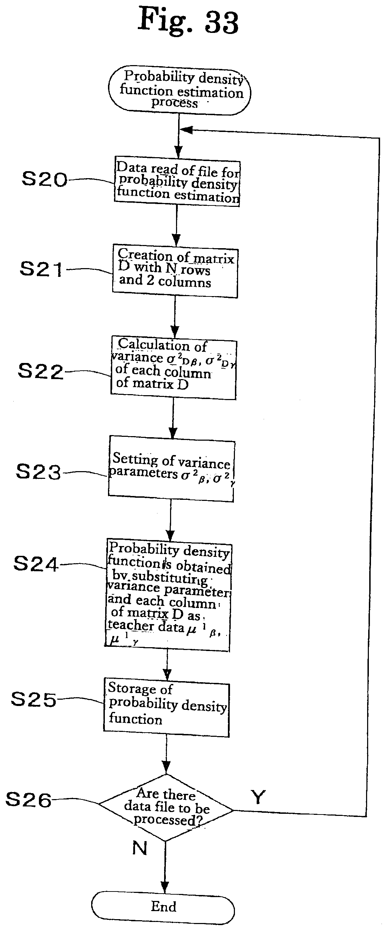

The probability density function estimation process executed by the probability density function module program based on the equation 11 performs the estimation process of the probability density function in two feature values, as described in detail later with reference to FIG 33.

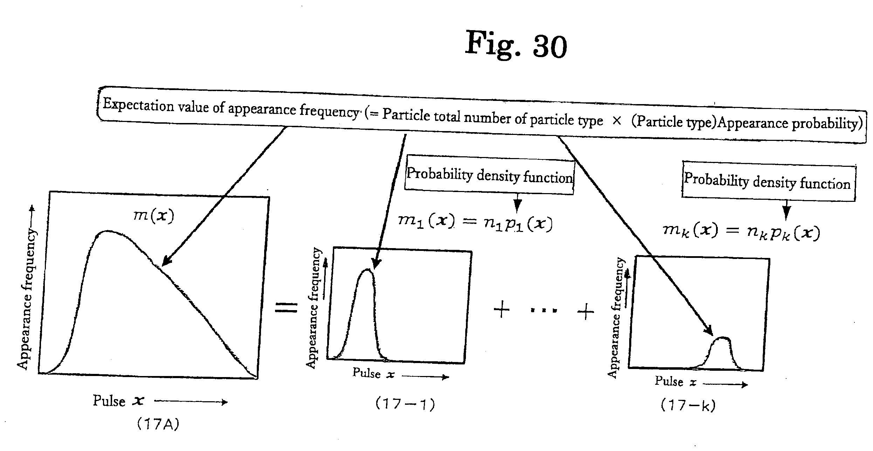

FIG. 30 is the image diagram showing the relationship between the total number of particles of k particle types, the appearance probability of particle type, and the expected value of appearance frequency of the entire data. FIG. 17 (A) shows the appearance frequency of the entire data. FIGs. (17-1) to (17-k) show the appearance frequency of particle types. The expected value of the appearance frequency at which the pulse [x1 is measured becomes the sum of the expected value of the appearance frequency at which the pulse [x1 is measured according to the particle type probability density function. As shown in FIG. 30, it can be expressed by the following equation 12 as the sum of expectation values of particle types from the total number of particles [ni1 of particle type and the particle type appearance probability [pi (x)1.

[Equation 12]

$$m(\boldsymbol{x}) = \sum_{i=1}^{k} m_i(\boldsymbol{x}) = \sum_{i=1}^{k} n_i p_i(\boldsymbol{x})$$

In the present embodiment, the probability density function data (see Expression 10) obtained by estimating the probability density function of the particle type obtained in advance is stored in the RAM 23 as the analysis reference data. The particle type number analysis is performed by determining the appearance frequency of the entire data to be analyzed based on the equation 12 through identifying the number of particle types to be matched from each analysis data. The number analysis is performed by estimating histograms of different particle types (appearance frequency (number of particles) for particle type).

In the particle type estimation process shown in FIG. 27, the data file creation process (step S1) for creating the data file due to the feature value by editing data, the particle number estimation process (step S2), and the calculation process of estimated particle type distribution (histogram creation process) (step S3) are performed. In the particle number estimation process, the estimation methods based on the maximum likelihood method, the Lagrangian multiplier method and the Hasselblad iteration method can be used.

<About Maximum Likelihood Estimation (the method of point estimation of population of probability distribution that it follows from data given in statistics)>

It is assumed that a data set [D1 = [x1, x2, x3,```xN1 has been obtained as an actual pulse estimation result. The likelihood at which the estimated j-th pulse height data appears is expressed by the following equation 13.

[Equation 13]

$$p(\boldsymbol{x}_j) = \frac{m(\boldsymbol{x}_j)}{N} = \frac{1}{N} \sum_{i=1}^{k} m_i(\boldsymbol{x}_j) = \frac{1}{N} \sum_{i=1}^{k} n_i p_i(\boldsymbol{x}_j)$$

Then, the likelihood of appearance of the data set D is expressed by the following equation 14.

[Equation 14]

$$\prod_{\boldsymbol{x}_j \in D} p(\boldsymbol{x}_j) = \frac{1}{N^N} \prod_{\boldsymbol{x}_j \in D} \sum_{i=1}^{k} n_i p_i(\boldsymbol{x}_j)$$

The particle type distribution that maximizes the likelihood in Equation 14 is the particle type distribution with the most likelihood value set [n] = [n1,```, nk]$^T$.

<About Lagrangian undetermined multiplier method (It is an analytical method to optimize under constraint conditions, prepare the undetermined multipliers for each constraint condition and make new functions of linear combinations using these multipliers as coefficients (unknown multipliers are new variables), and it is a method to solve the constraint problem as the normal extreme problem>

Maximizing the likelihood of occurrence of data set D is equivalent to maximizing the logarithmic likelihood that data set [D] appears. The following equation 15 shows the process of deriving the logarithmic likelihood to check the suitability of the Lagrange undetermined multiplier method.

[Equation 15]

$$\prod_{x_j \in D} p(x_j) = \frac{1}{N^N} \prod_{x_j \in D} \sum_{i=1}^{\hat{n}} n_i p_i(x_j) \to \max$$

$$\log \prod_{x_j \in D} p(x_j) = \log \left( \frac{1}{N^N} \prod_{x_j \in D} \sum_{i=1}^{k} n_i p_i(x_j) \right) \to \max$$

$$\sum_{x_j \in D} \log p(x_j) \propto \log L(n; D) \to \sum_{x_j \in D} \log \sum_{i=1}^{k} n_i p_i(x_j) \underset{n}{\to} \max$$

In Equation 15, the coefficient $1 / N^N$ in the middle is omitted in the final expression.

Here, the value set n = [n1,···,nk]$^T$ of the particle diameter number distribution has the constraint "the total is N" (see the following equation 16).

[Equation 16]

$$N = \sum_{i=1}^{k} n_i$$

Therefore, since the proposition of obtaining the most likelihood particle type distribution becomes a problem of constrained logarithmic likelihood maximization, it is possible to perform optimization by the Lagrangian undetermined multiplier method. The constrained logarithmic likelihood maximization equation that optimizes by the Lagrange undetermined multiplier method can be expressed by the following equation 17.

[Equation 171

$$\sum_{x_j \in D} \log \sum_{i=1}^{k} n_i p_i(x_j) - \lambda \left( \sum_{i=1}^{k} n_i - N \right) \underset{n,\lambda}{\to} \max \quad \text{(Lagrange undetermined multiplier method)}$$

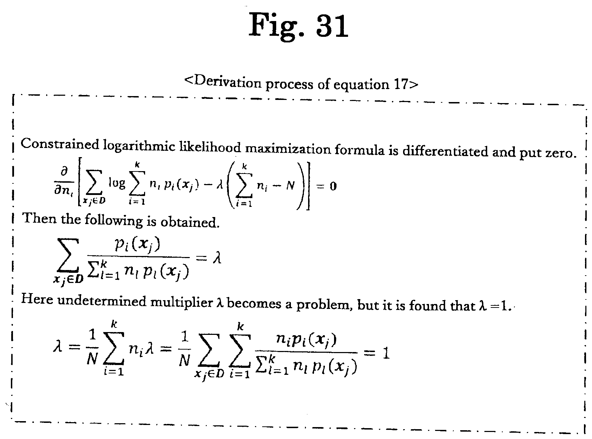

From the constrained logarithmic likelihood maximization equation shown in Equation 17, [k1 simultaneous equations shown in the following Equation 18 can be derived through the mathematical derivation process shown in FIG. 31.

[Equation 18]

$$\sum_{x_j \in D} \frac{p_i(x_j)}{\sum_{l=1}^{k} n_l\, p_l(x_j)} = 1$$

To solve numerically the simultaneous equations shown in Equation 18, it can be performed using the iterative method proposed by Hasselblad. According to the Hasselblad iteration method, the iterative calculation of the following Equation 19 may be performed. The details of this iterative method are described in the proposal paper (Hasselblad V., 1966, Estimation of parameters for a mixture of normal distributions. Technomerics, 8, pp. 431 - 444).

[Equation 19]

$$n_i^{(t+1)} = n_i^{(t)} \sum_{x_j \in D} \frac{p_i(x_j)}{\sum_{l=1}^{k} n_l^{(t)}\, p_l(x_j)}$$

For the iterative calculation of Equation 19, it is performed using the software of EM algorithm available on the market. As clear from the origin of naming, the EM algorithm is the method of calculating the probability distribution parameter by maximizing the likelihood function, that is, the algorithm which can maximize the expectation of the probability distribution being the likelihood function. According to the EM algorithm, the initial value of the desired parameter is set, the likelihood (expected value) is calculated from the initial value, and in many cases, using the condition that the partial differential of the likelihood function becomes zero, the maximum likelihood parameters can be calculated by iterative calculation. In the Hasselblad iterative arithmetic operation performed by using the EM algorithm, the initial value of the parameter to be obtained is set, the likelihood (expected value) is calculated from the initial value, and furthermore, under the condition that the partial differential of the likelihood function becomes zero, the maximum likelihood parameter is calculated by carrying out the iterative calculation.

<Particle Type Estimation Process>

In order to execute the particle type estimation process shown in FIG. 27, there will be described below the processes such as the data file creation process (step S1), the probability density function estimation process (step S2), the particle number estimation process (step S3) and the calculation process of the estimated particle type distribution (step S4).

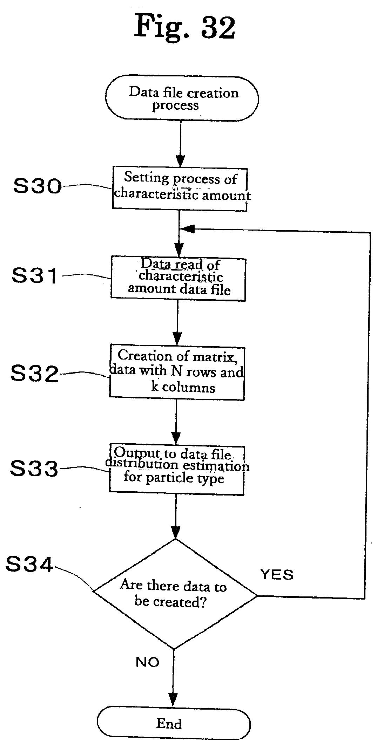

FIG. 32 shows the data file creation process (step S1) executed by the data file creation program.

By using the input means 6 of the PC 1, it is possible to perform the designation operations of k (2 in the embodiment) feature values for creating the data file. The combination input of the designated feature values is set in the RAM 23 (step S30). The data of the feature value data file for each feature value setting is read into the work area of the RAM 23 (step S31). The feature value data file is estimated and extracted by the BL estimation process in FIG. 22 and the feature value estimation process in FIG. 26 and stored in the file.

The matrix data of N rows and k columns is created by designating k feature values used for the number estimation (step S32). The created matrix data is outputted to the particle type distribution estimation data file and stored for each designated feature value (step S33). Upon completion of creation of all the data files for the designated feature value, the process ends (step S34).

FIG. 33 shows the estimation process of the probability density function (step S2) executed by the probability density function module program. In the probability density function estimation process, the estimation process of the probability density function in two feature values is performed based on the Equation 6.

Data of the data file of the probability density function estimation target created in the data file creation process (step S1) is read to form the matrix [D1 of N rows and 2 columns (steps S20 and S21). The dispersion shown in the following equation 20 for each row of the matrix [D1 is calculated (step S22).

[Equation 201

$$\sigma^2_{D_\beta}, \sigma^2_{D_\gamma}$$

Next, the dispersion parameter shown in the following Equation 21 is set using the standard deviation coefficient c as shown in the following Equation 22 (Step S23).

[Equation 211

$$\sigma^2_\beta, \sigma^2_\gamma$$

[Equation 221

$$\sigma^2_\beta = c^2 \sigma^2_{D_\beta} \quad \text{and} \quad \sigma^2_\gamma = c^2 \sigma^2_{D_\gamma}$$

The probability density function is obtained by substituting each line of the dispersion parameter and matrix [D1 as teacher data shown in the following Equation 23 and stored in the predetermined area of the RAM 23 (Steps S24 and S25). The process in steps S20 to S25 is performed until the probability density function is derived from all the process target data (step S26).

[Equation 231

$$\mu^i_\beta, \mu^i_\gamma$$

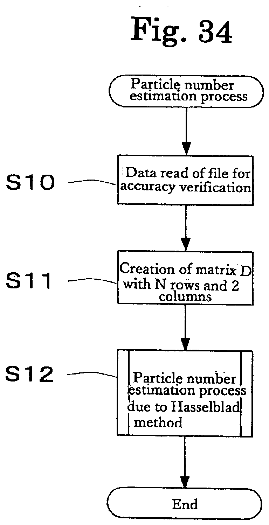

FIG. 34 shows the particle number estimation process (step S3).

First, similarly to the above-described steps S20 and S21, the data of the data file of the particle number estimation target created in the data file creation process is read, and the matrix [D1 of N rows and 2 columns is created (step S10, S 11). For the matrix [D1 data, the estimation process by the Hasselblad iteration method is executed (step S12).

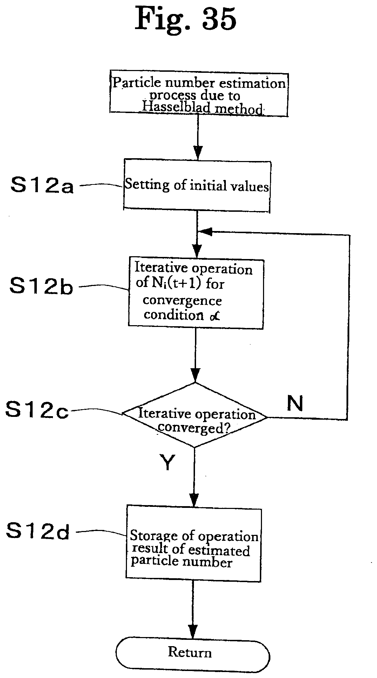

FIG. 35 shows the particle number estimation process due to the Hasselblad iteration method executed by the EM algorithm. FIG. 36 shows the process procedure by the EM algorithm.

First, after setting the initial value (process 23A), the number calculation based on the probability density function is sequentially executed (process 23B) (steps S12a and S12b). The iteration of the number calculation is executed until the convergence condition shown in (23C) is satisfied (step S12 c). The execution result (the estimated number data for each particle type) of the EM algorithm is stored in the predetermined area of the RAM 23 (step S12d).

In step 4, the estimated number data for each particle type obtained by the particle number estimation process is edited into the particle type number distribution data, and according to display designation, the histogram display output to the display means 7 becomes possible. Although not shown in FIG. 27, in the present embodiment, when designation of dispersion diagram output is received, it is possible to display and output the dispersion diagram of particle types due to the feature value data.

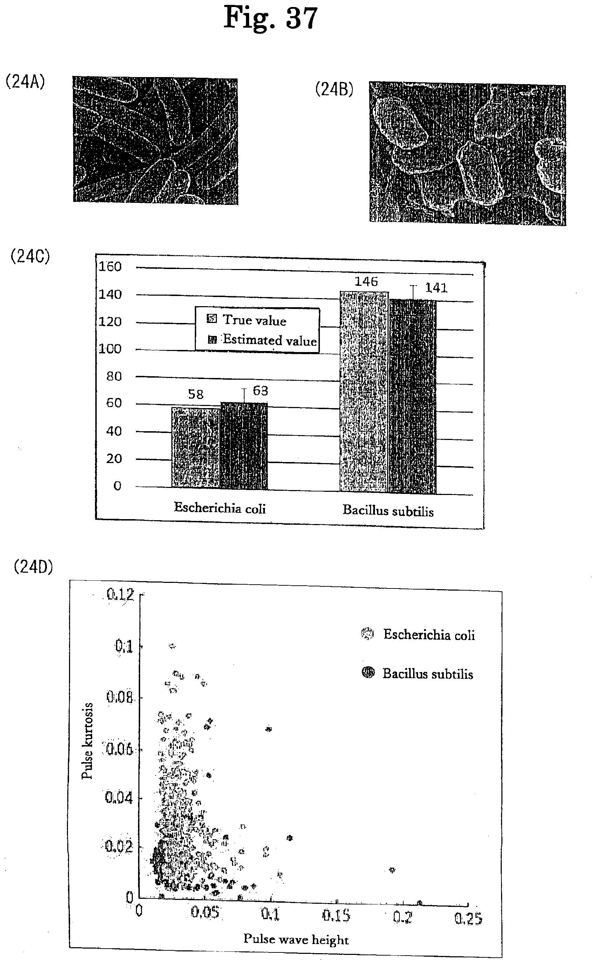

FIG. 37 shows an example of the results analyzed by the particle type number analyzing device according to the present embodiment. The (24A) and (24B) are the microscope enlarged photographs of E. coli and B. subtilis which are particle types to be analyzed. The (24C) and (24D) show the histogram and the dispersion diagram of the estimated number data for each particle type obtained by executing the particle number estimation process focusing on the pulse wave height and the pulse kurtosis as the feature value.

<About Verification 1 of Analytical Accuracy of Particle Type Number due to Feature Value>

The present inventors performed the verification 1 of the analysis performance of the particle type number under the following evaluation conditions using the measured current data of Escherichia coli and Bacillus subtilis in the above example.

The evaluation conditions of verification 1 are as follows.

(1) It is evaluated with the 1000 kHz experimental measurement data of Escherichia coli and Bacillus subtilis.

(2) As the feature values, four first type feature values of wavelength $\Delta t$, wave height h, peak position ratio r, and peak kurtosis k are calculated and used.

(3) The number estimation process on combinations of each feature value is performed.

(4) The actual measurement data of Escherichia coli and Bacillus subtilis are estimated and evaluated at random dividing for learning and testing. This estimation and evaluation is repeated 10 times, and the mean accuracy and standard deviation of them are calculated. In this case, the cross validation is used to evaluate the accuracy close to actual.

(5) A part of measured data of verification particles (Escherichia coli and Bacillus subtilis) are individually number analyzed, the rest are randomly mixed with the predetermined mixture ratio 6 for verification, and the number analysis results are compared. The data mixing program for the mixing random data is stored in the ROM 3, the random mixing of the data is executed using the PC1, and the number estimation for the randomly mixed data is performed. That is, in the matrix data of step S32 of FIG. 32, the random permutation matrix data of N rows and k columns created by the data mixing program is used. For the mixing ratio 6, seven types of mixing ratios of Escherichia coli of 10, 20, 30, 35, 40, 45 and 50% are used. The values of parameters (adjustment factors) m, k and $\alpha$ for BL estimation are set to 100000, 400 and 6, respectively, and the standard deviation coefficient c for estimation of the probability density function is set to 0.1. The convergence condition $\alpha$ for estimating the number of particle types is set to 0.1. As the value of the adjustment factor used for the evaluation, the values obtained by performing more strict adjustment in the same manner as in the simulation example shown in FIG. 25 are used.

The (25A) and (25B) of FIG. 38 show the estimation result data of the verification example using the pulse wavelength and the wave height as the feature values and the verification example using the pulse wavelength and the peak position ratio as the feature values, respectively.

The number of all pulses obtained by this verification was 146 in E. coli and 405 in B. subtilis.

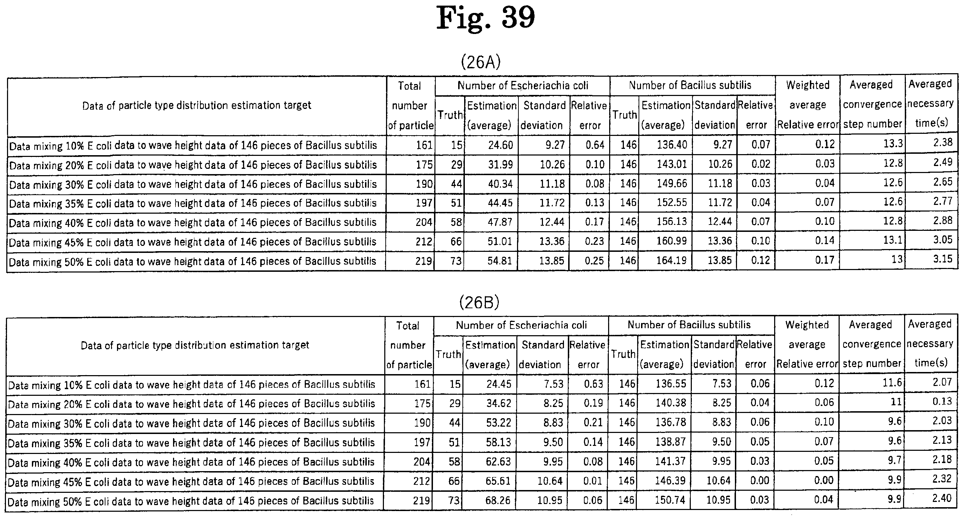

The (26A) and (26B) of FIG. 39 show the estimation result data of the verification example using the spread of the peak vicinity waveform and the pulse wavelength as the feature values and the verification example using the spread of the peak vicinity waveform and the wave height as the feature values.

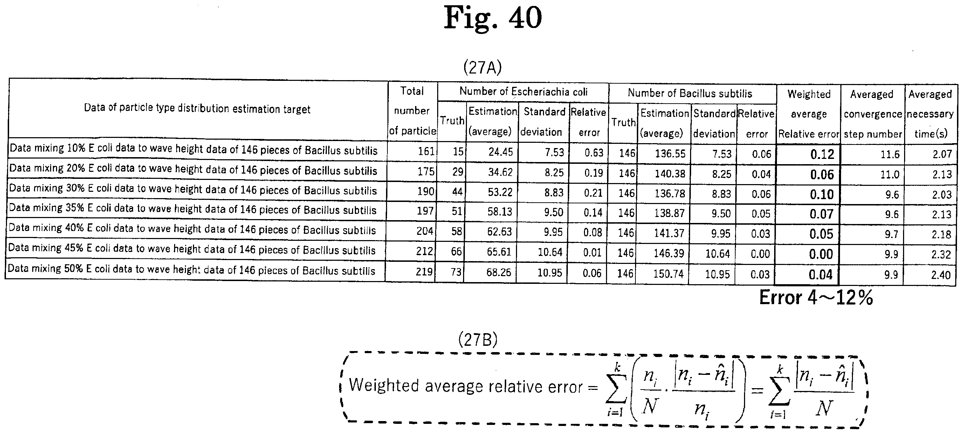

Evaluation of the particle type number can be performed by "weighted mean relative error" represented by the equation shown in (27B) of FIG. 40. "Weighted mean relative error" is the value obtained by multiplying the relative error of each particle diameter by the true number proportion of its particle diameter and adding it for the whole particle diameter.

The (27A) in FIG. 40 shows the number estimation result when the kurtosis and the pulse wave height are used as the feature value.

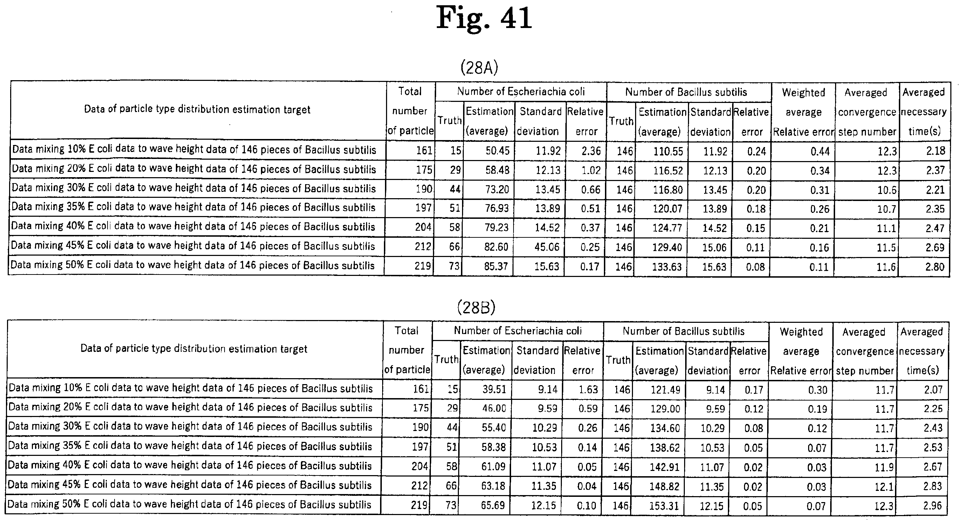

The (28A) and (28B) of FIG. 41 show the number estimation result for each mixing ratio 6 of the example using the pulse wavelength and the pulse wave height as the feature values and the number estimation result for each mixing ratio 6 of the example using the pulse wavelength and the peak position ratio as the feature values, respectively.

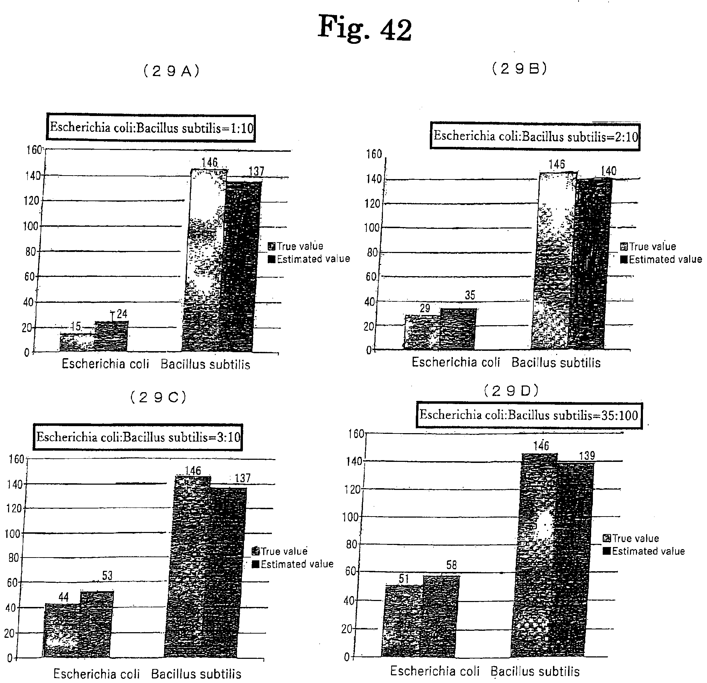

The (29A) to (29D) of FIG. 42 are the histograms showing each number estimation result when the mixing ratios of E. coli and B. subtilis are 1:10, 2:10, 3:10, and 35: 100, respectively.

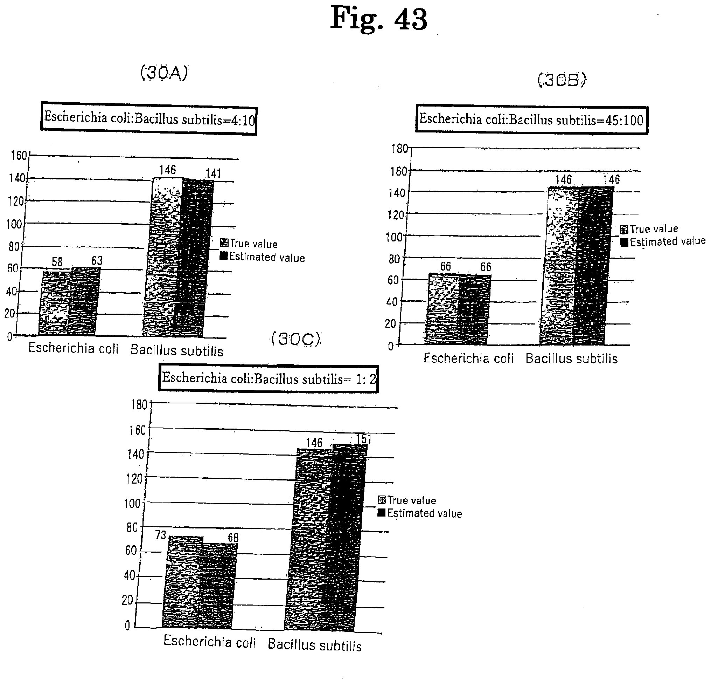

The (30A) to (30D) of FIG. 43 are the histograms showing each number estimation result when the mixing ratios of E. coli and B. subtilis are set to 4: 10, 45: 100 and 1: 2, respectively.

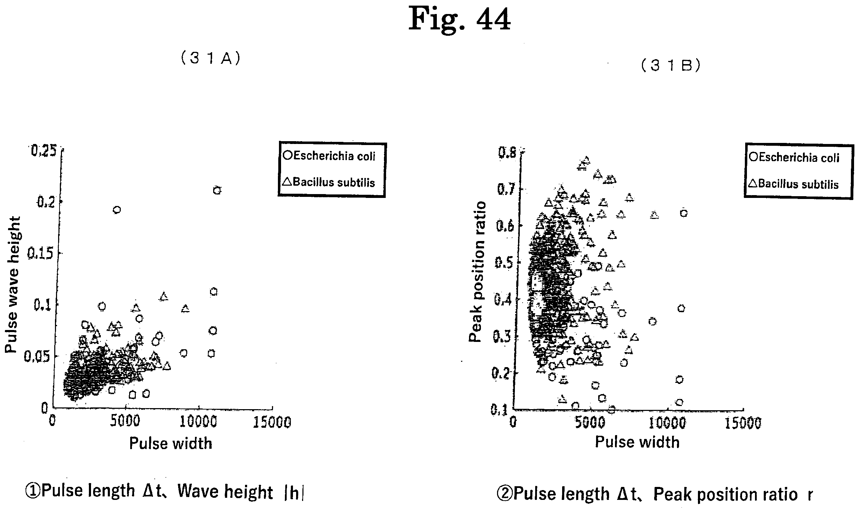

The (31A) and (31B) of FIG. 44 are the diagrams combining dispersed states of respective particles when the pulse wavelength and the pulse wave height are used as the feature values.

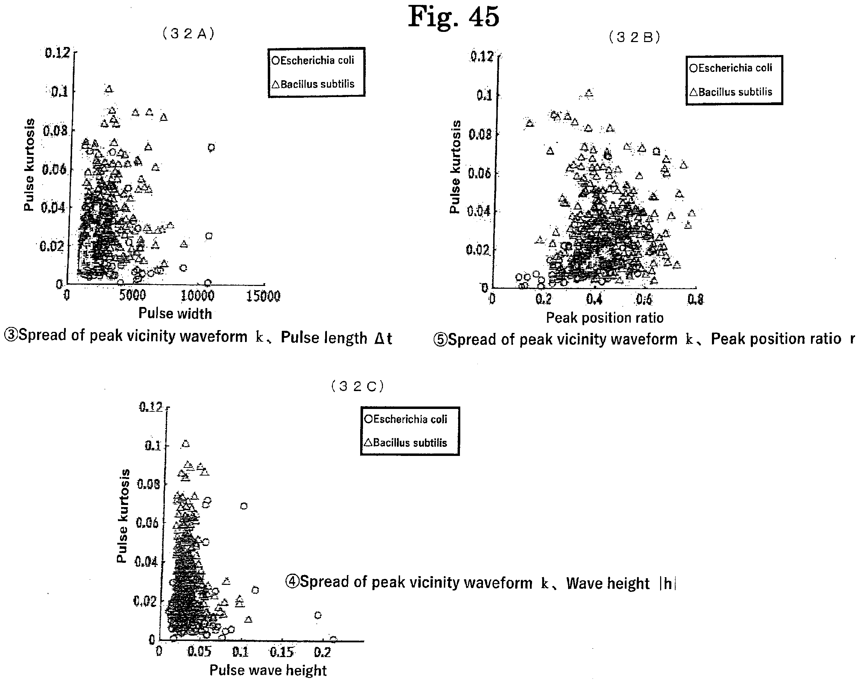

The (32A), (32B) and (32C) of FIG. 45 are the diagrams combining dispersed states of respective particles when the spread of the peak vicinity waveform and the pulse wavelength are used as the feature values, the spread of peak vicinity waveform and the peak position ratio are used as the feature value, and the spread of peak vicinity waveform and the pulse wave height are used as the feature values, respectively.

From the above performance evaluation experiments, the following evaluation results were obtained.

(1) In the data scatter diagrams of FIG. 44 and FIG. 45, regarding the four feature values, the feature of E. coli and B. subtilis greatly overlap, but it is recognized that there is a clear difference.

(2) From the estimation result of the type number distribution shown in (27 A) of FIG. 40 etc., the combination of the feature values between the pulse wave height and the peak kurtosis is the most accurate among the feature values of this evaluation verification, and the analysis accuracy of 4 to 12% can be obtained in the evaluation of the weighted mean relative error. In the above-described embodiment, all four types of feature values are extracted, but only a part of feature values (for example, pulse wave height and peak kurtosis) may be extracted for the number analysis based on above verification result.

<About Verification 2 of Analytical Accuracy of Particle Type Number due to Feature Value>

Using the measured current data of E. coli and B. subtilis in the above example, the verification 2 of the analysis performance of particle type number different from verification 1 is performed. In the verification 2, unlike the verification 1, the feature values of the first type and the second type (13 types of (1) to (13)) are calculated and used, and there were verified the relationship between the feature value and the number of sampling data, and the analysis performance according to each combination.

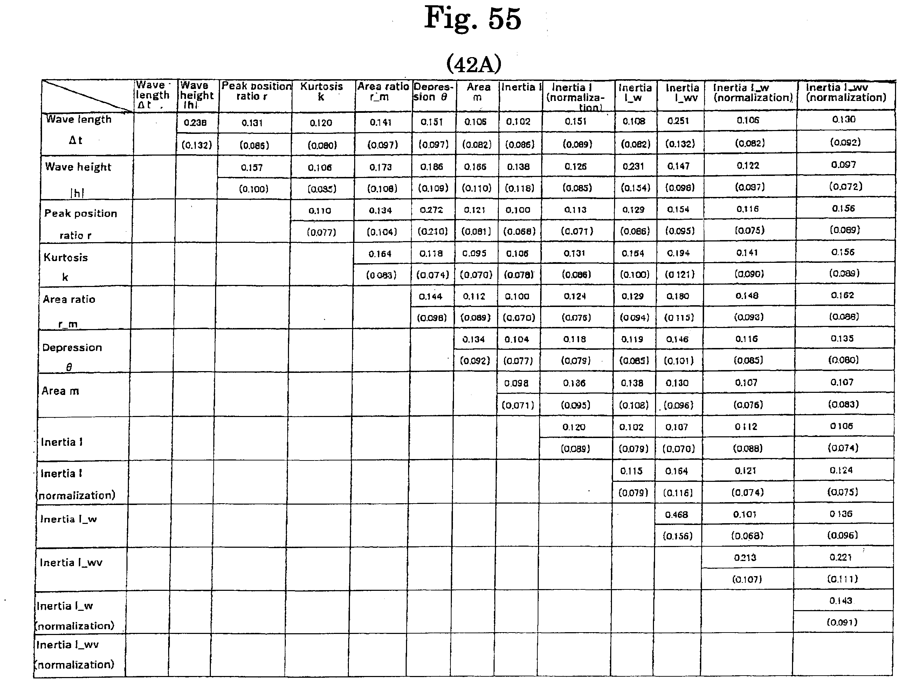

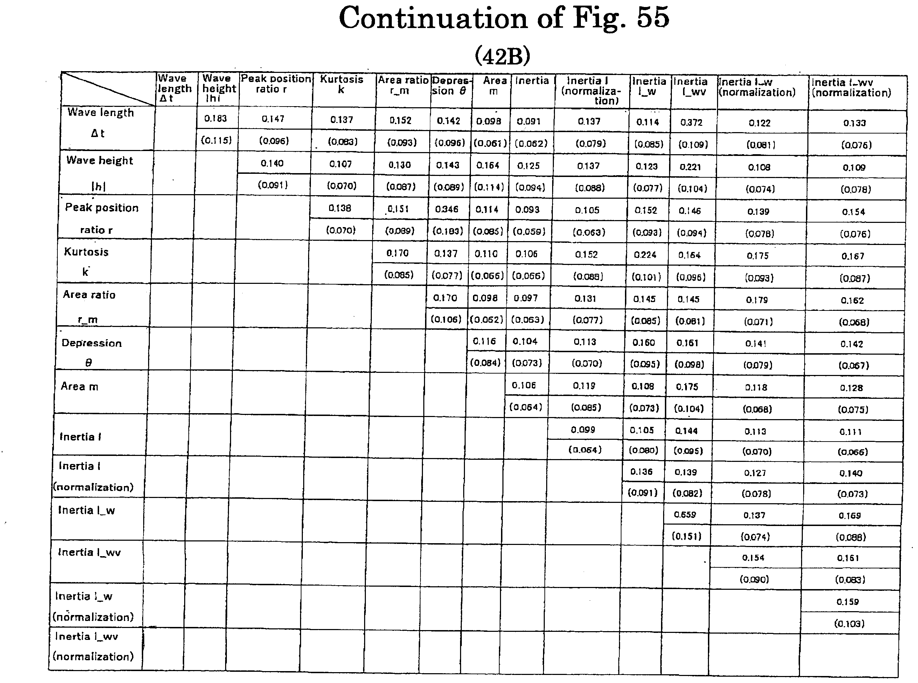

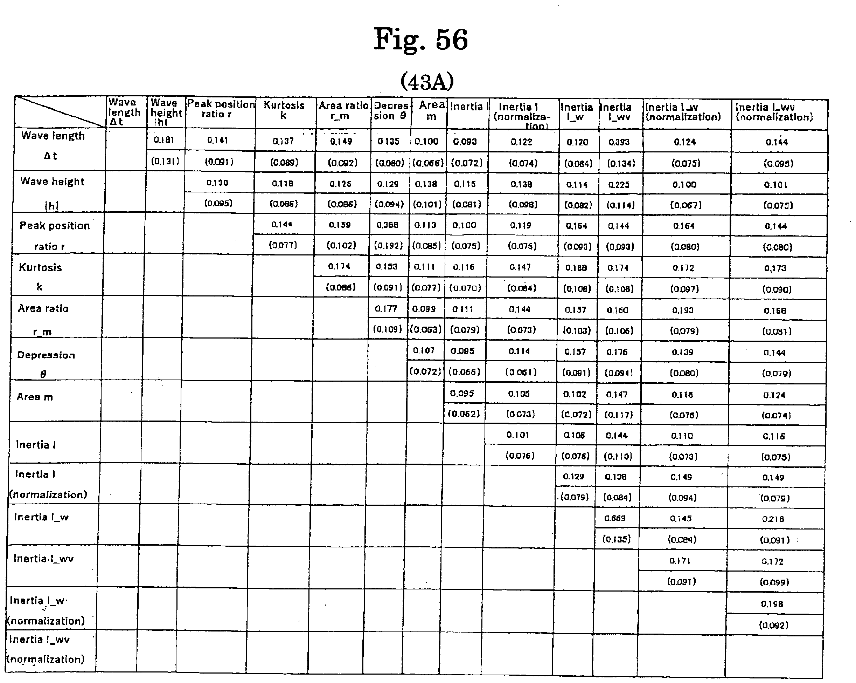

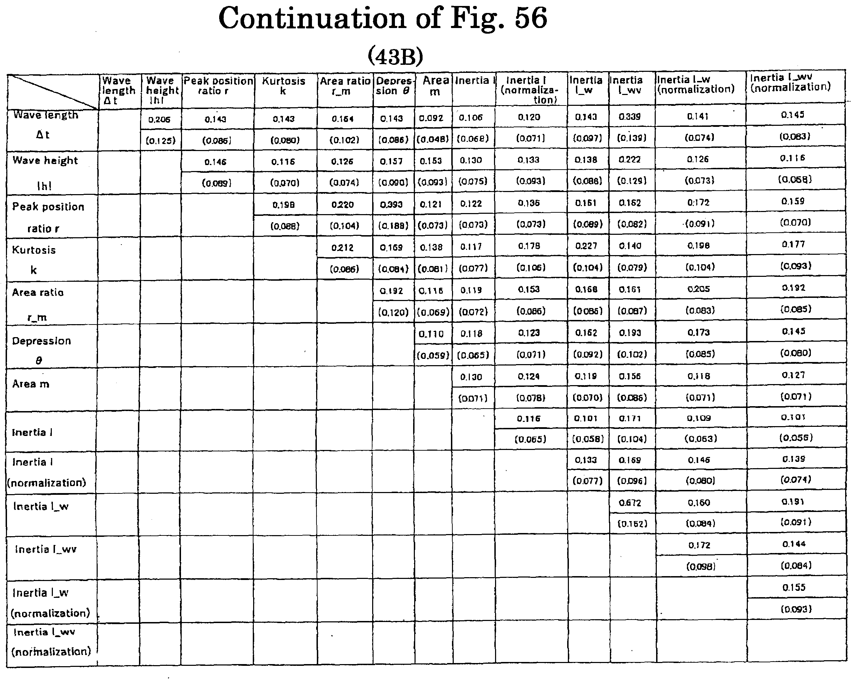

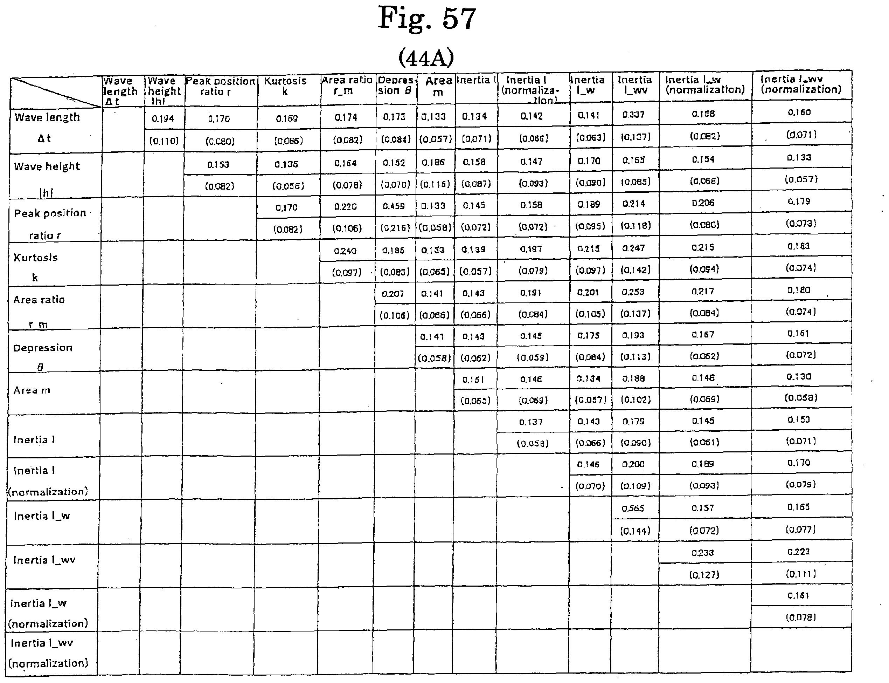

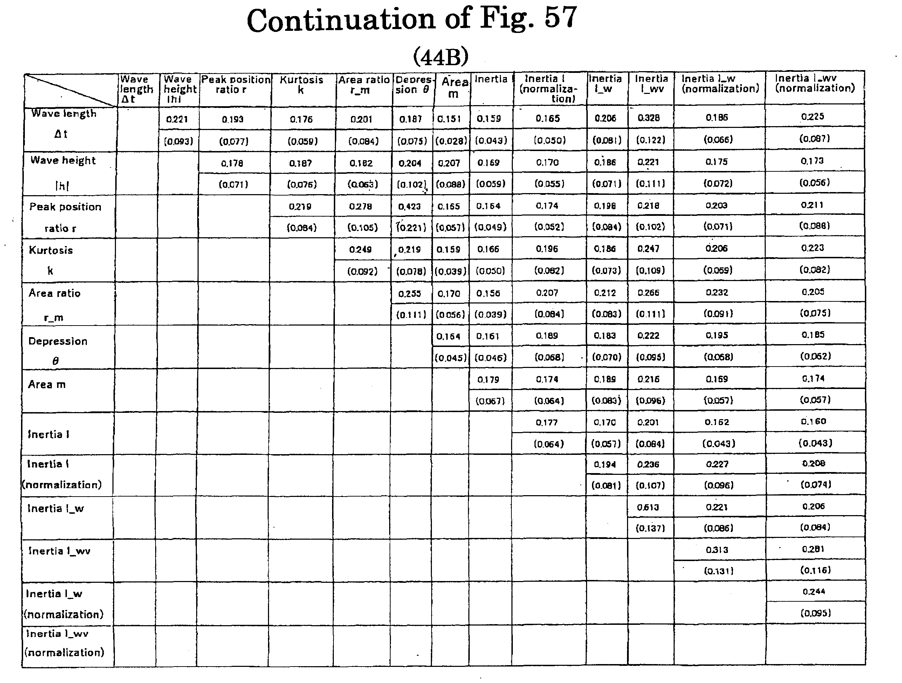

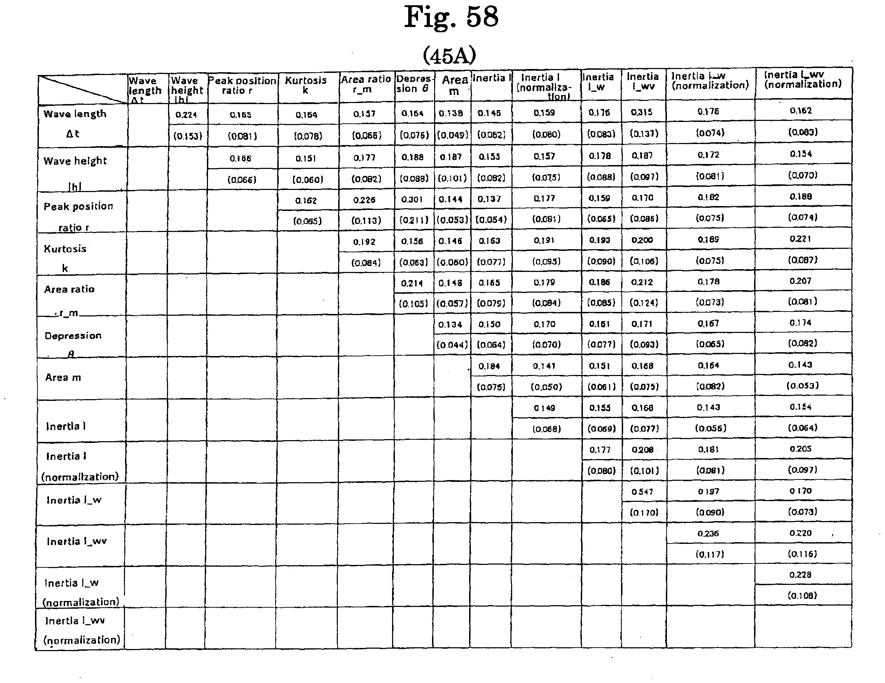

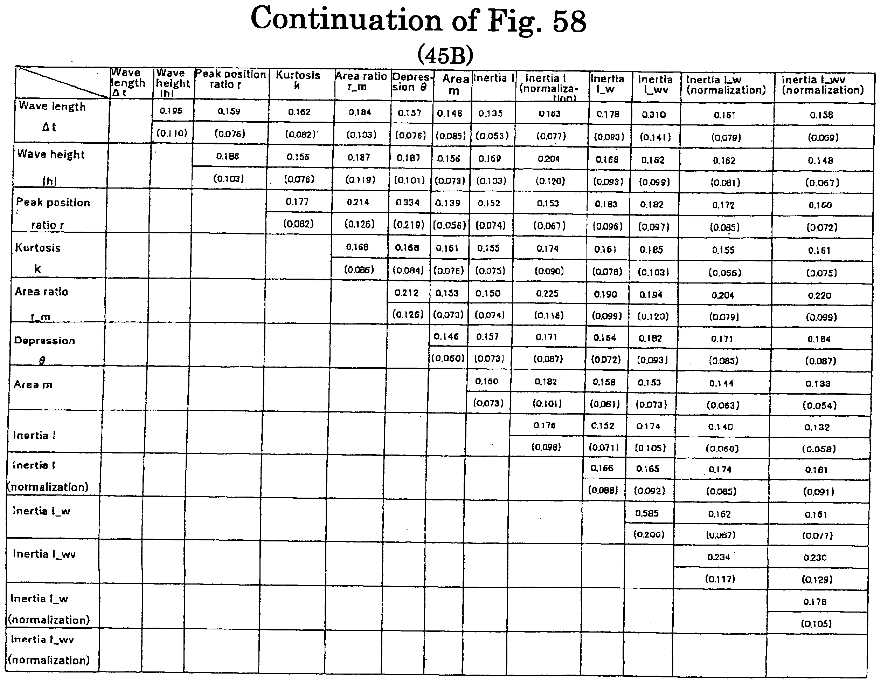

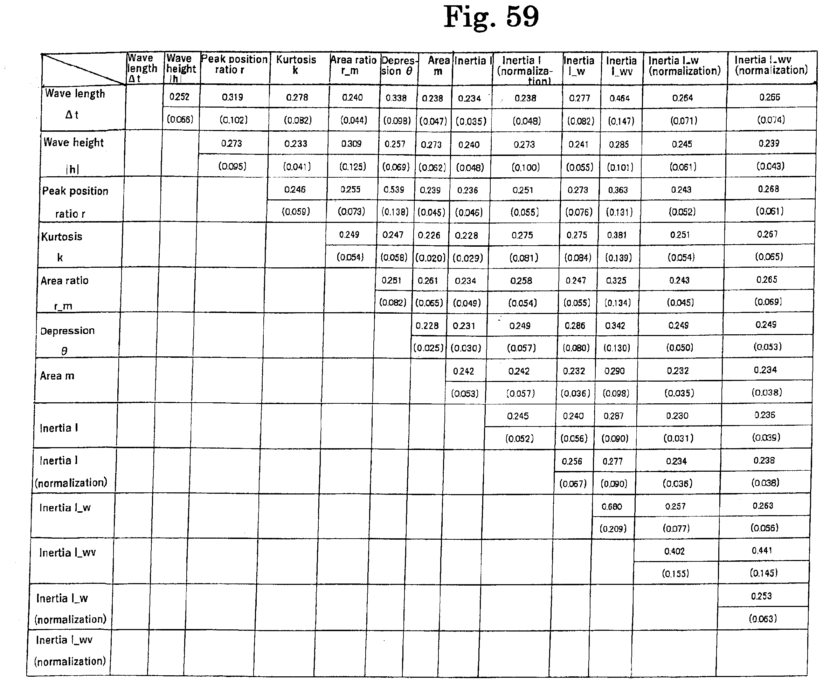

The (42A) and (42B) in FIG. 55 show the estimation evaluation results concerning each feature value combination when sampling is performed at 1 MHz, 500 kHz among all data. The (43A) and (43B) of FIG. 56 show the estimation evaluation results concerning each feature value combination when sampling is performed at 250 kHz and 125 kHz among all data. The (44A) and (44B) in FIG. 57 show the estimation evaluation results concerning each feature value combination when sampling is performed at 63 kHz and 32 kHz among all data. The (45A) and (45B) in FIG. 58 show the estimation evaluation results concerning each feature value combination when sampling is performed at 16 kHz and 8 kHz among all data. FIG. 59 shows the estimation evaluation results concerning each feature value combination when sampling is performed at 4 kHz.

The estimation evaluation results for each combination in these tables are obtained by the cross validation method in the same as (4) of verification 1. The mean accuracy is described in the upper side and the standard deviation indicated in parenthesis is in the lower side. The inertia I, inertia I (normalization), inertia I_w, inertia I_wv, inertia I_w (normalization), and inertia I_wv (normalization) in the table, respectively, show the feature values as the time inertia moment of (8), the normalized time inertia moment of (9), the wave width mean value inertia moment of (10), the wave width dispersion inertia moment of (12), the normalized wave width mean value inertia moment of (11), and the normalized wave width dispersion inertia moment of (13).

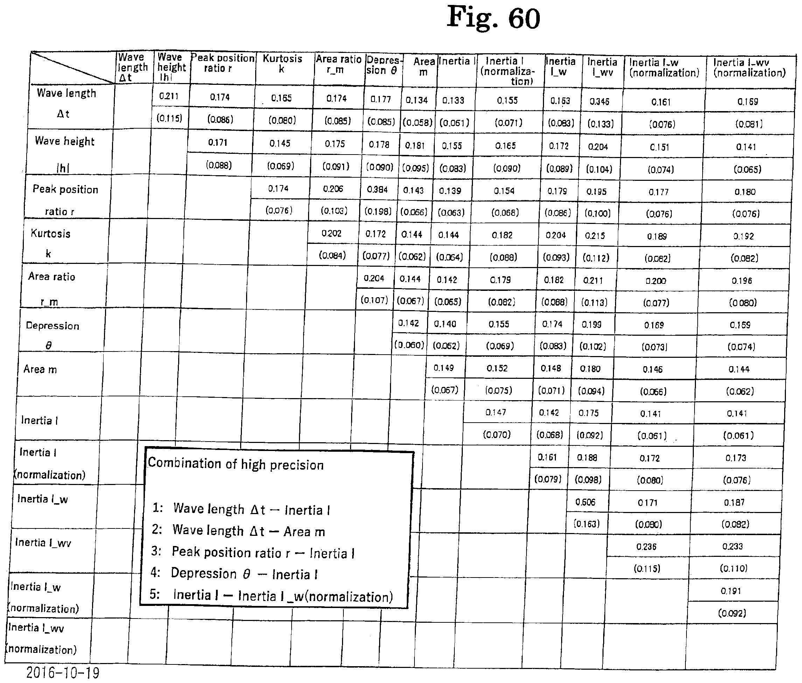

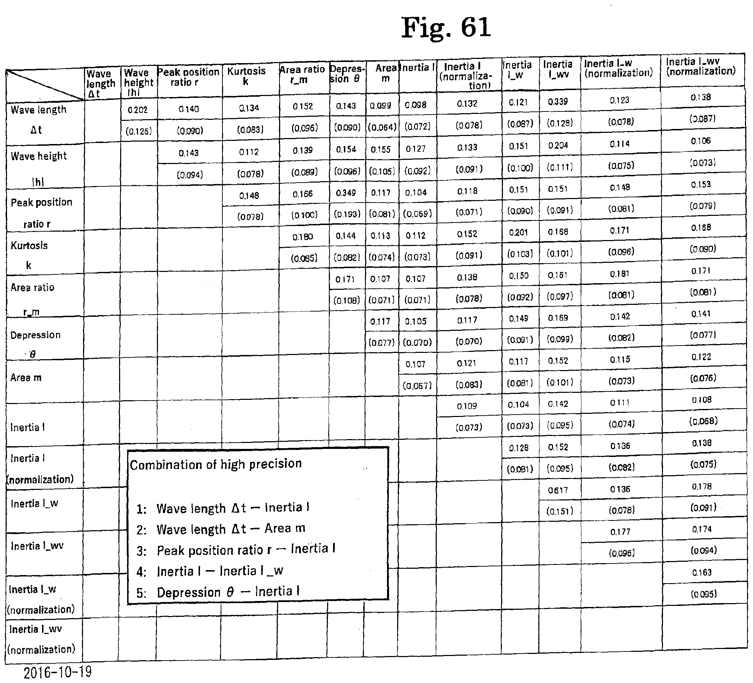

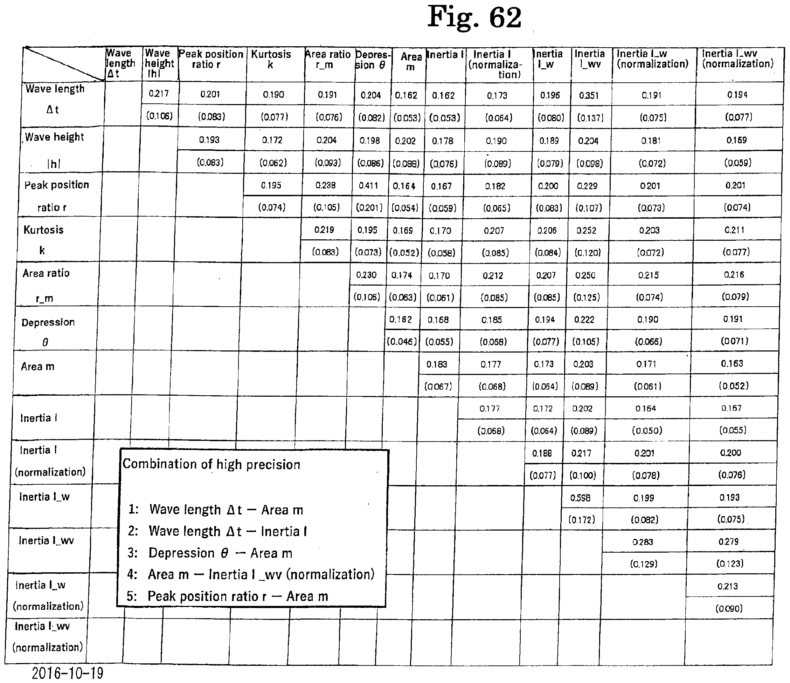

FIG. 60 shows the estimation evaluation result concerning each feature value combination among all sampling data. FIG. 61 shows the estimation evaluation result concerning each feature value combination when the high-density sampling is performed at 1 MHz to 125 kHz among all data. FIG. 62 shows the estimation evaluation result concerning each feature value combination when the low-density sampling is performed at 63 kHz to 4 kHz among all data.

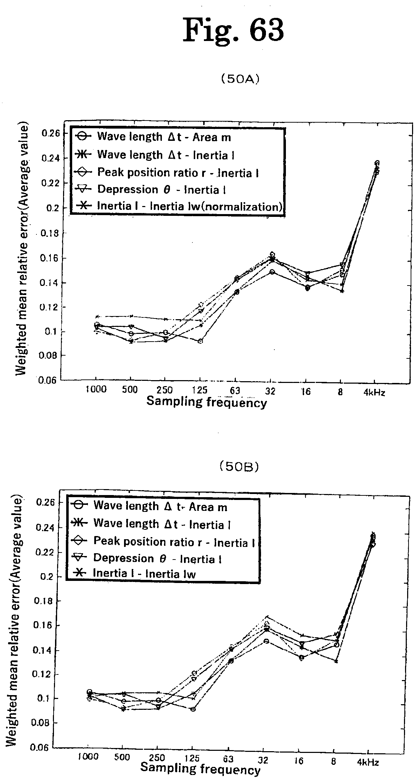

FIG. 63 shows the relationship between the sampling frequency and the weighted mean relative error (mean value) for the combination of the top five types of feature values that can obtain the high number estimation accuracy when all sampling data are used (50 A) and when the sampling is performed at high density (50 B). The combinations of the feature values in the top five in FIG. 63 are the wavelength $\Delta t$ - area m, the wavelength $\Delta t$ - inertia I, the peak position ratio r - inertia I, the depression 0 - inertia I, the inertia I - inertia I_w (normalization).

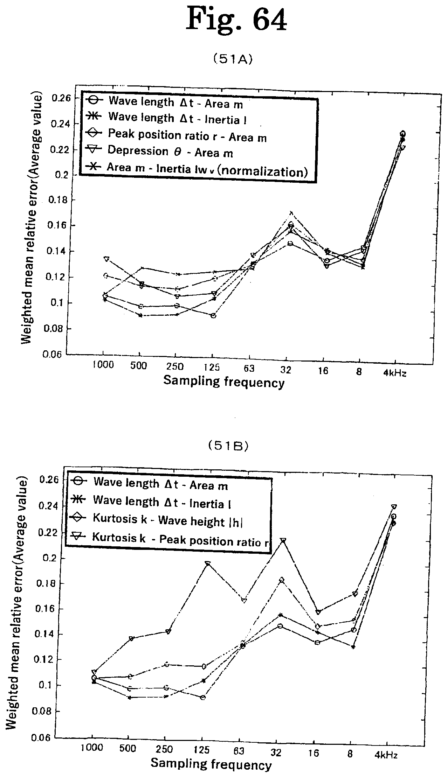

FIG. 64 shows the graph (51A) between the sampling frequency and the weighted mean relative error (mean value) with respect to the combination of the top five types of feature values that can obtain the high number estimation accuracy when the sampling is performed with low density, and shows the graph (51B) between the sampling frequency and the weighted mean relative error (mean value) with respect to the combination of the four types of feature values when all the sampling data are used.

The values on the vertical axis in FIGS. 63 and 64 are the mean values of weighted mean relative errors obtained by performing 50 cross validations. The combination of the top five feature values in (51A) is wavelength $\Delta t$ - area m, wavelength $\Delta t$ - inertia I, peak position ratio r - area m, depression 0 - area m, and area m - inertia I_wv (normalization). The combinations of the four types of feature values in (51B) are wavelength $\Delta t$ - area m, wavelength $\Delta t$ - inertia I, kurtosis k - wave height |h|, kurtosis k - peak position ratio r.

The results obtained from Verification 2 are as follows.

(R1) As shown in FIG. 60 and FIG. 63, when all the sampling data are used, in the combinations of the top five types of feature values such as the wavelength $\Delta t$ - inertia I, the wavelength $\Delta t$ - area m, the peak position ratio r - inertia I, the depression 0 - inertia I and inertia I - Inertia I_w (normalization), the high number estimation accuracy can be obtained. The number estimation accuracy (weighted mean relative error) due to the combinations of these feature values is, for example, about 9 to 10% in the sampling region of 250 to 1000 kHz with the wavelength $\Delta t$ - inertia I, about 9 to 10% in the sampling region of 125 to 250 kHz with the wavelength $\Delta t$ - area m and about 13 to 15% in the sampling region of 16 to 63 kHz with the wavelength $\Delta t$ - inertia I.

(R2) As shown in FIG. 61, when it is used the high-density sampling data smaller than full sampling data, the feature value giving the high number estimation accuracy are, if showing the combination of the top five types, the wavelength $\Delta t$ - inertia I, the wavelength $\Delta t$ - area m, the peak position ratio r - inertia I, the inertia I - inertia I_w, and the depression angle 0 - inertia I. The number estimation accuracy (weighted mean relative error) due to the combination of these feature values is, for example, about 9 to 10% in the sampling region of 250 to 1000 kHz with the wavelength $\Delta t$ - inertia I, about 9 to 10% in the sampling region 125 to 250 kHz with the wavelength $\Delta t$ - area m, and about 13 to 15% in the sampling region of 16 to 63 kHz with wavelength $\Delta t$ - inertia I.

(R3) As shown in FIG. 62, when it is used the low-density sampling data much smaller in comparison with high-density sampling data, the feature value giving the high number estimation accuracy are, if showing the combination of the top five types, the wavelength $\Delta t$ - area m, wavelength $\Delta t$ - inertia I, depression 0 - area m, area m - inertia I_wv (normalization), peak position ratio r - area m. The number estimation accuracy (weighted mean relative error) due to the combination of these feature values is about 9 to 10% in the sampling region of 250 to 1000 kHz with the wavelength $\Delta t$ - inertia I, about 9 to 10% in the sampling region of 125 to 250 kHz with the wavelength $\Delta t$ - area m, and about 13 to 16% in the sampling region of 16 to 63 kHz with wavelength $\Delta t$ - inertia I.

(R4) As can be seen from (R1) to (R3), the highly accurate number estimation can be carried out even by using the combination of feature value of the first type and the second type. Furthermore, according to the number analyzing method of the present invention, even if the sampling number is not sufficiently large, when the predetermined sampling number can be obtained, the number analysis can be performed with the same accuracy as when it is sufficient. For example, in the combination of the kurtosis k and the peak position ratio r examined in Verification 1, a maximum error of 12% was generated, but for example, in the case of using the feature value of the wavelength $\Delta t$ - inertia I, it is possible to perform the number estimation process with high accuracy of about 9% using the high density sampling data at 1 MHz to 125 kHz even if all data is not used, that is, it is partial data. Therefore, the number analysis function according to the present embodiment can be applied not only to the stationary number analysis, but also to the quarantine inspection and the medical site requiring urgency, so that it can be used as a suitable inspection tool that can be implemented quickly for judgement of the presence or absence of particles or the number of bacteria or the like.

<About Verification 3 of Number Analysis Process Time>

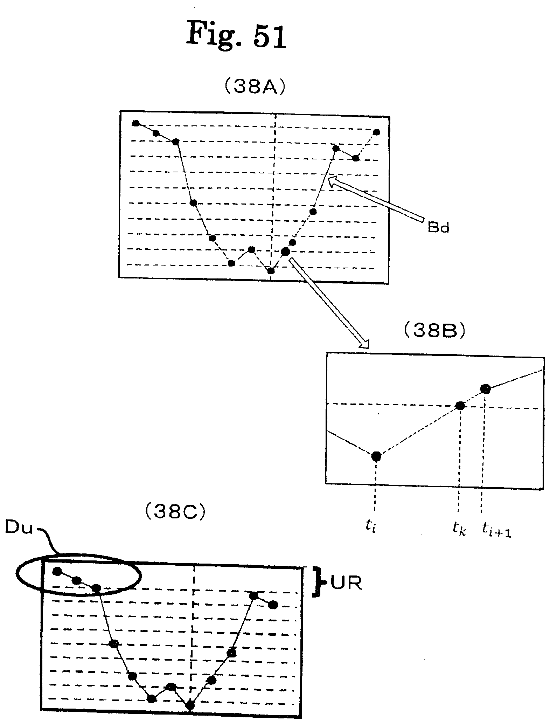

Since in the number estimation, the calculation time is required for the iterative calculation due to the Hasselblad method, the comparison and examination of the feature values are verified in verification 3 with respect to the relation between the required calculation time and the sampling frequency. In the comparative examination example of Verification 3, there are used four kinds of the feature value combinations such as the wavelength $\Delta t$ - area m, the wavelength $\Delta t$ - inertia I, the kurtosis k - wave height |h| and the kurtosis k - peak position ratio r shown in (51B) of FIG. 51. These combinations are combinations with good cross validation accuracy compared to other combinations. Since the time required for the calculation of the number analysis includes the time required for the feature value creation and the calculation time required for the iterative calculation due to the Hasselblad method, there are compared and studied the calculation time CT1 required for the feature value creation, the calculation time CT2 required for the iterative calculation due to the Hasselblad method and their total calculation time CT3 (= CT1 + CT2). In this case, each required calculation time is the mean value of each calculation time obtained by performing 50 cross validations.

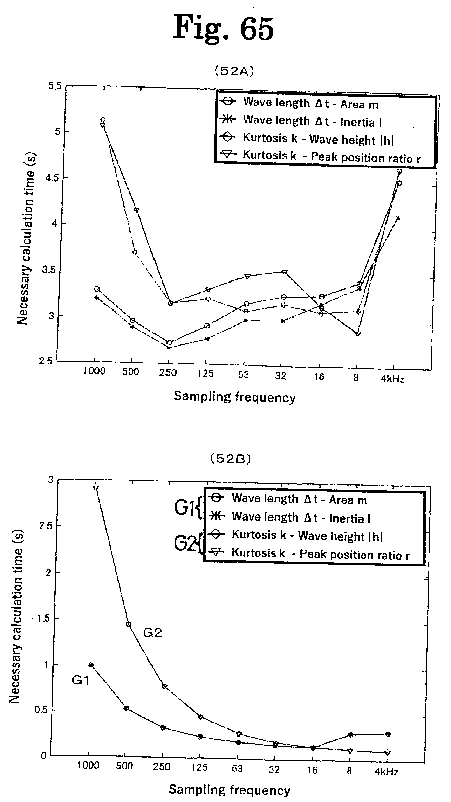

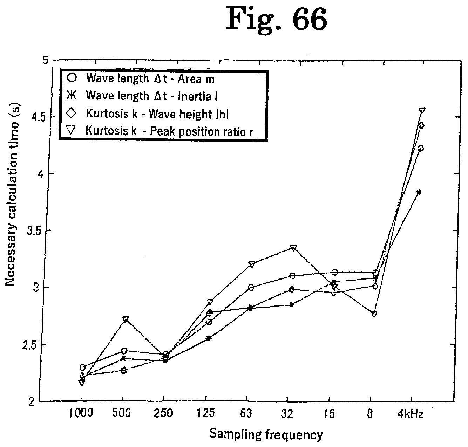

FIG. 65 is the graph (52A) of the sampling frequency (kHz) - the required calculation time (second) showing the total calculation time CT3 for each of the four types of feature value combinations, and the graph (52B) of the sampling frequency (kHz) - the required calculation time (second) showing the calculation time CT1 required for the feature value creation with respect to each of feature value combinations. FIG. 66 is the graph of the sampling frequency-the required calculation time (second) showing the calculation time CT2 for each feature value combination.

As shown in (52 A), the feature value combination G1 of the wavelength $\Delta t$ - area m and the wavelength $\Delta t$ - inertia I is almost the same total calculation time, and the feature value combination G2 of the kurtosis k - wave height |h| and the kurtosis k - the peak position ratio r has approximately the same total calculation time. As shown in (52 B), the calculation time required for generating each feature value of the feature value combination G1 is the same, and the calculation time required for generating each feature value of the feature value combination G2 is the same. As shown in FIG. 53, the time required for the iterative calculation due to the Hasselblad method can be processed in a short time of about 3, 5 seconds or less in the sampling region at 1 MHz to 16 kHz in any of the feature value combinations G1 and G2.

Obviously from the comparison result of the feature value combinations G1 and G2 of verification 3, even if it is the same type combination in the first type and the second type, even if it is the different mixing combination, the required calculation time using the feature value can be shortened. Therefore, according to the number analyzing device of the present embodiment, in addition to the stationary number analysis, for example, it is possible to quickly perform the process of discriminating the presence or absence of particles and the number of bacteria or the like in the quarantine inspection or the medical field requiring urgency.

As can be understood from the above performance evaluation, based on the data group of the detection signal detected by the nanopore device 8, there is executed the particle type distribution estimation program which is the number deriving means in the computer control program (number analysis program), and it is possible to perform the probability density estimation from the data group based on the feature value showing the feature of the waveform of the pulse signal corresponding to the particle passage obtained as the detection signal and it is possible to derive the number of the particle type. Therefore, by using the number analyzing device, it is possible to analyze the number or the number distribution corresponding to the type of analyte such as, for example, bacteria, microparticulate material, etc. with high accuracy, so simplification and cost reduction in the number analyzing inspection can be realized. By incorporating the detection signal from the nanopore device 8 directly into the number analyzing device so that data can be stored, the particle type integration analyzing system integrating inspection and analysis may be constructed.

The probability density estimation is performed from the data group based on the feature value, and the result of deriving the particle type number is displayed on the display means 7 as the output means or printed out on the printer. Therefore, according to the present embodiment, highly accurate derivation results (particle number, particle number distribution, estimation accuracy, etc.) can be notified promptly in the output form of, for example, the histogram or the dispersive diagram, so that for example, it is possible to use the number analysis function according to the present embodiment as the useful inspection tool in the quarantine portion or the medical field requiring urgency.

The present invention can be applied not only to a computer terminal such as a specific PC or the like mounted with a identification process program but also to a storage medium for identification analysis which stores a part or all of the identification process program. That is, since the identification analysis program stored in the identification analysis storage medium can be installed in a predetermined computer terminal and the desired computer can be operated to perform the identification analysis operation, it is possible to carry out the identification analysis simply and inexpensively. As the storage medium applicable to the present invention, there are a flexible disk, a magnetic disk, an optical disk, a CD, an MO, a DVD, a hard disk, a mobile terminal, or the like, and any storage medium readable by a computer can be selected and used.

FIG. 69 shows the classification analysis process according to the present embodiment.

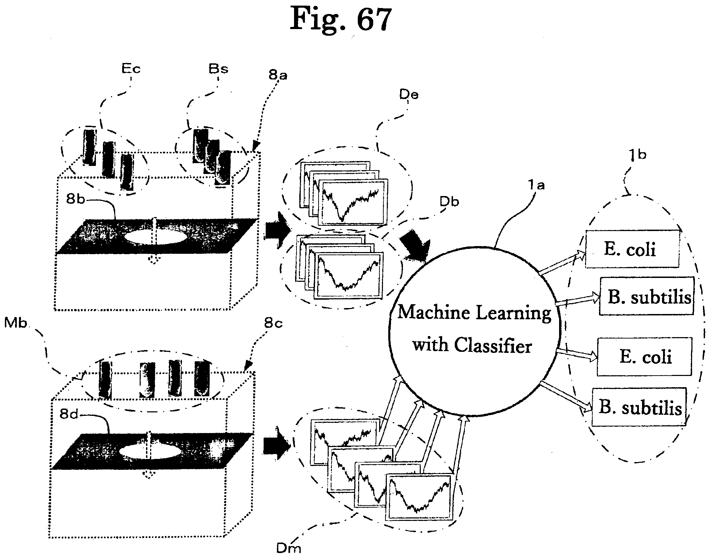

The computer analysis unit 1a in FIG. 67 corresponds to the PC 1 of the present embodiment. As preparation work for the analysis process, the removal process of nonconforming data, the designation of feature values, and the input of known data and data to be analyzed to the PC 1 are performed in the input process (step S100). As the feature values, the feature values of a part or all of the first type and the second type shown in the above (1) to (15) or the combination of one or more can be designated in advance in the input process.

For example, when E. coli Ec and B. subtilis Bs are used as analytes of which particle types are specified (specific analytes), each of the specific analytes is measured by the nanopore device 8a, and each pulse signal data is input to the PC 1 as known data, and the input data is stored in a memory area for storing known data in the RAM 4. When the content state of the specific analytes is unknown in the analysis target, data of the pulse signal obtained by performing the measurement by the nanopore device 8a is input as analysis data to the PC 1, and the input data is a memory area for storing analysis data in the RAM 4

When the classification analysis process is activated by the activation operation, it is determined whether or not the known data is input (step S110). When the known data has not been input, the display means 7 displays a guidance prompting the user so as to input the known data. In FIG. 69, the notification process steps according to various guidance displays are omitted. When the known data is input, the input known data is stored in the memory area for storing the known data in the RAM 4 and is used to create a feature value (steps S100 and S101).

When the known data is input, it is determined whether or not the feature value is specified (steps S110 and S111). When the feature value is designated, the vector value data of the feature value designated from the feature value storage data file DA based on the known data of the RAM 4 is taken into the learning data storage area of the RAM 4 (step S113). When the feature value is not specified, the vector value data of all the feature values are taken into the learning data storage area of the RAM 4 from the feature value storage data file DA based on the known data of the RAM 4 (step S112).

Next, it is determined whether or not the analysis data is input (step S114). When there is no analysis data input, the display means 7 displays a guidance prompting input of the analysis data. When the analysis data is input, the acquired analysis data is stored in the analysis data storage memory area in the RAM 4 (step S100). When the analysis data is input, as described, the feature value related to the analysis data is created and stored in the RAM 4 (step S101). When the analysis data is input, the vector value data of the feature value is fetched from the feature value storage data file DB based on the analysis data of the RAM 4 into the variable data storage area of the RAM 4 (step S115).

In the state of acquisition of feature values under the input of the known data and the analysis data, the guidance display for prompting execution of the classification analysis is performed. By performing the predetermined instruction operation in accordance with the guidance display, the classification analysis program is activated and the execution process of the classification analysis due to the machine learning is performed (step S116). In the present embodiment, for example, the machine learning classification analysis program configured by the algorithm based on Random Forest Method is stored in advance in the ROM 3.

The feature value due to the known data is set as the learning data, the feature value obtained from the data to be analyzed is set as a variable and by executing the classification analysis program, the classification analysis relating to the specified analyte in the data to be analyzed can be performed. At the time of execution of the classification analysis program, the pulse waveforms are converted into the numerical vectors of the same dimension, and the classification analysis is performed by identifying the individual pulses and determining how each vector is different.

The classification analysis method due to the machine learning according to the present invention is not limited to Random Forest Method, and, for example, it is possible to use the group learning such as K-nearest Neighbor Algorithm, Naive Bayes Classifier, Decision Tree, Neural Network, Support Vector Machine, Bagging Method, and Boosting Method.

When the execution process of classification analysis by machine learning is executed for all of the feature values by analysis data, the classification analysis process is finished, and the output process of the classification analysis result is performed (step S117). In the output process, for each of analysis data of unknown type, the display means 7 can display a classification result, and as an example of the specific analyte, it is displayed a ratio of those derived from the passage of E. coli Ec or B. subtilis Bs. The display mode that can be output is not limited to the classification result for each analysis data, and the display mode such as the corresponding total number of the analyte (for example, E. coli Ec or B. subtilis Bs) and the corresponding ratio of both can be used.

<Verification of process accuracy of classification analysis process>

About the process accuracy of the above-mentioned classification analysis process, the classification analysis is tried by applying the analysis method based upon various machine learnings and the accuracy of the classification analysis process due to the present embodiment is verified.

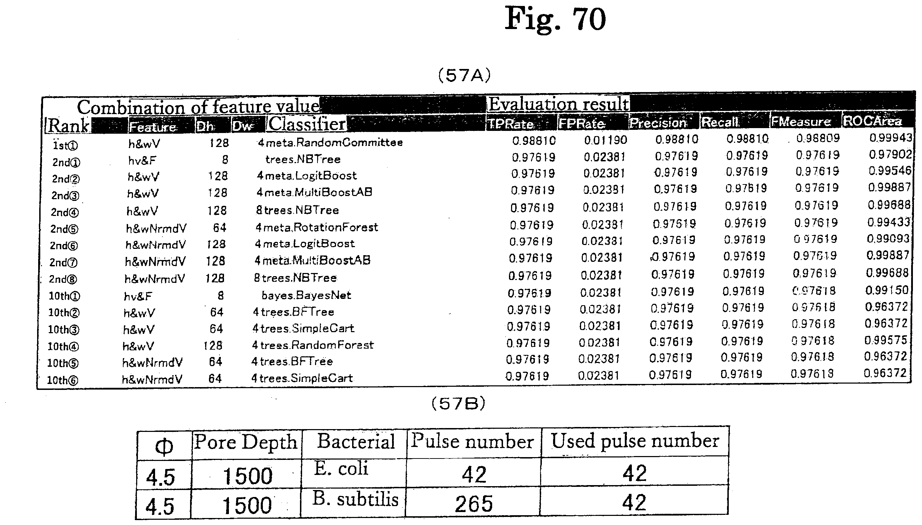

In (57A) of FIG. 70, when the feature value (Feature) and the algorithm of analysis method due to machine learning (hereinafter, referred as a classifier) are variously combined using the analysis sample shown in FIG.57B, the evaluation result obtained by performing the classification analysis process (refer to FIG. 69) according to the present invention is shown.

The analysis sample is two kinds of bacterial species (E. coli, Bacillus subtilis) as shown in (57B). For each bacterial species, the pulse shapes are obtained by measuring the passing waveform using the micro-nanopore device 8 with the inner diameter of through-hole 12 of 4.5Φ and the penetration distance (pore depth) of through-hole 12 of 1500mm and the 42 signal data (all of the measurement pulses in the case of E. coli and 42 pulses out of 265 measurement pulses in the case of B. subtilis) are used. During execution of the classifier, about 90% of the pulse signal data are used as the learning data and the remaining data are assigned to the variables.

As shown in (57A), the evaluation items are represented by F-measure (F-Measure), which consists of true positive rate (TPRate), false positive rate (FPRate), precision rate (Precision), recall rate (Recall), F value (FMeasure) and receiver operating characteristic curve area (ROC (Receiver Operating Characteristic) Curve Area).

FIG. 71 is the explanatory diagram of F-measure.

As shown in (58A), for the real numbers of the two bacterial species (E. coli real number: P, B. subtilis real number: N), when the predicted value of each bacterial species is assigned, the F-measure is expressed by 2TP / (2TP + FP + FN) as shown in (58B), assuming that the sum of true positive (TP), false positive (FP), true negative (FN) and false negative (TN) in each combination is 1.

In this verification, the classification analysis is attempted on about 4000 patterns by using the 67 kinds of classifiers with different algorithms and using various feature values or combinations of feature values. As a result, the significant analysis results are obtained for the combinations of 60 kinds of feature values. The (57A) of FIG. 70 is the table showing the classification results in the top 10 of excellent F-measure obtained by this verification.

As shown in (57A), the feature values in the top ten places include the 13-dimensional feature value vector (abbreviated as Fhv&Fj in the table) arranging 13 kinds of feature values of (1) to (11), (14) and (15), a combination (abbreviated as Fh&wVj in the table) of the wave height vector (abbreviated as Fhj in the table) and the mean value vector of (10) (abbreviated as FwVj in the table), and a combination (abbreviated as Fh&wNrmdVj in the table) of the wave height vector and the normalized mean value vector of (11) (abbreviated as FwNrmdVj in the table). The most excellent classification accuracy in (57A) is the case of the Random Forest Method classifier (F4 meta. Random Committeej) using a combination of h&wV as the feature value, and the classification accuracy shows the high accuracy of approximately 98.9%.

The present invention is not limited to a computer terminal such as the specific PC equipped with the classification analysis program, and can be applied to the classification analysis storage medium storing a part or all of the classification analysis program. That is, since the classification analysis program stored in the storage medium for classification analysis can be installed in the predetermined computer terminal and the classification analysis operation can be performed on the desired computer, the classification analysis can be performed simply and inexpensively. As the storage medium to which the present invention can be applied, any of computer-readable storage medium such as flexible disk, magnetic disk, optical disk, CD, MO, DVD, hard disk, and mobile terminal can be selected and used.

It is to be understood that the present invention is not limited to the above-described embodiments, but includes various modifications, design changes and the like within the technical scope without departing from the technical idea of the present invention.

## <INDUSTRIAL APPLICABILITY>

According to the present invention, since the identification of the nonconforming data and the classification analysis are performed with high accuracy, it is possible to be utilized to an information compression technology of DNA storage media and a drug discovery using an artificial base pairing, or in the fields of a fine dust mixed in a measurement sample and an analysis substance contained in a body fluid, it can be applied and developed in the fields of the identification / removal technologies of the nonconforming data derived from the fine substances such as red blood cells, white blood cells and platelets. In particular, the present invention can be applied to data analysis in a detection technique in which a sample containing DNA or RNA and contaminants is analyzed, for example, for performing DNA content analysis in sewage to detect the occurrence of a virus.

## <DENOTATION OF REFERENCE NUMERALS>

| | |
|---|---|
| 1 | Personal computer |
| 2 | CPU |
| 3 | ROM |
| 4 | RAM |
| 5 | Data file storage portion |
| 6 | Input means |
| 7 | Display means |
| 8 | Micro-nanopore device |
| 9 | Chamber |
| 10 | Substrate |
| 11 | Partition Wall |
| 12 | Through-hole |
| 13 | Electrode |
| 14 | Electrode |
| 15 | Power supply |
| 16 | Amplifier |
| 17 | Operational amplifier |
| 18 | Recess portion |
| 19 | Feedback resistor |
| 20 | Voltmeter |
| 21 | Subject |
| 22 | Escherichia coli |
| 23 | Bacillus subtilis |
| 24 | Electrolytic solution |
| MS | measurement space |
| D1 | electrode |
| D2 | electrode |
| ME | current measuring device |

## Claims

1. An identification classification analysis method comprising the steps of

   introducing a sample containing an analyte into a measurement space,
   obtaining pulse signal data detected due to said introduction,
   identifying nonconforming data detected due to elements other than said analyte from said pulse signal data by execution of an identification analysis program included in a computer control program,
   obtaining data to be analyzed through removing said nonconforming data from said pulse signal data, and

performing a classification analysis of said data to be analyzed by execution of a computer control program, wherein

said computer control program includes a classification analysis program that performs said classification analysis using a machine learning,

a feature value is obtained in advance which indicates a feature of waveform form of said pulse signal,

said feature value obtained in advance is set as the learning data for said machine learning,

said feature value obtained from said pulse signal of said data to be analyzed removed said nonconforming data is set as a variable, and

said classification analysis on said analyte is performed by executing said classification analysis program, **characterized in that** said feature value is one or more selected from a group of a wave height value of the waveform in a predetermined time width,

a pulse wavelength $t_a$,

a peak position ratio represented by ratio $t_b/t_a$ of time $t_a$ and $t_b$ leading from the pulse start to the pulse peak,

a kurtosis which represents the sharpness of the waveform,

a depression representing the slope leading from the pulse start to the pulse peak,

an area representing total sum of the time division area dividing the waveform with the predetermined times,

an area ratio of sum of the time division area leading from the pulse start to the pulse peak to the total waveform area,

a time inertia moment determined by mass and rotational radius when the mass is constructive to said time division area centered at the pulse start time and the rotational radius is constructive to time leading from said center to said time division area,

a normalized time inertia moment determined when said time inertia moment is normalized so as that the wave height becomes a reference value,

a mean value vector whose vector component is the mean value of the same wave height position in which the wave form is equally divided in the wave height direction and the mean value of time values is calculated for each division unit in before and after each pulse peak,

a normalized mean value vector which is normalized so as that the wavelength becomes a standard value for said mean value vector,

a wave width mean value inertia moment determined by mass distribution and rotational center when the mass distribution is constructive to mean value difference vector whose vector component is mean value difference of the same wave height position in which the wave form is equally divided in the wave height direction and the mean value of time values is calculated for each division unit in before and after each pulse peak and the rotational center is constructive to time axis of waveform foot,

a normalized wave width mean value inertia moment determined when said wave width mean value inertia moment is normalized so as that the wavelength becomes a standard value,

a wave width dispersion inertia moment determined by mass distribution and rotational center when the mass distribution is constructive to dispersion vector whose vector component is dispersion in which the wave form is equally divided in the wave height direction and the dispersion is calculated from time value for each division unit and the rotational center is constructive to time axis of waveform foot, and

a normalized wave width dispersion inertia moment determined when said wave width dispersion inertia moment is normalized so as that the wavelength becomes a standard value.

2. An identification classification analysis device comprising

a means for introducing a sample containing an analyte into a measurement space, a means for obtaining pulse signal data detected due to said introduction,

a means for identifying nonconforming data detected due to elements other than said analyte from said pulse signal data by execution of an identification analysis program included in a computer control program,

a means for obtaining data to be analyzed through removing said nonconforming data from said pulse signal data, and

a means for performing a classification analysis of said data to be analyzed by execution of said computer control program,

wherein

a nonconforming data storage means (5) is included for storing said nonconforming data, said computer control program includes a classification analysis program that performs said classification analysis using a machine learning,

a feature value is obtained in advance which indicates a feature of waveform form of said pulse signal,

said feature value obtained in advance is set as the learning data for said machine learning,

said feature value obtained from said pulse signal of said data to be analyzed removed said nonconforming data is set as a variable, and

said classification analysis on said analyte is performed by executing said classification analysis program, **characterized in that** said feature value is one or more selected from a group of a wave height value of the waveform in a predetermined time width,

a pulse wavelength $t_a$,

a peak position ratio represented by ratio $t_b/t_a$ of time $t_a$ and $t_b$ leading from the pulse start to the pulse peak,

a kurtosis which represents the sharpness of the waveform,

a depression representing the slope leading from the pulse start to the pulse peak,

an area representing total sum of the time division area dividing the waveform with the predetermined times,

an area ratio of sum of the time division area leading from the pulse start to the pulse peak to the total waveform area,

a time inertia moment determined by mass and rotational radius when the mass is constructive to said time division area centered at the pulse start time and the rotational radius is constructive to time leading from said center to said time division area,

a normalized time inertia moment determined when said time inertia moment is normalized so as that the wave height becomes a reference value,

a mean value vector whose vector component is the mean value of the same wave height position in which the wave form is equally divided in the wave height direction and the mean value of time values is calculated for each division unit in before and after each pulse peak,

a normalized mean value vector which is normalized so as that the wavelength becomes a standard value for said mean value vector,

a wave width mean value inertia moment determined by mass distribution and

rotational center when the mass distribution is constructive to mean value difference vector whose vector component is mean value difference of the same wave height position in which the wave form is equally divided in the wave height direction and the mean value of time values is calculated for each division unit in before and after each pulse peak and the rotational center is constructive to time axis of waveform foot,

a normalized wave width mean value inertia moment determined when said wave width mean value inertia moment is normalized so as that the wavelength becomes a standard value,

a wave width dispersion inertia moment determined by mass distribution and rotational center when the mass distribution is constructive to dispersion vector whose vector component is dispersion in which the wave form is equally divided in the wave height direction and the dispersion is calculated from time value for each division unit and the rotational center is constructive to time axis of waveform foot, and

a normalized wave width dispersion inertia moment determined when said wave width dispersion inertia moment is normalized so as that the wavelength becomes a standard value.


**Patentansprüche**

1.  Verfahren zur Identifikation-Klassifikationsanalyse umfassend die folgenden Schritte:

    Einführen einer Probe, die einen Analyten enthält, in einen Messraum, Erhalten von Impulssignaldaten, die aufgrund der Einführung erfasst werden,

    Identifikation von fehlerhaften Daten, die aufgrund von anderen Elementen als dem Analyten von den Impulssignaldaten durch Ausführung eines Identifikationsanalyseprogramms, das in einem Computersteuerungsprogramm enthalten ist, erkannt werden,

    Erhalten von zu analysierenden Daten durch Entfernen der fehlerhaften Daten aus den Impulssignaldaten und Durchführung einer Klassifikationsanalyse der zu analysierenden Daten durch Ausführung eines Computersteuerungsprogramms,

    wobei das Computersteuerungsprogramm ein

    Klassifikationsanalyseprogramm enthält, das die Klassifikationsanalyse unter Verwendung eines maschinellen Lernens durchführt,

    ein Eigenschaftswert im Voraus erhalten wird, der eine Eigenschaft der Wellenform des Impulssignals anzeigt,

    der im Voraus erhaltene Eigenschaftswert als Lerndaten für das maschinelle Lernen festgelegt wird,

    der Eigenschaftswert, der vom Impulssignal von den zu analysierenden Daten erhalten wird, entfernt wird

    die fehlerhaften Daten als Variable festgelegt werden und

    die Klassifikationsanalyse des Analyten durch Ausführung des Klassifikationsanalyseprogramms durchgeführt wird,

**dadurch gekennzeichnet,**

**dass** der Eigenschaftswert aus einem oder mehreren Werten besteht, der bzw. die aus einer Gruppe einer Höhe der Wellenform in einer vorbestimmten Zeitbreite ausgewählt wird bzw. werden,

eine Pulswellenlänge $t_a$,

ein Spitzenpositionsverhältnis, dargestellt durch das Verhältnis $t_b/t_a$ der Zeit $t_a$ und $t_b$, die von dem Impulsstart zu der Impulsspitze führt,

eine Kurtosis, die die Schärfe der Welle darstellt,

eine Vertiefung, die die Steigung, die von dem Impulsstart zu der Impulsspitze führt, darstellt,

einen Bereich, der die Gesamtsumme des Zeitteilungsbereichs darstellt, der die Wellenform mit den vorgegebenen Zeiten teilt,

ein Flächenverhältnis der Summe der Zeitteilungsfläche, die von dem Impulsstart zu der Impulsspitze führt, zur gesamten Wellenformfläche,

ein Zeit-Trägheitsmoment, das durch die Masse und den Rotationsradius bestimmt wird, wenn die Masse konstruktiv zu der Zeitteilungsfläche ist, die auf der Impulsstartzeit zentriert ist, und der Rotationsradius konstruktiv zu der Zeit ist, die von der Mitte zu der Zeitteilungsfläche führt, ein normalisiertes Zeit- Trägheitsmoment, das bestimmt wird, wenn das Zeit-Trägheitsmoment so normalisiert ist, dass die Wellenhöhe ein Referenzwert wird,

ein Mittelwertvektor, dessen Vektorkomponente der Mittelwert der gleichen Wellenhöhenposition ist, in der die Wellenform in der Wellenhöhenrichtung in gleiche Teile geteilt ist, und der Mittelwert der Zeitwerte für jede Teilungseinheit vor und nach jeder Impulsspitze berechnet wir

ein normalisierter Mittelwertvektor, der so normalisiert ist, dass die Wellenlänge zu einem Standardwert für den Mittelwertvektor wird,

ein Wellenbreiten-Mittelwert-Trägheitsmoment, das durch die Massenverteilung und das Rotationszentrum bestimmt wird, wenn die Massenverteilung konstruktiv zum Mittelwert-Differenzvektor ist,

dessen Vektorkomponente die Mittelwertdifferenz der gleichen Wellenhöhenposition ist, in der die Wellenform in der Wellenhöhenrichtung in gleiche Teile geteilt ist und der Mittelwert der Zeitwerte für jede Teilungseinheit vor und nach jeder Impulsspitze berechnet wird und der Drehpunkt konstruktiv auf die Zeitachse des Wellenformfußes ist,

ein normalisiertes Wellenbreiten-Mittelwert-Trägheitsmoment, das bestimmt wird, wenn das Wellenbreiten-Mittelwert-Trägheitsmoment so normalisiert ist, dass die Wellenlänge zu einem Standardwert wird, ein Wellenbreite-Dispersion-Trägheitsmoment, das durch die Massenverteilung und das Rotationszentrum bestimmt wird, wenn die Massenverteilung konstruktiv zum Dispersionsvektor ist, dessen Vektorkomponente die Dispersion ist, bei der die Wellenform in der Wellenhöhenrichtung in gleiche Teile geteilt ist und die Dispersion aus dem Zeitwert für jede Teilungseinheit berechnet wird und das Rotationszentrum konstruktiv zur Zeitachse des Wellenformfußes ist, und

ein normalisiertes Wellenbreite-Dispersion-Trägheitsmoment, das bestimmt wird, wenn das Wellenbreite-Dispersion-Trägheitsmoment so normalisiert ist, dass die Wellenlänge ein Standardwert wird.

2. Eine Vorrichtung zur Identifikation-Klassifikationsanalyse umfassend

ein Mittel zum Einführen einer Probe, die einen Analyten enthält, in einen Messraum,

ein Mittel zum Erhalten von Impulssignaldaten, die aufgrund der Einführung erfasst werden,

ein Mittel zur Identifikation von fehlerhaften Daten, die aufgrund von anderen Elementen als dem Analyten von den Impulssignaldaten durch Ausführung eines Identifikationsanalyseprogramms, das in einem Computersteuerungsprogramm enthalten ist, erkannt werden,

ein Mittel zum Erhalten von zu analysierenden Daten durch Entfernen der fehlerhaften Daten aus den Impulssignaldaten und

ein Mittel zur Durchführung einer Klassifikationsanalyse der zu analysierenden Daten durch Ausführung des Computersteuerungsprogramms,

wobei

eine Speichereinrichtung (5) für fehlerhafte Daten zum Speichern der fehlerhaften Daten enthalten ist,

das Computersteuerungsprogramm ein Klassifikationsanalyseprogramm enthält, das die Klassifikationsanalyse unter Verwendung eines maschinellen Lernens durchführt,

ein Eigenschaftswert im Voraus erhalten wird, der eine Eigenschaft der Wellenform des Impulssignals angibt,

der im Voraus erhaltene Eigenschaftswert als Lerndaten für das maschinelle Lernen festgelegt wird,

der Eigenschaftswert, der vom Impulssignal von den zu analysierenden Daten erhalten wird, entfernt wird

die fehlerhaften Daten als Variable festgelegt werden und

die Klassifikationsanalyse des Analyten durch Ausführung des Klassifikationsanalyseprogramms durchgeführt wird,

**dadurch gekennzeichnet,**

**dass** der Eigenschaftswert aus einem oder mehreren Werten besteht, der bzw. die aus einer Gruppe einer Höhe der Wellenform in einer vorbestimmten Zeitbreite ausgewählt wird bzw. werden,

eine Impulswellenlänge $t_a$.

ein Spitzenpositionsverhältnis, dargestellt durch das Verhältnis tb/ta der Zeit ta und tb, die von dem Impulsstart zu der Impulsspitze führt,

eine Kurtosis, die die Schärfe der Welle darstellt,

eine Vertiefung, die die Steigung, die von dem Impulsstart zu der Impulsspitze führt, darstellt,

einen Bereich, der die Gesamtsumme des Zeitteilungsbereichs darstellt, der die Wellenform mit den vorgegebenen Zeiten teilt,

ein Flächenverhältnis der Summe der Zeitteilungsfläche, die von dem Impulsstart zu der Impulsspitze führt, zur gesamten Wellenformfläche,

ein Zeit-Trägheitsmoment, das durch die Masse und den Rotationsradius bestimmt wird, wenn die Masse konstruktiv zu der Zeitteilungsfläche ist, die auf der Impulsstartzeit zentriert ist und der Rotationsradius konstruktiv zu der Zeit ist, die vom Zentrum zu der Zeitteilungsfläche führt,

ein normalisiertes Zeit-Trägheitsmoment, das bestimmt wird, wenn das Zeit- Trägheitsmoment so normalisiert ist, dass die Wellenhöhe

zu einem Referenzwert wird, einen Mittelwertvektor, dessen Vektorkomponente der Mittelwert der gleichen Wellenhöhenposition ist, in der die Wellenform in der Wellenhöhenrichtung in gleiche Teile geteilt ist und der Mittelwert der Zeitwerte für jede Teilungseinheit vor und nach jeder Impulsspitze berechnet wird,

ein normalisierter Mittelwertvektor, der so normalisiert ist, dass die Wellenlänge zu einem Standardwert für den Mittelwertvektor wird, ein Wellenbreiten-Mittelwert-Trägheitsmoment, das durch die Massenverteilung und das Rotationszentrum bestimmt wird, wenn die Massenverteilung konstruktiv zum Mittelwert-Differenzvektor ist, dessen Vektorkomponente die Mittelwertdifferenz der gleichen Wellenhöhenposition ist, in der die Wellenform in der Wellenhöhenrichtung in gleiche Teile geteilt ist und der Mittelwert der Zeitwerte für jede Teilungseinheit vor und nach jeder Impulsspitze berechnet wird und der Drehpunkt konstruktiv auf die Zeitachse des Wellenformfußes ist,

ein normalisiertes Wellenbreiten-Mittelwert-Trägheitsmoment, das bestimmt wird, wenn das Wellenbreiten-Mittelwert-Trägheitsmoment so normalisiert ist, dass die Wellenlänge zu einem Standardwert wird, ein Wellenbreite-Dispersion-Trägheitsmoment, das durch die Massenverteilung und das Rotationszentrum bestimmt wird, wenn die Massenverteilung konstruktiv zum Dispersionsvektor ist, dessen Vektorkomponente die Dispersion ist, bei der die Wellenform in der Wellenhöhenrichtung in gleiche Teile geteilt ist und die Dispersion aus dem Zeitwert für jede Teilungseinheit berechnet wird und das Rotationszentrum konstruktiv zur Zeitachse des Wellenformfußes ist und

ein normalisiertes Wellenbreite-Dispersion-Trägheitsmoment, das bestimmt wird, wenn das Wellenbreite-Dispersion-Trägheitsmoment so normalisiert ist, dass die Wellenlänge ein Standardwert wird.

**Revendications**

1. Procédé d'analyse de classification d'identification comprenant les étapes suivantes :

   introduction d'un échantillon contenant un analyte dans un espace de mesure,

   obtention de données de signal d'impulsion détectées en raison de ladite introduction,

   identification des données non conformes détectées en raison d'éléments autres que ledit analyte à partir desdites données de signal d'impulsion par l'exécution d'un programme d'analyse d'identification inclus dans un programme de commande informatique,

   obtention de données à analyser en supprimant lesdites données non conformes desdites données de signal d'impulsion, et

   réalisation d'une analyse de classification desdites données à analyser par l'exécution d'un programme de commande informatique,

   ledit programme de commande informatique comprenant un programme d'analyse de classification qui réalise ladite analyse de classification en utilisant un apprentissage automatique,

   une valeur de caractéristique étant obtenue à l'avance qui indique une caractéristique de forme d'onde dudit signal d'impulsion,

   ladite valeur de caractéristique obtenue à l'avance étant définie comme les données d'apprentissage pour ledit apprentissage automatique,

ladite valeur de caractéristique obtenue à partir dudit signal d'impulsion desdites données à analyser ayant supprimé lesdites données non conformes étant définie comme une variable, et

ladite analyse de classification sur ledit analyte étant réalisée en exécutant ledit programme d'analyse de classification,

**caractérisé en ce que** ladite valeur de caractéristique est une ou plusieurs valeurs sélectionnées dans un groupe constitué de :

une valeur de hauteur d'onde de la forme d'onde dans une largeur de temps prédéterminée,

une longueur d'onde d'impulsion $t_a$,

un rapport de position de pic représenté par le rapport $t_b/t_a$ des temps $t_a$ et $t_b$ menant du début de l'impulsion au pic de l'impulsion,

un aplatissement qui représente la netteté de la forme d'onde,

une dépression représentant la pente menant du début de l'impulsion au pic de l'impulsion,

une surface représentant la somme totale de la zone de division temporelle divisant la forme d'onde avec les temps prédéterminés,

un rapport de surface de la somme de la zone de division temporelle menant du début de l'impulsion au pic de l'impulsion sur la surface totale de la forme d'onde,

un moment d'inertie temporel déterminé par la masse et le rayon de rotation lorsque la masse est constructive par rapport à ladite zone de division temporelle centrée à l'instant de début d'impulsion et que le rayon de rotation est constructif par rapport au temps menant dudit centre à ladite zone de division temporelle,

un moment d'inertie temporel normalisé déterminé lorsque ledit moment d'inertie temporel est normalisé de sorte que la hauteur d'onde devient une valeur de référence,

un vecteur de valeur moyenne dont la composante vectorielle est la valeur moyenne de la même position de hauteur d'onde dans laquelle la forme d'onde est divisée de manière égale dans la direction de la hauteur d'onde et la valeur moyenne des valeurs de temps est calculée pour chaque unité de division avant et après chaque pic d'impulsion,

un vecteur de valeur moyenne normalisé qui est normalisé de telle sorte que la longueur d'onde devient une valeur standard pour ledit vecteur de valeur moyenne,

un moment d'inertie de valeur moyenne de largeur d'onde déterminé par la distribution de masse et le centre de rotation lorsque la distribution de masse est constructive par rapport au vecteur de différence de valeur moyenne dont la composante vectorielle est la différence de valeur moyenne de la même position de hauteur d'onde dans laquelle la forme d'onde est divisée de manière égale dans la direction de la hauteur d'onde et la valeur moyenne des valeurs de temps est calculée pour chaque unité de division avant et après chaque pic d'impulsion, et le centre de rotation est constructif par rapport à l'axe de temps du pied de forme d'onde,

un moment d'inertie de valeur moyenne de largeur d'onde normalisée déterminé lorsque ledit moment d'inertie de valeur moyenne de largeur d'onde est normalisé de sorte que la longueur d'onde devienne une valeur standard,

un moment d'inertie de dispersion de largeur d'onde déterminé par la distribution de masse et le centre de rotation lorsque la distribution de masse est constructive par rapport au vecteur de dispersion dont la composante vectorielle est la dispersion dans laquelle la forme d'onde est divisée de manière égale dans la direction de la hauteur d'onde et la dispersion est calculée à partir de la valeur de temps pour chaque unité de division, et le centre de rotation est constructif par rapport à l'axe de temps du pied de forme d'onde, et

un moment d'inertie de dispersion de largeur d'onde normalisé déterminé lorsque ledit moment d'inertie de dispersion de largeur d'onde est normalisé de sorte que la longueur d'onde devienne une valeur standard.

2. Dispositif d'analyse de classification d'identification comprenant :

un moyen pour introduire un échantillon contenant un analyte dans un espace de mesure,

un moyen pour obtenir des données de signal d'impulsion détectées en raison de ladite introduction,

un moyen pour identifier des données non conformes détectées en raison d'éléments autres que ledit analyte à partir desdites données de signal d'impulsion par l'exécution d'un programme d'analyse d'identification inclus dans un programme de commande informatique,

un moyen pour obtenir des données à analyser en supprimant lesdites données non conformes desdites données de signal d'impulsion, et

un moyen pour réaliser une analyse de classification desdites données à analyser par l'exécution dudit programme de commande informatique,

un moyen de stockage de données non conformes (5) étant inclus pour stocker lesdites données non conformes, ledit programme de commande informatique comprenant un programme d'analyse de classification qui réalise ladite analyse de classification en utilisant un apprentissage automatique,

une valeur de caractéristique étant obtenue à l'avance qui indique une caractéristique de forme d'onde dudit signal d'impulsion,

ladite valeur de caractéristique obtenue à l'avance étant définie comme les données d'apprentissage pour ledit apprentissage automatique,

ladite valeur de caractéristique obtenue à partir dudit signal d'impulsion desdites données à analyser ayant supprimé lesdites données non conformes étant définie comme une variable, et

ladite analyse de classification sur ledit analyte étant réalisée en exécutant ledit programme d'analyse de classification,

**caractérisé en ce que** ladite valeur de caractéristique est une ou plusieurs valeurs sélectionnées dans un groupe constitué de :

une valeur de hauteur d'onde de la forme d'onde dans une largeur de temps prédéterminée,

une longueur d'onde d'impulsion $t_a$,

un rapport de position de pic représenté par le rapport $t_b/t_a$ des temps $t_a$ et $t_b$ menant du début de l'impulsion au pic de l'impulsion,

un aplatissement qui représente la netteté de la forme d'onde,

une dépression représentant la pente menant du début de l'impulsion au pic de l'impulsion,

une surface représentant la somme totale de la zone de division temporelle divisant la forme d'onde avec les temps prédéterminés,

un rapport de surface de la somme de la zone de division temporelle menant du début de l'impulsion au pic de l'impulsion sur la surface totale de la forme d'onde,

un moment d'inertie temporel déterminé par la masse et le rayon de rotation lorsque la masse est constructive par rapport à ladite zone de division temporelle centrée à l'instant de début d'impulsion et que le rayon de rotation est constructif par rapport au temps menant dudit centre à ladite zone de division temporelle,

un moment d'inertie temporel normalisé déterminé lorsque ledit moment d'inertie temporel est normalisé de sorte que la hauteur d'onde devient une valeur de référence,

un vecteur de valeur moyenne dont la composante vectorielle est la valeur moyenne de la même position de hauteur d'onde dans laquelle la forme d'onde est divisée de manière égale dans la direction de la hauteur d'onde et la valeur moyenne des valeurs de temps est calculée pour chaque unité de division avant et après chaque pic d'impulsion,

un vecteur de valeur moyenne normalisé qui est normalisé de telle sorte que la longueur d'onde devient une valeur standard pour ledit vecteur de valeur moyenne,

un moment d'inertie de valeur moyenne de largeur d'onde déterminé par la distribution de masse et le centre de rotation lorsque la distribution de masse est constructive par rapport au vecteur de différence de valeur moyenne dont la composante vectorielle est la différence de valeur moyenne de la même position de hauteur d'onde dans laquelle la forme d'onde est divisée de manière égale dans la direction de la hauteur d'onde et la valeur moyenne des valeurs de temps est calculée pour chaque unité de division avant et après chaque pic d'impulsion, et le centre de rotation est constructif par rapport à l'axe de temps du pied de forme d'onde,

un moment d'inertie de valeur moyenne de largeur d'onde normalisée déterminé lorsque ledit moment d'inertie de valeur moyenne de largeur d'onde est normalisé de sorte que la longueur d'onde devienne une valeur standard,

un moment d'inertie de dispersion de largeur d'onde déterminé par la distribution de masse et le centre de rotation lorsque la distribution de masse est constructive par rapport au vecteur de dispersion dont la composante vectorielle est la dispersion dans laquelle la forme d'onde est divisée de manière égale dans la direction de la hauteur d'onde et la dispersion est calculée à partir de la valeur de temps pour chaque unité de division, et le centre de rotation est constructif par rapport à l'axe de temps du pied de forme d'onde, et

un moment d'inertie de dispersion de largeur d'onde normalisé déterminé lorsque ledit moment d'inertie de dispersion de largeur d'onde est normalisé de sorte que la longueur d'onde devienne une valeur standard.

（1A）

（1B）

# Fig. 3

# Fig. 4

EP 3 623 793 B1

# Fig. 5

End

## Fig. 6

| | |
|---|---|
| functions.SMO | rules.ZeroR |
| bayes.DMNBtext | trees.ADTree |
| bayes.NaiveBayes | trees.BFTree |
| bayes.NaiveBayesUpdateable | trees.DecisionStump |
| functions.SimpleLogistic | trees.FT |
| functions.SPegasos | trees.J48 |
| functions.VotedPerceptron | trees.J48graft |
| lazy.IBk | trees.LADTree |
| lazy.LWL | trees.RandomForest |
| rules.JRip | trees.RandomTree |
| rules.PART | trees.REPTree |

EP 3 623 793 B1

# Fig. 7

Perform the $d_h$ equal division of the wavelength

Measured electric current value

Do normalization / do not

Set each average value as components

Measurement time

EP 3 623 793 B1

# Fig. 8

# Fig. 9

## (9A)

When there are given the input x,

the class label (positive example/negative example) y and

the flag s showing whether the data are labeled or not,

learning $p(y|x)$ from $\{\langle x, s \rangle\}$

## (9B)

- $x$ : an example
- $y \in \{0,1\}$ : a binary label (1 is the positive, 0 is the negative)
- $s = 1 \rightarrow x$ is labeled
  $s = 0 \rightarrow x$ is unlabeled
- The positive example only is labeled.

  The labeled sample is always the positive example.

  The unlabeled sample is unknown for the positive or the negative.

  When the sample is negative example, the probability labeled is zero.

$$p(s = 1|x, y = 0) = 0$$

# Fig. 10

## (10a)

$$g(x) = p(s = 1|x)$$
$$= p(y = 1 \wedge s = 1|x)$$
$$= p(y = 1|x)\,p(s = 1|y = 1, x)$$
$$= p(y = 1|x)\,p(s = 1|y = 1)$$

## (10b)

$$w(x) = p(y = 1|x, s = 0)$$
$$= \frac{p(s = 0|x, y = 1)\,p(y = 1|x)}{p(s = 0|x)}$$
$$= \frac{[1 - p(s = 1|x, y = 1)]p(y = 1|x)}{1 - p(s = 1|x)}$$
$$= \frac{(1 - c)p(y = 1|x)}{1 - p(s = 1|x)}$$
$$= \frac{(1 - c)p(s = 1|x)/c}{1 - p(s = 1|x)}$$
$$= \frac{1 - c}{c}\frac{p(s = 1|x)}{1 - p(s = 1|x)}$$
$$= \frac{1 - c}{c}\frac{g(x)}{1 - g(x)}$$

# Fig. 11

| | |
|---|---|
| **P** : Positive example (y=1) | **N** : Negative example (y=0) |
| **L** : Labeled (s=1) | **U** : Unlabeled (s=0) |

- The data for learning consist of two subsets 「labeled (s=1)」 and 「unlabeled (s=0)」.

- When these two subsets are selected as the inputs of the normal learning algorithm, the output becomes the probability g(x) that the sample is labeled: $g(x) = p(s = 1|x)$.

Training data

$$g(x) = p(s = 1|x)$$

If $x \in P$,

$$g(x) = p(s = 1|x \in P)$$
$$= p(s = 1|x, y = 1)$$
$$= c$$

EP 3 623 793 B1

# Fig. 12

PU classifier process

P1-1 — $g(x) = p(s = 1|x)$
is learned by the training set.

P1-2 — $c = \frac{1}{n}\Sigma_{x \in P}\, g(x)$
is estimated by the validation set.
P is the labeled data in the validation
set V.

P1-3 — The positive example / negative
example of the test data are identified.
If $p(y = 1|x) = \frac{1}{c}p(s = 1|x)$

$= \frac{g(x)}{c} > 0.5$,

it is judged as the positive example.

終了

Fig. 13

(13B)

(13A)

EP 3 623 793 B1

## Fig. 14

Fig. 15

# Fig. 16

Escherichia coli pulse Extraction data

Bacillus subtilis pulse Extraction data

Characteristic amount extraction program

Escherichia coli pulse Characteristic amount data

Bacillus subtilis pulse Characteristic amount data

Data file generation program

Bacillus subtilis data for particle type distribution estimation

Bacillus subtilis data for probability density function estimation

Escherichia coli data for particle type distribution estimation

Escherichia coli data for probability density function estimation

Probability density function module

Data mixture program

Data for accuracy verification

Particle type distribution estimation program

Particle type distribution estimation results

EP 3 623 793 B1

# Fig. 17

# Continuation 1 of Fig. 17

# Continuation 2 of Fig. 17

# Fig. 18

# Fig. 19

EP 3 623 793 B1

(6A)

$$x_t = Fx_{t-1} + Gu_{t-1}$$

$$u_t \sim N(0, \sigma_{u,t}^2)$$

$x_t$ : State vector at time t (Estimation target)    $F$ : State transition matrix

$u_t$ : System control input at time t    $G$ : System control input matrix

$\sim N(0, \sigma_{u,t}^2)$ : Follows the normal distribution with average 0 and dispersion $\sigma_{u,t}^2$

(6B)

$$y_t = Hx_t + v_t$$

$$v_t \sim N(0, \sigma_{v,t}^2)$$

$y_t$ : Observation vector at time t    $H$ : Observation matrix

$v_t$ : Observation noise at time t

$\sim N(0, \sigma_{v,t}^2)$ : follows the normal distribution with average 0 and dispersion $\sigma_{v,t}^2$

(6C)

$$x_t = x_{t-1} + u_{t-1}$$

$$y_t = x_t + v_t$$

$x_t$ : Base line level of the real electric current at time t

$y_t$ : Electric current measured at time t

$u_t \sim N(0, \sigma_u^2)$ : System control input

$v_t \sim N(0, \sigma_{v,t}^2)$ : Observation noise at time t

# Fig. 20

Base line $x_t$

IonicO. 1V-PstO. 50 $\mu$ m-2000Dilution-21

x10^-8

1.36509

Observation noise $v_t$

$y_t$ Electric current measured at time t

Sampling Rate: 1000000 Hz

0

1.32414

51053232

EP 3 623 793 B1

# Fig. 21

- Prediction of estimation value at time t based upon amended estimation value at time t-1

**(8A)Prediction**

$$\hat{x}_{t|t-1} = \hat{x}_{t-1|t-1}$$

- Prediction of covariance of post-estimation error

$$P_{t|t-1} = FP_{t-1|t-1}F^T + G\sigma_u^2 G^T = P_{t-1|t-1} + \sigma_u^2$$

※ Post-estimation error
$$= x_t - \hat{x}_t$$
Difference between true value and estimation of state vector

**(8B)Renewal**

**A**

- Calculation of variance in past m steps of estimation value for actual value of measurement value

$$e_t = y_t - H\hat{x}_{t|t-1} = y_t - \hat{x}_{t|t-1}$$

$$\sigma_{v,t}^2 = \frac{1}{m}\sum_{k=t-m+1}^{t} e_k^2 = \sigma_{v,t-1}^2 + \frac{1}{m}\left\{e_t^2 - e_{t-m}^2\right\}$$

- Calculation of Kalman gain at time t

$$K_t = P_{t|t-1}H^T\left(HP_{t|t-1}H^T + \sigma_{v,t}^2\right)^{-1} = \frac{P_{t|t-1}}{P_{t|t-1} + \sigma_{v,t}^2}$$

※ Kalman gain
Derived as matrix to minimize post-estimation error

**B**

- Renewal of estimation value at time t based upon measurement value at the same time

$$\hat{x}_{t|t} = \hat{x}_{t|t-1} + K_t e_t$$

- Renewal of covariance of post-estimation error

$$P_{t|t} = (I - K_t H)P_{t|t-1} = (1 - K_t)P_{t|t-1}$$

# Fig. 22

BL estimation process

S51 — Setting of initial value of Kalman filter at time m

S52 — Prediction and renewal of Kalman filter at time m+1

S53 — Prediction and renewal A of Kalman filter at time t

S54 — $|e_t| > \alpha \cdot \sigma_{v,t}$ ? $e_t = y_t - \hat{x}_{t|t-1}$

S56 — $v \leftarrow v+1$

S55 — Renewal B of Kalman filter at time t

S57 — $|e_t| > \alpha \cdot \sigma_{v,t}$ Following continuously by k times from starting point of time s?

S58 — Judgement that required period of hold started (Start time of hold is set as s and results of Kalman filter of time s+1~s+k-1 are abandoned)

S59 — Drop maximum value of pulse at time t is renewed

S60 — $|y_t - \hat{x}_{t|t-1}| \leq \alpha \cdot \sigma_{v,t}$ ?

S61 — $z \leftarrow z+1$

S62 — $|y_t - \hat{x}_{t|t-1}| \leq \alpha \cdot \sigma_{v,t}$ ? Following continuously by k times from starting point of time s2?

S63 — Drop maximum value is set as estimation value of pulse wave height

S64 — Judgement that required period of hold has ended (End time of hold is set as s2)

S65 — Value of time s is set as initial value of resumption time of Kalman filter, and Kalman filter is retroactively carried out for period of time s2~s2+k-1

# Fig. 23

(10A)

1. PstBeads(0.9μm+0.78μm), SinPore(1.2μm), 27,07,2012　　$\times 10^{-8}$

0.89424

0.77871

0　　　　Sampling Rate: 1000000 Hz　　　　46952160

10C

(10B)

1. PstBeads(0.9μm+0.78μm), SinPore(1.2μm), 27,07,2012　　$\times 10^{-8}$
(42889633-43898362)

0.86179

0.77871

0　　$m=100,000$　　Sampling Rate: 1000000 Hz　　1008729

EP 3 623 793 B1

# Fig. 24

# Fig. 25

(12A)

m=10000

| $k \setminus \alpha$ | 2 | 3 | 4 | 6 |
|---|---|---|---|---|
| 10 | 1,578 | 79 | 18 | 13 |
| 30 | 186 | 19 | 12 | 12 |
| 50 | 41 | 14 | 12 | 12 |
| 70 | 21 | 12 | 12 | 12 |
| 90 | 15 | 12 | 12 | 12 |

m=50000 (12B)

| $k \setminus \alpha$ | 2 | 3 | 4 | 6 |
|---|---|---|---|---|
| 10 | 1,519 | 89 | 32 | 13 |
| 30 | 237 | 24 | 15 | 12 |
| 50 | 74 | 14 | 12 | 12 |
| 70 | 35 | 12 | 12 | 12 |
| 90 | 24 | 12 | 12 | 12 |

m=100000 (12C)

| $k \setminus \alpha$ | 2 | 3 | 4 | 6 |
|---|---|---|---|---|
| 10 | 1,402 | 104 | 37 | 14 |
| 30 | 239 | 31 | 12 | 11 |
| 50 | 92 | 17 | 11 | 11 |
| 70 | 52 | 11 | 11 | 11 |
| 90 | 26 | 11 | 11 | 11 |

# Fig. 26

Characteristic amount
extraction process

S41 — Characteristic
amount extraction
is possible? —— NO

YES

S42 — Creation of various
vectors

S43 — Completing
creation of all
vector? —— NO

YES

S44 — Storage of vector data

S45 — Extraction of
characteristic amount

End

# Fig. 27

Particle type distribution estimating process

S1 — Creation of data file for estimation

S2 — Probability density function estimating process

S3 — Particle number estimating process

S4 — Calculation of estimated particle type distribution (Creation process of histogram)

End

# Fig. 28

(15A)

Base line

Wave height h

1. PstBeads (0.9 μm+0.78 μm), SiNPore (1.2 μm)
27.07.2012

x10^-8

0.89424

0.77871

26389623    Sampling Rate: 1000000 Hz    26392268

Pulse start time  $t_s$

Pulse end time  $t_e$

Pulse width  $\Delta t = t_e - t_s$

(15B)

Wave height h

Escherichia coli

Bacillus subtilis

Pulse width  $\Delta t$

Fig. 29

(16A)

(16B)

(16C)

EP 3 623 793 B1

# Fig. 30

Expectation value of appearance frequency (= Particle total number of particle type × (Particle type)Appearance probability)

$m(x)$

Appearance frequency →

Pulse $x$ ⟶

(17A)

Probability density function

$$m_1(x) = n_1 p_1(x)$$

Appearance frequency

Pulse $x$ ⟶

(17−1)

Probability density function

$$m_k(x) = n_k p_k(x)$$

Appearance frequency

Pulse $x$ ⟶

(17−k)

$=$  $+$ ⋯ $+$

EP 3 623 793 B1

# Fig. 31

<Derivation process of equation 17>

Constrained logarithmic likelihood maximization formula is differentiated and put zero.

$$\frac{\partial}{\partial n_i}\left[\sum_{x_j \in D} \log \sum_{i=1}^{k} n_i\, p_i(x_j) - \lambda\left(\sum_{i=1}^{k} n_i - N\right)\right] = 0$$

Then the following is obtained.

$$\sum_{x_j \in D} \frac{p_i(x_j)}{\sum_{l=1}^{k} n_l\, p_l(x_j)} = \lambda$$

Here undetermined multiplier $\lambda$ becomes a problem, but it is found that $\lambda = 1$.

$$\lambda = \frac{1}{N}\sum_{i=1}^{k} n_i \lambda = \frac{1}{N}\sum_{x_j \in D}\sum_{i=1}^{k} \frac{n_i p_i(x_j)}{\sum_{l=1}^{k} n_l\, p_l(x_j)} = 1$$

# Fig. 32

```
        ╭─────────────────╮
        │ Data file creation│
        │     process     │
        ╰─────────────────╯
                │
                ▼
  S30    ┌──────────────────┐
         │ Setting process of│
         │characteristic amount│
         └──────────────────┘
                │
                ▼
  S31    ┌──────────────────┐
         │   Data read of   │
         │  characteristic  │
         │ amount data file │
         └──────────────────┘
                │
                ▼
  S32    ┌──────────────────┐
         │ Creation of matrix,│
         │data with N rows and│
         │    k columns     │
         └──────────────────┘
                │
                ▼
  S33    ┌──────────────────┐
         │ Output to data file│
         │distribution estimation│
         │  for particle type │
         └──────────────────┘
                │
                ▼
  S34        ◇ Are there data to   ── YES ──┐
             be created?                     │
                │ NO                         │
                ▼                            │
        ╭─────────────────╮                 │
        │       End       │                 │
        ╰─────────────────╯                 │
```

76

# Fig. 33

Probability density function estimation process

S20 — Data read of file for probability density function estimation

S21 — Creation of matrix D with N rows and 2 columns

S22 — Calculation of variance $\sigma^2_{D\beta}$, $\sigma^2_{D\gamma}$ of each column of matrix D

S23 — Setting of variance parameters $\sigma^2_\beta$, $\sigma^2_\gamma$

S24 — Probability density function is obtained by substituting variance parameter and each column of matrix D as teacher data $\mu^1_\beta$, $\mu^1_\gamma$

S25 — Storage of probability density function

S26 — Are there data file to be processed? — Y

N

End

# Fig. 34

```
        ┌─────────────────────┐
        │   Particle number   │
        │ estimation process  │
        └─────────────────────┘
                   │
                   ▼
        ┌─────────────────────┐
S10 ────│ Data read of file for│
        │ accuracy verification│
        └─────────────────────┘
                   │
                   ▼
        ┌─────────────────────┐
        │ Creation of matrix D │
S11 ────│   with N rows and 2  │
        │       columns        │
        └─────────────────────┘
                   │
                   ▼
        ┌─────────────────────┐
        │  Particle number     │
S12 ────│ estimation process   │
        │ due to Hasselblad    │
        │ method               │
        └─────────────────────┘
                   │
                   ▼
        ┌─────────────────────┐
        │        End          │
        └─────────────────────┘
```

# Fig. 35

Particle number estimation
process due to
Hasselblad method

S12a — Setting of initial values

S12b — Iterative operation
of $N_i(t+1)$ for
convergence
condition $\alpha$

S12c — Iterative operation
converged?   N

Y

S12d — Storage of operation
result of estimated
particle number

Return

# Fig. 36

(23A) Setting of initial values

$$n_i^{(0)} = \frac{N}{k}$$

(23B) Iterative operation

$$n_i^{(1)} = n_i^{(0)} \sum_{x_j \in D} \frac{p_i(x_j)}{\sum_{l=1}^{k} n_l^{(0)} p_l(x_j)}$$

$$\Downarrow$$

$$n_i^{(2)} = n_i^{(1)} \sum_{x_j \in D} \frac{p_i(x_j)}{\sum_{l=1}^{k} n_l^{(1)} p_l(x_j)}$$

$$\Downarrow$$

$$\vdots$$

(23C) Convergence conditions

$$\max_i \left( \left| n_i^{(t)} - n_i^{(t-1)} \right| \right) \leqq \alpha$$

# Fig. 37

(24A)

(24B)

(24C)

(24D)

# Fig. 38

(25A)

| Data of particle type distribution estimation target | Total number of particle | Number of Escheriachia coli | | | | Number of Bacillus subtilis | | | | Weighted average Relative error | Averaged convergence step number | Averaged necessary time(s) |
| --- | --- | --- | --- | --- | --- | --- | --- | --- | --- | --- | --- | --- |
| | | Truth | Estimation (average) | Standard deviation | Relative error | Truth | Estimation (average) | Standard deviation | Relative error | | | |
| Data mixing 10% E coli data to wave height data of 146 pieces of Bacillus subtilis | 161 | 15 | 50.45 | 11.92 | 2.36 | 146 | 110.55 | 11.92 | 0.24 | 0.44 | 12.3 | 2.18 |
| Data mixing 20% E coli data to wave height data of 146 pieces of Bacillus subtilis | 175 | 29 | 58.48 | 12.13 | 1.02 | 146 | 116.52 | 12.13 | 0.20 | 0.34 | 12.3 | 2.37 |
| Data mixing 30% E coli data to wave height data of 146 pieces of Bacillus subtilis | 190 | 44 | 73.20 | 13.45 | 0.66 | 146 | 116.80 | 13.45 | 0.20 | 0.31 | 10.6 | 2.21 |
| Data mixing 35% E coli data to wave height data of 146 pieces of Bacillus subtilis | 197 | 51 | 76.93 | 13.89 | 0.51 | 146 | 120.07 | 13.89 | 0.18 | 0.26 | 10.7 | 2.35 |
| Data mixing 40% E coli data to wave height data of 146 pieces of Bacillus subtilis | 204 | 58 | 79.23 | 14.52 | 0.37 | 146 | 124.77 | 14.52 | 0.15 | 0.21 | 11.1 | 2.47 |
| Data mixing 45% E coli data to wave height data of 146 pieces of Bacillus subtilis | 212 | 66 | 82.60 | 15.06 | 0.25 | 146 | 129.40 | 15.06 | 0.11 | 0.16 | 11.5 | 2.69 |
| Data mixing 50% E coli data to wave height data of 146 pieces of Bacillus subtilis | 219 | 73 | 85.37 | 15.63 | 0.17 | 146 | 133.63 | 15.63 | 0.08 | 0.11 | 11.6 | 2.80 |

(25B)

| Data of particle type distribution estimation target | Total number of particle | Number of Escheriachia coli | | | | Number of Bacillus subtilis | | | | Weighted average Relative error | Averaged convergence step number | Averaged necessary time(s) |
| --- | --- | --- | --- | --- | --- | --- | --- | --- | --- | --- | --- | --- |
| | | Truth | Estimation (average) | Standard deviation | Relative error | Truth | Estimation (average) | Standard deviation | Relative error | | | |
| Data mixing 10% E coli data to wave height data of 146 pieces of Bacillus subtilis | 161 | 15 | 39.51 | 9.14 | 1.63 | 146 | 121.49 | 9.14 | 9.14 | 0.30 | 11.7 | 2.07 |
| Data mixing 20% E coli data to wave height data of 146 pieces of Bacillus subtilis | 175 | 29 | 46.00 | 9.59 | 0.59 | 146 | 129.00 | 9.59 | 9.59 | 0.19 | 11.7 | 2.25 |
| Data mixing 30% E coli data to wave height data of 146 pieces of Bacillus subtilis | 190 | 44 | 55.40 | 10.29 | 0.26 | 146 | 134.60 | 10.29 | 10.29 | 0.12 | 11.7 | 2.43 |
| Data mixing 35% E coli data to wave height data of 146 pieces of Bacillus subtilis | 197 | 51 | 58.38 | 10.53 | 0.14 | 146 | 138.62 | 10.53 | 10.53 | 0.07 | 11.7 | 2.53 |
| Data mixing 40% E coli data to wave height data of 146 pieces of Bacillus subtilis | 204 | 58 | 61.09 | 11.07 | 0.05 | 146 | 142.91 | 11.07 | 11.07 | 0.03 | 11.9 | 2.67 |
| Data mixing 45% E coli data to wave height data of 146 pieces of Bacillus subtilis | 212 | 66 | 63.18 | 11.35 | 0.04 | 146 | 148.82 | 11.35 | 11.35 | 0.03 | 12.1 | 2.83 |
| Data mixing 50% E coli data to wave height data of 146 pieces of Bacillus subtilis | 219 | 73 | 65.69 | 12.15 | 0.10 | 146 | 153.31 | 12.15 | 12.15 | 0.07 | 12.3 | 2.96 |

EP 3 623 793 B1

# Fig. 39

(26A)

| Data of particle type distribution estimation target | Total number of particle | Number of Escheriachia coli | | | | Number of Bacillus subtilis | | | | Weighted average Relative error | Averaged convergence step number | Averaged necessary time(s) |
|---|---|---|---|---|---|---|---|---|---|---|---|---|
| | | Truth | Estimation (average) | Standard deviation | Relative error | Truth | Estimation (average) | Standard deviation | Relative error | | | |
| Data mixing 10% E coli data to wave height data of 146 pieces of Bacillus subtilis | 161 | 15 | 24.60 | 9.27 | 0.64 | 146 | 136.40 | 9.27 | 0.07 | 0.12 | 13.3 | 2.38 |
| Data mixing 20% E coli data to wave height data of 146 pieces of Bacillus subtilis | 175 | 29 | 31.99 | 10.26 | 0.10 | 146 | 143.01 | 10.26 | 0.02 | 0.03 | 12.8 | 2.49 |
| Data mixing 30% E coli data to wave height data of 146 pieces of Bacillus subtilis | 190 | 44 | 40.34 | 11.18 | 0.08 | 146 | 149.66 | 11.18 | 0.03 | 0.04 | 12.6 | 2.65 |
| Data mixing 35% E coli data to wave height data of 146 pieces of Bacillus subtilis | 197 | 51 | 44.45 | 11.72 | 0.13 | 146 | 152.55 | 11.72 | 0.04 | 0.07 | 12.6 | 2.77 |
| Data mixing 40% E coli data to wave height data of 146 pieces of Bacillus subtilis | 204 | 58 | 47.87 | 12.44 | 0.17 | 146 | 156.13 | 12.44 | 0.07 | 0.10 | 12.8 | 2.88 |
| Data mixing 45% E coli data to wave height data of 146 pieces of Bacillus subtilis | 212 | 66 | 51.01 | 13.36 | 0.23 | 146 | 160.99 | 13.36 | 0.10 | 0.14 | 13.1 | 3.05 |
| Data mixing 50% E coli data to wave height data of 146 pieces of Bacillus subtilis | 219 | 73 | 54.81 | 13.85 | 0.25 | 146 | 164.19 | 13.85 | 0.12 | 0.17 | 13 | 3.15 |

(26B)

| Data of particle type distribution estimation target | Total number of particle | Number of Escheriachia coli | | | | Number of Bacillus subtilis | | | | Weighted average Relative error | Averaged convergence step number | Averaged necessary time(s) |
|---|---|---|---|---|---|---|---|---|---|---|---|---|
| | | Truth | Estimation (average) | Standard deviation | Relative error | Truth | Estimation (average) | Standard deviation | Relative error | | | |
| Data mixing 10% E coli data to wave height data of 146 pieces of Bacillus subtilis | 161 | 15 | 24.45 | 7.53 | 0.63 | 146 | 136.55 | 7.53 | 0.06 | 0.12 | 11.6 | 2.07 |
| Data mixing 20% E coli data to wave height data of 146 pieces of Bacillus subtilis | 175 | 29 | 34.62 | 8.25 | 0.19 | 146 | 140.38 | 8.25 | 0.04 | 0.06 | 11 | 0.13 |
| Data mixing 30% E coli data to wave height data of 146 pieces of Bacillus subtilis | 190 | 44 | 53.22 | 8.83 | 0.21 | 146 | 136.78 | 8.83 | 0.06 | 0.10 | 9.6 | 2.03 |
| Data mixing 35% E coli data to wave height data of 146 pieces of Bacillus subtilis | 197 | 51 | 58.13 | 9.50 | 0.14 | 146 | 138.87 | 9.50 | 0.05 | 0.07 | 9.6 | 2.13 |
| Data mixing 40% E coli data to wave height data of 146 pieces of Bacillus subtilis | 204 | 58 | 62.63 | 9.95 | 0.08 | 146 | 141.37 | 9.95 | 0.03 | 0.05 | 9.7 | 2.18 |
| Data mixing 45% E coli data to wave height data of 146 pieces of Bacillus subtilis | 212 | 66 | 65.61 | 10.64 | 0.01 | 146 | 146.39 | 10.64 | 0.00 | 0.00 | 9.9 | 2.32 |
| Data mixing 50% E coli data to wave height data of 146 pieces of Bacillus subtilis | 219 | 73 | 68.26 | 10.95 | 0.06 | 146 | 150.74 | 10.95 | 0.03 | 0.04 | 9.9 | 2.40 |

EP 3 623 793 B1

# Fig. 40

(27A)

| Data of particle type distribution estimation target | Total number of particle | Number of Escheriachia coli | | | | Number of Bacillus subtilis | | | | Weighted average Relative error | Averaged convergence step number | Averaged necessary time(s) |
|---|---|---|---|---|---|---|---|---|---|---|---|---|
| | | Truth | Estimation (average) | Standard deviation | Relative error | Truth | Estimation (average) | Standard deviation | Relative error | | | |
| Data mixing 10% E coli data to wave height data of 146 pieces of Bacillus subtilis | 161 | 15 | 24.45 | 7.53 | 0.63 | 146 | 136.55 | 7.53 | 0.06 | 0.12 | 11.6 | 2.07 |
| Data mixing 20% E coli data to wave height data of 146 pieces of Bacillus subtilis | 175 | 29 | 34.62 | 8.25 | 0.19 | 146 | 140.38 | 8.25 | 0.04 | 0.06 | 11.0 | 2.13 |
| Data mixing 30% E coli data to wave height data of 146 pieces of Bacillus subtilis | 190 | 44 | 53.22 | 8.83 | 0.21 | 146 | 136.78 | 8.83 | 0.06 | 0.10 | 9.6 | 2.03 |
| Data mixing 35% E coli data to wave height data of 146 pieces of Bacillus subtilis | 197 | 51 | 58.13 | 9.50 | 0.14 | 146 | 138.87 | 9.50 | 0.05 | 0.07 | 9.6 | 2.13 |
| Data mixing 40% E coli data to wave height data of 146 pieces of Bacillus subtilis | 204 | 58 | 62.63 | 9.95 | 0.08 | 146 | 141.37 | 9.95 | 0.03 | 0.05 | 9.7 | 2.18 |
| Data mixing 45% E coli data to wave height data of 146 pieces of Bacillus subtilis | 212 | 66 | 65.61 | 10.64 | 0.01 | 146 | 146.39 | 10.64 | 0.00 | 0.00 | 9.9 | 2.32 |
| Data mixing 50% E coli data to wave height data of 146 pieces of Bacillus subtilis | 219 | 73 | 68.26 | 10.95 | 0.06 | 146 | 150.74 | 10.95 | 0.03 | 0.04 | 9.9 | 2.40 |

**Error 4~12%**

(27B)

$$\text{Weighted average relative error} = \sum_{i=1}^{k}\left( \frac{n_i}{N} \cdot \frac{|n_i - \hat{n}_i|}{n_i} \right) = \sum_{i=1}^{k} \frac{|n_i - \hat{n}_i|}{N}$$

EP 3 623 793 B1

# Fig. 41

(28A)

| Data of particle type distribution estimation target | Total number of particle | Number of Escheriachia coli | | | | Number of Bacillus subtilis | | | | Weighted average Relative error | Averaged convergence step number | Averaged necessary time(s) |
|---|---|---|---|---|---|---|---|---|---|---|---|---|
| | | Truth | Estimation (average) | Standard deviation | Relative error | Truth | Estimation (average) | Standard deviation | Relative error | | | |
| Data mixing 10% E coli data to wave height data of 146 pieces of Bacillus subtilis | 161 | 15 | 50.45 | 11.92 | 2.36 | 146 | 110.55 | 11.92 | 0.24 | 0.44 | 12.3 | 2.18 |
| Data mixing 20% E coli data to wave height data of 146 pieces of Bacillus subtilis | 175 | 29 | 58.48 | 12.13 | 1.02 | 146 | 116.52 | 12.13 | 0.20 | 0.34 | 12.3 | 2.37 |
| Data mixing 30% E coli data to wave height data of 146 pieces of Bacillus subtilis | 190 | 44 | 73.20 | 13.45 | 0.66 | 146 | 116.80 | 13.45 | 0.20 | 0.31 | 10.6 | 2.21 |
| Data mixing 35% E coli data to wave height data of 146 pieces of Bacillus subtilis | 197 | 51 | 76.93 | 13.89 | 0.51 | 146 | 120.07 | 13.89 | 0.18 | 0.26 | 10.7 | 2.35 |
| Data mixing 40% E coli data to wave height data of 146 pieces of Bacillus subtilis | 204 | 58 | 79.23 | 14.52 | 0.37 | 146 | 124.77 | 14.52 | 0.15 | 0.21 | 11.1 | 2.47 |
| Data mixing 45% E coli data to wave height data of 146 pieces of Bacillus subtilis | 212 | 66 | 82.60 | 45.06 | 0.25 | 146 | 129.40 | 15.06 | 0.11 | 0.16 | 11.5 | 2.69 |
| Data mixing 50% E coli data to wave height data of 146 pieces of Bacillus subtilis | 219 | 73 | 85.37 | 15.63 | 0.17 | 146 | 133.63 | 15.63 | 0.08 | 0.11 | 11.6 | 2.80 |

(28B)

| Data of particle type distribution estimation target | Total number of particle | Number of Escheriachia coli | | | | Number of Bacillus subtilis | | | | Weighted average Relative error | Averaged convergence step number | Averaged necessary time(s) |
|---|---|---|---|---|---|---|---|---|---|---|---|---|
| | | Truth | Estimation (average) | Standard deviation | Relative error | Truth | Estimation (average) | Standard deviation | Relative error | | | |
| Data mixing 10% E coli data to wave height data of 146 pieces of Bacillus subtilis | 161 | 15 | 39.51 | 9.14 | 1.63 | 146 | 121.49 | 9.14 | 0.17 | 0.30 | 11.7 | 2.07 |
| Data mixing 20% E coli data to wave height data of 146 pieces of Bacillus subtilis | 175 | 29 | 46.00 | 9.59 | 0.59 | 146 | 129.00 | 9.59 | 0.12 | 0.19 | 11.7 | 2.25 |
| Data mixing 30% E coli data to wave height data of 146 pieces of Bacillus subtilis | 190 | 44 | 55.40 | 10.29 | 0.26 | 146 | 134.60 | 10.29 | 0.08 | 0.12 | 11.7 | 2.43 |
| Data mixing 35% E coli data to wave height data of 146 pieces of Bacillus subtilis | 197 | 51 | 58.38 | 10.53 | 0.14 | 146 | 138.62 | 10.53 | 0.05 | 0.07 | 11.7 | 2.53 |
| Data mixing 40% E coli data to wave height data of 146 pieces of Bacillus subtilis | 204 | 58 | 61.09 | 11.07 | 0.05 | 146 | 142.91 | 11.07 | 0.02 | 0.03 | 11.9 | 2.67 |
| Data mixing 45% E coli data to wave height data of 146 pieces of Bacillus subtilis | 212 | 66 | 63.18 | 11.35 | 0.04 | 146 | 148.82 | 11.35 | 0.02 | 0.03 | 12.1 | 2.83 |
| Data mixing 50% E coli data to wave height data of 146 pieces of Bacillus subtilis | 219 | 73 | 65.69 | 12.15 | 0.10 | 146 | 153.31 | 12.15 | 0.05 | 0.07 | 12.3 | 2.96 |

# Fig. 42

(29A)

Escherichia coli:Bacillus subtilis=1:10

True value
Estimated value

Escherichia coli   Bacillus subtilis

(29B)

Escherichia coli:Bacillus subtilis=2:10

True value
Estimated value

Escherichia coli   Bacillus subtilis

(29C)

Escherichia coli:Bacillus subtilis=3:10

True value
Estimated value

Escherichia coli   Bacillus subtilis

(29D)

Escherichia coli:Bacillus subtilis=35:100

True value
Estimated value

Escherichia coli   Bacillus subtilis

EP 3 623 793 B1

Fig. 43

# Fig. 44

(31A)

(31B)

① Pulse length Δt、Wave height |h|

② Pulse length Δt、Peak position ratio r

# Fig. 45

(32A)

③Spread of peak vicinity waveform k、Pulse length Δt

(32B)

⑤Spread of peak vicinity waveform k、Peak position ratio r

(32C)

④Spread of peak vicinity waveform k、Wave height |h|

EP 3 623 793 B1

# Fig. 46

# Fig. 47

（34A）

（34B）

# Fig. 48

(35A)

d equal division of wavelength

(35B)

A1  A2  A3  A4  A5  A6  A7  A8  A9  A10

Fig. 49

(36A)

If pulse step is sufficiently large, average value of each interval is set as component.

(36B)

If pulse step is below vector dimension, component is obtained by spline interpolation.

# Fig. 50

(37A)

(37B)

10 dimensions

Do normalization in direction
of wavelength ∕do not

# Fig. 51

(38A)

Bd

(38B)

$t_i$          $t_k$   $t_{i+1}$

(38C)

Du

UR

# Fig. 52

（39A）

（39B）

# Fig. 53

(40A)

(40B)

dh division

dw division

```
0 0 0 0 0 0 0 0 0 0 2
0 0 0 0 2 0 0 0 1 3
0 0 0 1 0 1 0 0 0 1 1
0 0 0 1 3 0 2 0 2 2 0
0 0 0 2 1 1 6 0 3 2 0
0 0 0 6 3 0 2 2 2 1 0
0 0 1 0 2 0 0 1 2 2 0
0 0 4 0 1 2 0 2 1 1 0
1 0 0 0 1 0 1 0 0 0
2 1 1 0 0 1 0 0 0 0
2 2 2 0 0 2 0 0 0 0 0
1 1 1 0 0 0 0 0 0 0 0
3 1 1 0 0 0 0 0 0 0 0
```

40a

40b

# Fig. 54

Extraction process of characteristic amount

S71 — Setting of pulse width Δt from pulse extraction data

S72 — Calculation·storage of peak position ratio

S73 — Calculation of dispersion σ2 Storage of spread amount κ of peak vicinity waveform

S74 — Calculation·storage of depression θ

S75 — Calculation·storage of area m

S76 — Calculation·storage of area ratio rm

S77 — Calculation·storage of time inertia moment

S78 — Calculation·storage of normalized time inertia moment

S79 — Calculation·storage of mean wave width inertia moment

S80 — Calculation·storage of normalized mean wave width inertia moment

S81 — Calculation·storage of wave width dispersion inertia moment

S82 — Calculation·storage of normalized wave width dispersion inertia moment

S83 — Extraction of characteristic amout is done from all data?

N

Y

S84 — Storage of data file of characteristic amount

S85 — There are files to be processed?

Y

N

End

(A)

98

# Fig. 55

## (42A)

| | Wave length Δt | Wave height \|h\| | Peak position ratio r | Kurtosis k | Area ratio r_m | Depression θ | Area m | Inertia I | Inertia I (normalization) | Inertia I_w | Inertia I_wv | Inertia I_w (normalization) | Inertia I_wv (normalization) |
|---|---|---|---|---|---|---|---|---|---|---|---|---|---|
| Wave length Δt | | 0.238 | 0.131 | 0.120 | 0.141 | 0.151 | 0.106 | 0.102 | 0.151 | 0.108 | 0.251 | 0.106 | 0.130 |
| | | (0.132) | (0.086) | (0.080) | (0.097) | (0.097) | (0.082) | (0.086) | (0.089) | (0.082) | (0.132) | (0.082) | (0.092) |
| Wave height \|h\| | | | 0.157 | 0.106 | 0.173 | 0.186 | 0.166 | 0.138 | 0.126 | 0.231 | 0.147 | 0.122 | 0.097 |
| | | | (0.100) | (0.095) | (0.108) | (0.109) | (0.110) | (0.118) | (0.085) | (0.154) | (0.098) | (0.097) | (0.072) |
| Peak position ratio r | | | | 0.110 | 0.134 | 0.272 | 0.121 | 0.100 | 0.113 | 0.129 | 0.154 | 0.116 | 0.156 |
| | | | | (0.077) | (0.104) | (0.210) | (0.081) | (0.068) | (0.071) | (0.086) | (0.095) | (0.075) | (0.089) |
| Kurtosis k | | | | | 0.164 | 0.118 | 0.095 | 0.106 | 0.131 | 0.164 | 0.194 | 0.141 | 0.156 |
| | | | | | (0.083) | (0.074) | (0.070) | (0.078) | (0.086) | (0.100) | (0.121) | (0.090) | (0.089) |
| Area ratio r_m | | | | | | 0.144 | 0.112 | 0.100 | 0.124 | 0.129 | 0.180 | 0.148 | 0.162 |
| | | | | | | (0.098) | (0.089) | (0.070) | (0.076) | (0.094) | (0.115) | (0.093) | (0.088) |
| Depression θ | | | | | | | 0.134 | 0.104 | 0.118 | 0.119 | 0.146 | 0.116 | 0.135 |
| | | | | | | | (0.092) | (0.077) | (0.079) | (0.085) | (0.101) | (0.085) | (0.080) |
| Area m | | | | | | | | 0.098 | 0.136 | 0.138 | 0.130 | 0.107 | 0.107 |
| | | | | | | | | (0.071) | (0.095) | (0.108) | (0.096) | (0.076) | (0.083) |
| Inertia I | | | | | | | | | 0.120 | 0.102 | 0.107 | 0.112 | 0.106 |
| | | | | | | | | | (0.089) | (0.079) | (0.070) | (0.088) | (0.074) |
| Inertia I (normalization) | | | | | | | | | | 0.115 | 0.164 | 0.121 | 0.124 |
| | | | | | | | | | | (0.079) | (0.116) | (0.074) | (0.075) |
| Inertia I_w | | | | | | | | | | | 0.468 | 0.101 | 0.136 |
| | | | | | | | | | | | (0.156) | (0.068) | (0.096) |
| Inertia I_wv | | | | | | | | | | | | 0.213 | 0.221 |
| | | | | | | | | | | | | (0.107) | (0.111) |
| Inertia I_w (normalization) | | | | | | | | | | | | | 0.143 |
| | | | | | | | | | | | | | (0.091) |
| Inertia I_wv (normalization) | | | | | | | | | | | | | |

EP 3 623 793 B1

# Continuation of Fig. 55

## (42B)

| | Wave length Δt | Wave height \|h\| | Peak position ratio r | Kurtosis k | Area ratio r_m | Depression θ | Area m | Inertia I | Inertia I (normalization) | Inertia I_w | Inertia I_wv | Inertia I_w (normalization) | Inertia I_wv (normalization) |
|---|---|---|---|---|---|---|---|---|---|---|---|---|---|
| Wave length Δt | | 0.183 | 0.147 | 0.137 | 0.152 | 0.142 | 0.098 | 0.091 | 0.137 | 0.114 | 0.372 | 0.122 | 0.133 |
| | | (0.115) | (0.096) | (0.083) | (0.093) | (0.096) | (0.061) | (0.062) | (0.079) | (0.085) | (0.109) | (0.081) | (0.076) |
| Wave height \|h\| | | | 0.140 | 0.107 | 0.130 | 0.143 | 0.164 | 0.125 | 0.137 | 0.123 | 0.221 | 0.108 | 0.109 |
| | | | (0.091) | (0.070) | (0.087) | (0.089) | (0.114) | (0.094) | (0.088) | (0.077) | (0.104) | (0.074) | (0.078) |
| Peak position ratio r | | | | 0.138 | 0.151 | 0.346 | 0.114 | 0.093 | 0.105 | 0.152 | 0.146 | 0.139 | 0.154 |
| | | | | (0.070) | (0.089) | (0.183) | (0.085) | (0.059) | (0.063) | (0.093) | (0.094) | (0.078) | (0.076) |
| Kurtosis k | | | | | 0.170 | 0.137 | 0.110 | 0.106 | 0.152 | 0.224 | 0.164 | 0.175 | 0.167 |
| | | | | | (0.085) | (0.077) | (0.066) | (0.066) | (0.088) | (0.101) | (0.096) | (0.093) | (0.087) |
| Area ratio r_m | | | | | | 0.170 | 0.098 | 0.097 | 0.131 | 0.145 | 0.145 | 0.179 | 0.162 |
| | | | | | | (0.106) | (0.062) | (0.063) | (0.077) | (0.085) | (0.081) | (0.071) | (0.068) |
| Depression θ | | | | | | | 0.116 | 0.104 | 0.113 | 0.160 | 0.161 | 0.141 | 0.142 |
| | | | | | | | (0.084) | (0.073) | (0.070) | (0.095) | (0.098) | (0.079) | (0.067) |
| Area m | | | | | | | | 0.106 | 0.119 | 0.108 | 0.175 | 0.118 | 0.128 |
| | | | | | | | | (0.064) | (0.085) | (0.073) | (0.104) | (0.068) | (0.075) |
| Inertia I | | | | | | | | | 0.099 | 0.105 | 0.144 | 0.113 | 0.111 |
| | | | | | | | | | (0.064) | (0.080) | (0.095) | (0.070) | (0.066) |
| Inertia I (normalization) | | | | | | | | | | 0.136 | 0.139 | 0.127 | 0.140 |
| | | | | | | | | | | (0.091) | (0.082) | (0.078) | (0.073) |
| Inertia I_w | | | | | | | | | | | 0.659 | 0.137 | 0.169 |
| | | | | | | | | | | | (0.151) | (0.074) | (0.088) |
| Inertia I_wv | | | | | | | | | | | | 0.154 | 0.161 |
| | | | | | | | | | | | | (0.090) | (0.083) |
| Inertia I_w (normalization) | | | | | | | | | | | | | 0.159 |
| | | | | | | | | | | | | | (0.103) |
| Inertia I_wv (normalization) | | | | | | | | | | | | | |

EP 3 623 793 B1

# Fig. 56

## (43A)

| | Wave length Δt | Wave height \|h\| | Peak position ratio r | Kurtosis k | Area ratio r_m | Depression θ | Area m | Inertia I | Inertia I (normalization) | Inertia I_w | Inertia I_wv | Inertia I_w (normalization) | Inertia I_wv (normalization) |
|---|---|---|---|---|---|---|---|---|---|---|---|---|---|
| Wave length Δt | | 0.181 (0.131) | 0.141 (0.091) | 0.137 (0.089) | 0.149 (0.092) | 0.135 (0.080) | 0.100 (0.066) | 0.093 (0.072) | 0.122 (0.074) | 0.120 (0.084) | 0.393 (0.134) | 0.124 (0.075) | 0.144 (0.095) |
| Wave height \|h\| | | | 0.130 (0.095) | 0.118 (0.086) | 0.126 (0.086) | 0.129 (0.094) | 0.138 (0.101) | 0.116 (0.081) | 0.138 (0.098) | 0.114 (0.082) | 0.225 (0.114) | 0.100 (0.067) | 0.101 (0.075) |
| Peak position ratio r | | | | 0.144 (0.077) | 0.159 (0.102) | 0.368 (0.192) | 0.113 (0.085) | 0.100 (0.075) | 0.119 (0.076) | 0.164 (0.093) | 0.144 (0.093) | 0.164 (0.080) | 0.144 (0.080) |
| Kurtosis k | | | | | 0.174 (0.086) | 0.153 (0.091) | 0.111 (0.077) | 0.116 (0.070) | 0.147 (0.084) | 0.188 (0.108) | 0.174 (0.108) | 0.172 (0.097) | 0.173 (0.090) |
| Area ratio r_m | | | | | | 0.177 (0.109) | 0.099 (0.063) | 0.111 (0.079) | 0.144 (0.073) | 0.157 (0.103) | 0.160 (0.106) | 0.193 (0.079) | 0.168 (0.081) |
| Depression θ | | | | | | | 0.107 (0.072) | 0.095 (0.066) | 0.114 (0.061) | 0.157 (0.091) | 0.176 (0.094) | 0.139 (0.080) | 0.144 (0.079) |
| Area m | | | | | | | | 0.095 (0.062) | 0.105 (0.073) | 0.102 (0.072) | 0.147 (0.117) | 0.116 (0.075) | 0.124 (0.074) |
| Inertia I | | | | | | | | | 0.101 (0.076) | 0.106 (0.076) | 0.144 (0.110) | 0.110 (0.073) | 0.116 (0.075) |
| Inertia I (normalization) | | | | | | | | | | 0.129 (0.079) | 0.138 (0.084) | 0.149 (0.094) | 0.149 (0.079) |
| Inertia I_w | | | | | | | | | | | 0.669 (0.135) | 0.145 (0.084) | 0.218 (0.091) |
| Inertia I_wv | | | | | | | | | | | | 0.171 (0.091) | 0.172 (0.099) |
| Inertia I_w (normalization) | | | | | | | | | | | | | 0.198 (0.092) |
| Inertia I_wv (normalization) | | | | | | | | | | | | | |

EP 3 623 793 B1

# Continuation of Fig. 56

## (43B)

| | Wave length Δt | Wave height \|h\| | Peak position ratio r | Kurtosis k | Area ratio r_m | Depression θ | Area m | Inertia I | Inertia I (normalization) | Inertia I_w | Inertia I_wv | Inertia I_w (normalization) | Inertia I_wv (normalization) |
|---|---|---|---|---|---|---|---|---|---|---|---|---|---|
| Wave length Δt | | 0.206 | 0.143 | 0.143 | 0.164 | 0.143 | 0.092 | 0.106 | 0.120 | 0.143 | 0.339 | 0.141 | 0.145 |
| | | (0.125) | (0.086) | (0.080) | (0.102) | (0.086) | (0.048) | (0.068) | (0.071) | (0.097) | (0.139) | (0.074) | (0.083) |
| Wave height \|h\| | | | 0.146 | 0.116 | 0.126 | 0.157 | 0.153 | 0.130 | 0.133 | 0.138 | 0.222 | 0.126 | 0.116 |
| | | | (0.089) | (0.070) | (0.074) | (0.090) | (0.093) | (0.075) | (0.093) | (0.086) | (0.129) | (0.073) | (0.068) |
| Peak position ratio r | | | | 0.198 | 0.220 | 0.393 | 0.121 | 0.122 | 0.136 | 0.161 | 0.162 | 0.172 | 0.159 |
| | | | | (0.088) | (0.104) | (0.188) | (0.073) | (0.073) | (0.073) | (0.089) | (0.082) | (0.091) | (0.070) |
| Kurtosis k | | | | | 0.212 | 0.169 | 0.138 | 0.117 | 0.178 | 0.227 | 0.140 | 0.198 | 0.177 |
| | | | | | (0.086) | (0.084) | (0.081) | (0.077) | (0.106) | (0.104) | (0.079) | (0.104) | (0.093) |
| Area ratio r_m | | | | | | 0.192 | 0.116 | 0.119 | 0.153 | 0.168 | 0.161 | 0.205 | 0.192 |
| | | | | | | (0.120) | (0.069) | (0.072) | (0.086) | (0.086) | (0.087) | (0.083) | (0.085) |
| Depression θ | | | | | | | 0.110 | 0.118 | 0.123 | 0.162 | 0.193 | 0.173 | 0.145 |
| | | | | | | | (0.059) | (0.065) | (0.071) | (0.092) | (0.102) | (0.085) | (0.080) |
| Area m | | | | | | | | 0.130 | 0.124 | 0.119 | 0.156 | 0.118 | 0.127 |
| | | | | | | | | (0.071) | (0.078) | (0.070) | (0.086) | (0.071) | (0.071) |
| Inertia I | | | | | | | | | 0.116 | 0.101 | 0.171 | 0.109 | 0.101 |
| | | | | | | | | | (0.065) | (0.058) | (0.104) | (0.063) | (0.056) |
| Inertia I (normalization) | | | | | | | | | | 0.133 | 0.169 | 0.146 | 0.139 |
| | | | | | | | | | | (0.077) | (0.096) | (0.080) | (0.074) |
| Inertia I_w | | | | | | | | | | | 0.672 | 0.160 | 0.191 |
| | | | | | | | | | | | (0.162) | (0.084) | (0.091) |
| Inertia I_wv | | | | | | | | | | | | 0.172 | 0.144 |
| | | | | | | | | | | | | (0.098) | (0.084) |
| Inertia I_w (normalization) | | | | | | | | | | | | | 0.155 |
| | | | | | | | | | | | | | (0.093) |
| Inertia I_wv (normalization) | | | | | | | | | | | | | |

EP 3 623 793 B1

# Fig. 57

## (44A)

| | Wave length Δt | Wave height \|h\| | Peak position ratio r | Kurtosis k | Area ratio r_m | Depression θ | Area m | Inertia I | Inertia I (normalization) | Inertia I_w | Inertia I_wv | Inertia I_w (normalization) | Inertia I_wv (normalization) |
|---|---|---|---|---|---|---|---|---|---|---|---|---|---|
| Wave length Δt | | 0.194 (0.110) | 0.170 (0.080) | 0.169 (0.086) | 0.174 (0.082) | 0.173 (0.084) | 0.133 (0.057) | 0.134 (0.071) | 0.142 (0.066) | 0.141 (0.063) | 0.337 (0.137) | 0.168 (0.082) | 0.160 (0.071) |
| Wave height \|h\| | | | 0.163 (0.082) | 0.135 (0.056) | 0.164 (0.078) | 0.152 (0.070) | 0.186 (0.116) | 0.158 (0.087) | 0.147 (0.093) | 0.170 (0.090) | 0.165 (0.085) | 0.154 (0.068) | 0.133 (0.057) |
| Peak position ratio r | | | | 0.170 (0.082) | 0.220 (0.106) | 0.459 (0.216) | 0.133 (0.058) | 0.145 (0.072) | 0.158 (0.072) | 0.189 (0.095) | 0.214 (0.118) | 0.206 (0.080) | 0.179 (0.073) |
| Kurtosis k | | | | | 0.240 (0.097) | 0.185 (0.083) | 0.153 (0.065) | 0.139 (0.057) | 0.197 (0.079) | 0.215 (0.097) | 0.247 (0.142) | 0.215 (0.094) | 0.183 (0.074) |
| Area ratio r_m | | | | | | 0.207 (0.106) | 0.141 (0.066) | 0.143 (0.066) | 0.191 (0.084) | 0.201 (0.105) | 0.253 (0.137) | 0.217 (0.084) | 0.180 (0.074) |
| Depression θ | | | | | | | 0.141 (0.058) | 0.143 (0.062) | 0.145 (0.059) | 0.175 (0.084) | 0.193 (0.113) | 0.167 (0.062) | 0.161 (0.072) |
| Area m | | | | | | | | 0.151 (0.065) | 0.146 (0.069) | 0.134 (0.057) | 0.188 (0.102) | 0.148 (0.069) | 0.130 (0.058) |
| Inertia I | | | | | | | | | 0.137 (0.058) | 0.143 (0.066) | 0.179 (0.090) | 0.145 (0.061) | 0.153 (0.071) |
| Inertia I (normalization) | | | | | | | | | | 0.146 (0.070) | 0.200 (0.109) | 0.189 (0.093) | 0.170 (0.079) |
| Inertia I_w | | | | | | | | | | | 0.565 (0.144) | 0.157 (0.072) | 0.165 (0.077) |
| Inertia I_wv | | | | | | | | | | | | 0.233 (0.127) | 0.223 (0.111) |
| Inertia I_w (normalization) | | | | | | | | | | | | | 0.161 (0.078) |
| Inertia I_wv (normalization) | | | | | | | | | | | | | |

EP 3 623 793 B1

# Continuation of Fig. 57

## (44B)

| | Wave length Δt | Wave height |h| | Peak position ratio r | Kurtosis k | Area ratio r_m | Depression θ | Area m | Inertia I | Inertia I (normalization) | Inertia I_w | Inertia I_wv | Inertia I_w (normalization) | Inertia I_wv (normalization) |
|---|---|---|---|---|---|---|---|---|---|---|---|---|---|
| Wave length Δt | | 0.221 | 0.193 | 0.176 | 0.201 | 0.187 | 0.151 | 0.159 | 0.165 | 0.206 | 0.328 | 0.186 | 0.225 |
| | | (0.093) | (0.077) | (0.059) | (0.084) | (0.075) | (0.028) | (0.043) | (0.050) | (0.081) | (0.122) | (0.066) | (0.087) |
| Wave height |h| | | | 0.178 | 0.187 | 0.182 | 0.204 | 0.207 | 0.169 | 0.170 | 0.186 | 0.221 | 0.175 | 0.173 |
| | | | (0.071) | (0.076) | (0.063) | (0.102) | (0.088) | (0.059) | (0.055) | (0.071) | (0.111) | (0.072) | (0.056) |
| Peak position ratio r | | | | 0.219 | 0.278 | 0.423 | 0.165 | 0.164 | 0.174 | 0.198 | 0.218 | 0.203 | 0.211 |
| | | | | (0.084) | (0.105) | (0.221) | (0.057) | (0.049) | (0.052) | (0.084) | (0.102) | (0.071) | (0.088) |
| Kurtosis k | | | | | 0.249 | 0.219 | 0.159 | 0.166 | 0.196 | 0.186 | 0.247 | 0.206 | 0.223 |
| | | | | | (0.092) | (0.078) | (0.039) | (0.050) | (0.082) | (0.073) | (0.109) | (0.069) | (0.082) |
| Area ratio r_m | | | | | | 0.255 | 0.170 | 0.156 | 0.207 | 0.212 | 0.265 | 0.232 | 0.205 |
| | | | | | | (0.111) | (0.056) | (0.039) | (0.084) | (0.083) | (0.111) | (0.091) | (0.075) |
| Depression θ | | | | | | | 0.164 | 0.161 | 0.189 | 0.183 | 0.222 | 0.195 | 0.185 |
| | | | | | | | (0.045) | (0.046) | (0.068) | (0.070) | (0.095) | (0.068) | (0.062) |
| Area m | | | | | | | | 0.179 | 0.174 | 0.189 | 0.216 | 0.169 | 0.174 |
| | | | | | | | | (0.067) | (0.064) | (0.083) | (0.096) | (0.057) | (0.057) |
| Inertia I | | | | | | | | | 0.177 | 0.170 | 0.201 | 0.162 | 0.160 |
| | | | | | | | | | (0.064) | (0.057) | (0.084) | (0.043) | (0.043) |
| Inertia I (normalization) | | | | | | | | | | 0.194 | 0.236 | 0.227 | 0.208 |
| | | | | | | | | | | (0.081) | (0.107) | (0.096) | (0.074) |
| Inertia I_w | | | | | | | | | | | 0.613 | 0.221 | 0.206 |
| | | | | | | | | | | | (0.137) | (0.086) | (0.084) |
| Inertia I_wv | | | | | | | | | | | | 0.313 | 0.281 |
| | | | | | | | | | | | | (0.131) | (0.116) |
| Inertia I_w (normalization) | | | | | | | | | | | | | 0.244 |
| | | | | | | | | | | | | | (0.095) |
| Inertia I_wv (normalization) | | | | | | | | | | | | | |

EP 3 623 793 B1

# Fig. 58

## (45A)

| | Wave length Δt | Wave height \|h\| | Peak position ratio r | Kurtosis k | Area ratio r_m | Depression θ | Area m | Inertia I | Inertia I (normalization) | Inertia I_w | Inertia I_wv | Inertia I_w (normalization) | Inertia I_wv (normalization) |
|---|---|---|---|---|---|---|---|---|---|---|---|---|---|
| Wave length Δt | | 0.224 | 0.165 | 0.164 | 0.157 | 0.164 | 0.138 | 0.145 | 0.159 | 0.176 | 0.315 | 0.176 | 0.162 |
| | | (0.153) | (0.081) | (0.078) | (0.066) | (0.076) | (0.049) | (0.062) | (0.080) | (0.083) | (0.137) | (0.074) | (0.083) |
| Wave height \|h\| | | | 0.166 | 0.151 | 0.177 | 0.188 | 0.187 | 0.155 | 0.157 | 0.178 | 0.187 | 0.172 | 0.154 |
| | | | (0.066) | (0.060) | (0.082) | (0.088) | (0.101) | (0.082) | (0.075) | (0.088) | (0.097) | (0.081) | (0.070) |
| Peak position ratio r | | | | 0.162 | 0.226 | 0.301 | 0.144 | 0.137 | 0.177 | 0.159 | 0.170 | 0.182 | 0.188 |
| | | | | (0.065) | (0.113) | (0.211) | (0.053) | (0.054) | (0.081) | (0.065) | (0.086) | (0.075) | (0.074) |
| Kurtosis k | | | | | 0.192 | 0.156 | 0.146 | 0.163 | 0.191 | 0.193 | 0.200 | 0.189 | 0.221 |
| | | | | | (0.084) | (0.063) | (0.060) | (0.077) | (0.095) | (0.090) | (0.106) | (0.075) | (0.087) |
| Area ratio r_m | | | | | | 0.214 | 0.148 | 0.165 | 0.179 | 0.186 | 0.212 | 0.178 | 0.207 |
| | | | | | | (0.105) | (0.057) | (0.079) | (0.084) | (0.085) | (0.124) | (0.073) | (0.081) |
| Depression θ | | | | | | | 0.134 | 0.150 | 0.170 | 0.161 | 0.171 | 0.167 | 0.174 |
| | | | | | | | (0.044) | (0.064) | (0.070) | (0.077) | (0.093) | (0.065) | (0.082) |
| Area m | | | | | | | | 0.184 | 0.141 | 0.151 | 0.168 | 0.164 | 0.143 |
| | | | | | | | | (0.075) | (0.050) | (0.061) | (0.075) | (0.082) | (0.053) |
| Inertia I | | | | | | | | | 0.149 | 0.155 | 0.168 | 0.143 | 0.154 |
| | | | | | | | | | (0.068) | (0.069) | (0.077) | (0.056) | (0.064) |
| Inertia I (normalization) | | | | | | | | | | 0.177 | 0.208 | 0.181 | 0.205 |
| | | | | | | | | | | (0.080) | (0.101) | (0.081) | (0.097) |
| Inertia I_w | | | | | | | | | | | 0.547 | 0.197 | 0.170 |
| | | | | | | | | | | | (0.170) | (0.090) | (0.073) |
| Inertia I_wv | | | | | | | | | | | | 0.236 | 0.220 |
| | | | | | | | | | | | | (0.117) | (0.116) |
| Inertia I_w (normalization) | | | | | | | | | | | | | 0.228 |
| | | | | | | | | | | | | | (0.108) |
| Inertia I_wv (normalization) | | | | | | | | | | | | | |

EP 3 623 793 B1

# Continuation of Fig. 58

## (45B)

| | Wave length Δt | Wave height \|h\| | Peak position ratio r | Kurtosis k | Area ratio r_m | Depression θ | Area m | Inertia I | Inertia I (normalization) | Inertia I_w | Inertia I_wv | Inertia I_w (normalization) | Inertia I_wv (normalization) |
|---|---|---|---|---|---|---|---|---|---|---|---|---|---|
| Wave length Δt | | 0.195 (0.110) | 0.159 (0.076) | 0.162 (0.082) | 0.184 (0.103) | 0.157 (0.076) | 0.148 (0.085) | 0.135 (0.053) | 0.163 (0.077) | 0.178 (0.093) | 0.310 (0.141) | 0.161 (0.079) | 0.158 (0.069) |
| Wave height \|h\| | | | 0.186 (0.103) | 0.156 (0.076) | 0.187 (0.119) | 0.187 (0.101) | 0.156 (0.073) | 0.169 (0.103) | 0.204 (0.120) | 0.168 (0.093) | 0.162 (0.099) | 0.162 (0.081) | 0.148 (0.067) |
| Peak position ratio r | | | | 0.177 (0.082) | 0.214 (0.126) | 0.334 (0.219) | 0.139 (0.056) | 0.152 (0.074) | 0.153 (0.067) | 0.183 (0.096) | 0.182 (0.097) | 0.172 (0.085) | 0.160 (0.072) |
| Kurtosis k | | | | | 0.168 (0.086) | 0.168 (0.084) | 0.161 (0.076) | 0.155 (0.075) | 0.174 (0.090) | 0.161 (0.078) | 0.185 (0.103) | 0.155 (0.066) | 0.161 (0.075) |
| Area ratio r_m | | | | | | 0.212 (0.126) | 0.153 (0.073) | 0.150 (0.074) | 0.225 (0.118) | 0.190 (0.099) | 0.194 (0.120) | 0.204 (0.079) | 0.220 (0.099) |
| Depression θ | | | | | | | 0.146 (0.050) | 0.157 (0.073) | 0.171 (0.087) | 0.164 (0.072) | 0.182 (0.093) | 0.171 (0.085) | 0.184 (0.087) |
| Area m | | | | | | | | 0.160 (0.073) | 0.182 (0.101) | 0.158 (0.081) | 0.153 (0.073) | 0.144 (0.063) | 0.133 (0.054) |
| Inertia I | | | | | | | | | 0.176 (0.098) | 0.152 (0.071) | 0.174 (0.105) | 0.140 (0.060) | 0.132 (0.058) |
| Inertia I (normalization) | | | | | | | | | | 0.166 (0.088) | 0.165 (0.092) | 0.174 (0.085) | 0.161 (0.091) |
| Inertia I_w | | | | | | | | | | | 0.585 (0.200) | 0.162 (0.087) | 0.161 (0.077) |
| Inertia I_wv | | | | | | | | | | | | 0.234 (0.117) | 0.230 (0.129) |
| Inertia I_w (normalization) | | | | | | | | | | | | | 0.178 (0.105) |
| Inertia I_wv (normalization) | | | | | | | | | | | | | |

EP 3 623 793 B1

# Fig. 59

| | Wave length Δt | Wave height |h| | Peak position ratio r | Kurtosis k | Area ratio r_m | Depression θ | Area m | Inertia I | Inertia I (normalization) | Inertia I_w | Inertia I_wv | Inertia I_w (normalization) | Inertia I_wv (normalization) |
|---|---|---|---|---|---|---|---|---|---|---|---|---|---|
| Wave length Δt | | 0.252 | 0.319 | 0.278 | 0.240 | 0.338 | 0.238 | 0.234 | 0.238 | 0.277 | 0.464 | 0.264 | 0.266 |
| | | (0.066) | (0.102) | (0.082) | (0.044) | (0.098) | (0.047) | (0.035) | (0.048) | (0.082) | (0.147) | (0.071) | (0.074) |
| Wave height |h| | | | 0.273 | 0.233 | 0.309 | 0.257 | 0.273 | 0.240 | 0.273 | 0.241 | 0.285 | 0.245 | 0.239 |
| | | | (0.095) | (0.041) | (0.125) | (0.069) | (0.062) | (0.048) | (0.100) | (0.055) | (0.101) | (0.061) | (0.043) |
| Peak position ratio r | | | | 0.246 | 0.255 | 0.539 | 0.239 | 0.236 | 0.251 | 0.273 | 0.363 | 0.243 | 0.268 |
| | | | | (0.059) | (0.073) | (0.138) | (0.045) | (0.046) | (0.055) | (0.076) | (0.131) | (0.052) | (0.061) |
| Kurtosis k | | | | | 0.249 | 0.247 | 0.226 | 0.228 | 0.275 | 0.275 | 0.381 | 0.251 | 0.267 |
| | | | | | (0.054) | (0.058) | (0.020) | (0.029) | (0.081) | (0.084) | (0.139) | (0.054) | (0.065) |
| Area ratio r_m | | | | | | 0.261 | 0.261 | 0.234 | 0.258 | 0.247 | 0.325 | 0.243 | 0.265 |
| | | | | | | (0.082) | (0.065) | (0.049) | (0.054) | (0.055) | (0.134) | (0.045) | (0.069) |
| Depression θ | | | | | | | 0.228 | 0.231 | 0.249 | 0.286 | 0.342 | 0.249 | 0.249 |
| | | | | | | | (0.025) | (0.030) | (0.057) | (0.080) | (0.130) | (0.050) | (0.053) |
| Area m | | | | | | | | 0.242 | 0.242 | 0.232 | 0.290 | 0.232 | 0.234 |
| | | | | | | | | (0.053) | (0.057) | (0.036) | (0.098) | (0.035) | (0.038) |
| Inertia I | | | | | | | | | 0.245 | 0.240 | 0.287 | 0.230 | 0.236 |
| | | | | | | | | | (0.052) | (0.056) | (0.090) | (0.031) | (0.039) |
| Inertia I (normalization) | | | | | | | | | | 0.256 | 0.277 | 0.234 | 0.238 |
| | | | | | | | | | | (0.067) | (0.090) | (0.036) | (0.038) |
| Inertia I_w | | | | | | | | | | | 0.680 | 0.257 | 0.263 |
| | | | | | | | | | | | (0.209) | (0.077) | (0.056) |
| Inertia I_wv | | | | | | | | | | | | 0.402 | 0.441 |
| | | | | | | | | | | | | (0.155) | (0.145) |
| Inertia I_w (normalization) | | | | | | | | | | | | | 0.253 |
| | | | | | | | | | | | | | (0.063) |
| Inertia I_wv (normalization) | | | | | | | | | | | | | |

EP 3 623 793 B1

# Fig. 60

| | Wave length Δt | Wave height \|h\| | Peak position ratio r | Kurtosis k | Area ratio r_m | Depression θ | Area m | Inertia I | Inertia I (normalization) | Inertia I_w | Inertia I_wv | Inertia I_w (normalization) | Inertia I_wv (normalization) |
|---|---|---|---|---|---|---|---|---|---|---|---|---|---|
| Wave length Δt | | 0.211 | 0.174 | 0.165 | 0.174 | 0.177 | 0.134 | 0.133 | 0.155 | 0.153 | 0.346 | 0.161 | 0.169 |
| | | (0.115) | (0.086) | (0.080) | (0.085) | (0.085) | (0.058) | (0.061) | (0.071) | (0.083) | (0.133) | (0.076) | (0.081) |
| Wave height \|h\| | | | 0.171 | 0.145 | 0.175 | 0.178 | 0.181 | 0.155 | 0.165 | 0.172 | 0.204 | 0.151 | 0.141 |
| | | | (0.088) | (0.069) | (0.091) | (0.090) | (0.095) | (0.083) | (0.090) | (0.089) | (0.104) | (0.074) | (0.065) |
| Peak position ratio r | | | | 0.174 | 0.206 | 0.384 | 0.143 | 0.139 | 0.154 | 0.179 | 0.195 | 0.177 | 0.180 |
| | | | | (0.076) | (0.103) | (0.198) | (0.056) | (0.063) | (0.068) | (0.086) | (0.100) | (0.076) | (0.076) |
| Kurtosis k | | | | | 0.202 | 0.172 | 0.144 | 0.144 | 0.182 | 0.204 | 0.215 | 0.189 | 0.192 |
| | | | | | (0.084) | (0.077) | (0.062) | (0.064) | (0.088) | (0.093) | (0.112) | (0.082) | (0.082) |
| Area ratio r_m | | | | | | 0.204 | 0.144 | 0.142 | 0.179 | 0.182 | 0.211 | 0.200 | 0.196 |
| | | | | | | (0.107) | (0.067) | (0.065) | (0.082) | (0.088) | (0.113) | (0.077) | (0.080) |
| Depression θ | | | | | | | 0.142 | 0.140 | 0.155 | 0.174 | 0.199 | 0.169 | 0.169 |
| | | | | | | | (0.060) | (0.062) | (0.069) | (0.083) | (0.102) | (0.073) | (0.074) |
| Area m | | | | | | | | 0.149 | 0.152 | 0.148 | 0.180 | 0.146 | 0.144 |
| | | | | | | | | (0.067) | (0.075) | (0.071) | (0.094) | (0.066) | (0.062) |
| Inertia I | | | | | | | | | 0.147 | 0.142 | 0.175 | 0.141 | 0.141 |
| | | | | | | | | | (0.070) | (0.068) | (0.092) | (0.061) | (0.061) |
| Inertia I (normalization) | | | | | | | | | | 0.161 | 0.188 | 0.172 | 0.173 |
| | | | | | | | | | | (0.079) | (0.098) | (0.080) | (0.076) |
| Inertia I_w | | | | | | | | | | | 0.606 | 0.171 | 0.187 |
| | | | | | | | | | | | (0.163) | (0.080) | (0.082) |
| Inertia I_wv | | | | | | | | | | | | 0.236 | 0.233 |
| | | | | | | | | | | | | (0.115) | (0.110) |
| Inertia I_w (normalization) | | | | | | | | | | | | | 0.191 |
| | | | | | | | | | | | | | (0.092) |
| Inertia I_wv (normalization) | | | | | | | | | | | | | |

Combination of high precision

1: Wave length Δt — Inertia I
2: Wave length Δt — Area m
3: Peak position ratio r — Inertia I
4: Depression θ — Inertia I
5: Inertia I — Inertia I _w(normalization)

EP 3 623 793 B1

# Fig. 61

EP 3 623 793 B1

| | Wave length Δt | Wave height \|h\| | Peak position ratio r | Kurtosis k | Area ratio r_m | Depression θ | Area m | Inertia I | Inertia I (normaliza-tion) | Inertia I_w | Inertia I_wv | Inertia I_w (normalization) | Inertia I_wv (normalization) |
|---|---|---|---|---|---|---|---|---|---|---|---|---|---|
| Wave length Δt | | 0.202 (0.126) | 0.140 (0.090) | 0.134 (0.083) | 0.152 (0.096) | 0.143 (0.090) | 0.099 (0.064) | 0.098 (0.072) | 0.132 (0.078) | 0.121 (0.087) | 0.339 (0.128) | 0.123 (0.078) | 0.138 (0.087) |
| Wave height \|h\| | | | 0.143 (0.094) | 0.112 (0.078) | 0.139 (0.089) | 0.154 (0.096) | 0.155 (0.105) | 0.127 (0.092) | 0.133 (0.091) | 0.151 (0.100) | 0.204 (0.111) | 0.114 (0.075) | 0.106 (0.073) |
| Peak position ratio r | | | | 0.148 (0.078) | 0.166 (0.100) | 0.349 (0.193) | 0.117 (0.081) | 0.104 (0.059) | 0.118 (0.071) | 0.151 (0.090) | 0.151 (0.091) | 0.148 (0.081) | 0.153 (0.079) |
| Kurtosis k | | | | | 0.180 (0.085) | 0.144 (0.082) | 0.113 (0.074) | 0.112 (0.073) | 0.152 (0.091) | 0.201 (0.103) | 0.168 (0.101) | 0.171 (0.096) | 0.168 (0.090) |
| Area ratio r_m | | | | | | 0.171 (0.108) | 0.107 (0.071) | 0.107 (0.071) | 0.138 (0.078) | 0.150 (0.092) | 0.161 (0.097) | 0.181 (0.081) | 0.171 (0.081) |
| Depression θ | | | | | | | 0.117 (0.077) | 0.105 (0.070) | 0.117 (0.070) | 0.149 (0.091) | 0.169 (0.099) | 0.142 (0.082) | 0.141 (0.077) |
| Area m | | | | | | | | 0.107 (0.067) | 0.121 (0.083) | 0.117 (0.081) | 0.152 (0.101) | 0.115 (0.073) | 0.122 (0.076) |
| Inertia I | | | | | | | | | 0.109 (0.073) | 0.104 (0.073) | 0.142 (0.095) | 0.111 (0.074) | 0.108 (0.068) |
| Inertia I (normalization) | | | | | | | | | | 0.128 (0.081) | 0.152 (0.095) | 0.136 (0.082) | 0.138 (0.075) |
| Inertia I_w | | | | | | | | | | | 0.617 (0.151) | 0.136 (0.078) | 0.178 (0.091) |
| Inertia I_wv | | | | | | | | | | | | 0.177 (0.096) | 0.174 (0.094) |
| Inertia I_w (normalization) | | | | | | | | | | | | | 0.163 (0.095) |
| Inertia I_wv (normalization) | | | | | | | | | | | | | |

Combination of high precision

1: Wave length Δt — Inertia I
2: Wave length Δt — Area m
3: Peak position ratio r — Inertia I
4: Inertia I — Inertia I _w
5: Depression θ — Inertia I

2016-10-19

# Fig. 62

| | Wave length Δt | Wave height \|h\| | Peak position ratio r | Kurtosis k | Area ratio r_m | Depression θ | Area m | Inertia I | Inertia I (normalization) | Inertia I_w | Inertia I_wv | Inertia I_w (normalization) | Inertia I_wv (normalization) |
|---|---|---|---|---|---|---|---|---|---|---|---|---|---|
| Wave length Δt | | 0.217 (0.106) | 0.201 (0.083) | 0.190 (0.077) | 0.191 (0.076) | 0.204 (0.082) | 0.162 (0.053) | 0.162 (0.053) | 0.173 (0.064) | 0.196 (0.080) | 0.351 (0.137) | 0.191 (0.075) | 0.194 (0.077) |
| Wave height \|h\| | | | 0.193 (0.083) | 0.172 (0.062) | 0.204 (0.093) | 0.198 (0.086) | 0.202 (0.088) | 0.178 (0.076) | 0.190 (0.089) | 0.189 (0.079) | 0.204 (0.098) | 0.181 (0.072) | 0.159 (0.059) |
| Peak position ratio r | | | | 0.195 (0.074) | 0.238 (0.105) | 0.411 (0.201) | 0.164 (0.054) | 0.167 (0.059) | 0.182 (0.065) | 0.200 (0.083) | 0.229 (0.107) | 0.201 (0.073) | 0.201 (0.074) |
| Kurtosis k | | | | | 0.219 (0.083) | 0.195 (0.073) | 0.169 (0.052) | 0.170 (0.058) | 0.207 (0.085) | 0.206 (0.084) | 0.252 (0.120) | 0.203 (0.072) | 0.211 (0.077) |
| Area ratio r_m | | | | | | 0.230 (0.106) | 0.174 (0.063) | 0.170 (0.061) | 0.212 (0.085) | 0.207 (0.085) | 0.250 (0.125) | 0.215 (0.074) | 0.216 (0.079) |
| Depression θ | | | | | | | 0.162 (0.046) | 0.168 (0.055) | 0.185 (0.068) | 0.194 (0.077) | 0.222 (0.105) | 0.190 (0.066) | 0.191 (0.071) |
| Area m | | | | | | | | 0.183 (0.067) | 0.177 (0.068) | 0.173 (0.064) | 0.203 (0.089) | 0.171 (0.061) | 0.163 (0.052) |
| Inertia I | | | | | | | | | 0.177 (0.068) | 0.172 (0.064) | 0.202 (0.089) | 0.164 (0.050) | 0.167 (0.055) |
| Inertia I (normalization) | | | | | | | | | | 0.188 (0.077) | 0.217 (0.100) | 0.201 (0.078) | 0.200 (0.076) |
| Inertia I_w | | | | | | | | | | | 0.598 (0.172) | 0.199 (0.082) | 0.193 (0.075) |
| Inertia I_wv | | | | | | | | | | | | 0.283 (0.129) | 0.279 (0.123) |
| Inertia I_w (normalization) | | | | | | | | | | | | | 0.213 (0.090) |
| Inertia I_wv (normalization) | | | | | | | | | | | | | |

Combination of high precision

1: Wave length Δt — Area m

2: Wave length Δt — Inertia I

3: Depression θ — Area m

4: Area m — Inertia I _wv (normalization)

5: Peak position ratio r — Area m

2016-10-19

# Fig. 63

(50A)

(50B)

# Fig. 64

(51A)

(51B)

# Fig. 65

(52A)

(52B)

# Fig. 66

# Fig. 67

# Fig. 68

```
            ┌─────────────────┐
            │ Control Process │
            │     of PC1      │
            └────────┬────────┘
                     │
                     ▼
S100 ──────  ┌─────────────────┐
             │  Input Process  │
             └────────┬────────┘
                      │
                      ▼
             ┌──────────────────┐
             │Acquisition process│
S101 ──────  │ of feature value │
             │  of input data   │
             └────────┬─────────┘
                      │
                      ▼
                    ╱─╲              N
S102 ──────       ╱Analyte╲────────────────┐
                 ╱ type    ╲               │
                 ╲specified?╱               │
                   ╲─╲─╱─╱                  │
                     │ Y                    │
                     ▼                      │
                    ╱─╲         N           │
S103 ──────       ╱Classifi-╲──────┐        │
                 ╱ cation    ╲      │        │
                 ╲ analysis  ╱      │        │
                  ╲specified?╱      │        │
                    ╲─╱─╱            │        │
                     │ Y            │ ┌── S105│
                     ▼              ▼         │
S104 ──── ┌─────────────┐   ┌──────────────┐ │
          │Classification│   │   Number     │ │
          │analysis process│ │analysis process│
          └──────┬───────┘   └──────┬───────┘ │
                 │◄─────────────────┘         │
                 ▼                            │
S106 ──── ┌──────────────┐                    │
          │Output Process│                    │
          └──────┬───────┘                    │
                 │                            │
                 ▼◄───────────────────────────┘
          ┌──────────────┐
          │     End      │
          └──────────────┘
```

116

# Fig. 69

Classification analysis process

S110 — Known data exist? — N

Y

S111 — Feature value specified ? — N

Y

S112 — Acquisition of all feature value data

S113 — Acquisition of specified feature value data

S114 — Analysis data ? — N

Y

S115 — Acquisition of feature value data on analysis data

S116 — Execution of classification analysis program due to machine learning

S117 — Output process of classification analysis results

End

# Fig. 70

(57A)

| Rank | Feature | Dh | Dw | Classifier | TPRate | FPRate | Precision | Recall | FMeasure | ROCArea |
|---|---|---|---|---|---|---|---|---|---|---|
| 1st① | h&wV | 128 | | 4 meta.RandomCommittee | 0.98810 | 0.01190 | 0.98810 | 0.98810 | 0.98809 | 0.99943 |
| 2nd① | hv&F | 8 | | trees.NBTree | 0.97619 | 0.02381 | 0.97619 | 0.97619 | 0.97619 | 0.97902 |
| 2nd② | h&wV | 128 | | 4 meta.LogitBoost | 0.97619 | 0.02381 | 0.97619 | 0.97619 | 0.97619 | 0.99546 |
| 2nd③ | h&wV | 128 | | 4 meta.MultiBoostAB | 0.97619 | 0.02381 | 0.97619 | 0.97619 | 0.97619 | 0.99887 |
| 2nd④ | h&wV | 128 | | 8 trees.NBTree | 0.97619 | 0.02381 | 0.97619 | 0.97619 | 0.97619 | 0.99688 |
| 2nd⑤ | h&wNrmdV | 64 | | 4 meta.RotationForest | 0.97619 | 0.02381 | 0.97619 | 0.97619 | 0.97619 | 0.99433 |
| 2nd⑥ | h&wNrmdV | 128 | | 4 meta.LogitBoost | 0.97619 | 0.02381 | 0.97619 | 0.97619 | 0.97619 | 0.99093 |
| 2nd⑦ | h&wNrmdV | 128 | | 4 meta.MultiBoostAB | 0.97619 | 0.02381 | 0.97619 | 0.97619 | 0.97619 | 0.99887 |
| 2nd⑧ | h&wNrmdV | 128 | | 8 trees.NBTree | 0.97619 | 0.02381 | 0.97619 | 0.97619 | 0.97619 | 0.99688 |
| 10th① | hv&F | 8 | | bayes.BayesNet | 0.97619 | 0.02381 | 0.97619 | 0.97619 | 0.97618 | 0.99150 |
| 10th② | h&wV | 64 | | 4 trees.BFTree | 0.97619 | 0.02381 | 0.97619 | 0.97619 | 0.97618 | 0.96372 |
| 10th③ | h&wV | 64 | | 4 trees.SimpleCart | 0.97619 | 0.02381 | 0.97619 | 0.97619 | 0.97618 | 0.96372 |
| 10th④ | h&wV | 128 | | 4 trees.RandomForest | 0.97619 | 0.02381 | 0.97619 | 0.97619 | 0.97618 | 0.99575 |
| 10th⑤ | h&wNrmdV | 64 | | 4 trees.BFTree | 0.97619 | 0.02381 | 0.97619 | 0.97619 | 0.97618 | 0.96372 |
| 10th⑥ | h&wNrmdV | 64 | | 4 trees.SimpleCart | 0.97619 | 0.02381 | 0.97619 | 0.97619 | 0.97618 | 0.96372 |

Combination of feature value / Evaluation result

(57B)

| Φ | Pore Depth | Bacterial | Pulse number | Used pulse number |
|---|---|---|---|---|
| 4.5 | 1500 | E. coli | 42 | 42 |
| 4.5 | 1500 | B. subtilis | 265 | 42 |

EP 3 623 793 B1

# Fig. 71

(58A)

|  | Real number of E.coli:P (Positive) | Real Number of B.subtilis:N (Negative) |  |
|---|---|---|---|
| E.coli and Prediction:PP (Predicted Positive) | TP (True Positive) | FP (False Positive) | TP/PP Precision |
| B.subtilis and prediction:PN (Predicted Negative) | FN (False Negative) | TN (True Negative) | FN/PN |
|  | TP/P TPR(True Positive rate) Recall | FP/N FPR(False Positive rate) |  |

(58B) $$\text{F-measure} = \frac{2TP}{2TP + FP + FN}$$

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013137209 A

- US 2015275263 A1

**Non-patent literature cited in the description**

- **FROSENSTEIN, J. K. ; WANUNUA, M. ; MERCHANT, C. A. ; DRNDIC, M. ; SHEPARD, K. L.** Integrated nanopore sensing platform with sub-microsecond temporal resolution. *Nature Methods,* 2012, 487-492
- Weka3:Data Mining Software in JavaJ. Machine Learning Group at the University of Waikato
- Learning Classifiers from Only Positive and Unlabeled Data. **ELKAN, C. ; NOTO, K.** KDD '08 Proceedings of the 14th ACM SIGKDD international conference on Knowledge discovery. ACM, 2008, 213-220

- **FTSUTSUI, M. ; TANIGUCHI, M. ; YOKOTA, K. ; KAWAI, T.** Identifying Single Nucleotides by Tunneling Current. *Nature Nanotechnology,* 2010, vol. 5, 286-290
- **HASSELBLAD V.** Estimation of parameters for a mixture of normal distributions. *Technomerics,* 1966, vol. 8, 431-444